# EUROPEAN PATENT SPECIFICATION

(11) **EP 2 855 526 B1**
(45) Date of publication and mention of the grant of the patent: **07.11.2018**
(21) Application number: 13713373.2
(22) Date of filing: 15.03.2013
(51) Int. Cl.: A61K 51/10, C07K 16/28, A61K 39/00

(54) **ANTI-MACROPHAGE MANNOSE RECEPTOR IMMUNOGLOBULIN SINGLE VARIABLE DOMAINS FOR TARGETING AND IN VIVO IMAGING OF TUMOR-ASSOCIATED MACROPHAGES**
EINZELNE IMMUNOGLOBULIN D'IMMUNOGLOBULIN VARIABLE ANTIMAKROPHAGEN-MANNOSE-REZEPTORDOMÄNEN ZUR ANZIELUNG UND IN-VIVO-BILDGEBUNG VON TUMORASSOZIIERTEN MAKROPHAGEN
DOMAINES VARIABLES INDIVIDUELS CONTRE LE RÉCEPTEUR MANNOSE DES MACROPHAGES POUR LE CIBLAGE ET L'IMAGERIE IN VIVO DE MACROPHAGES ASSOCIÉS À DES TUMEURS

(30) Priority: 24.05.2012 US 201213480350
(43) Date of publication of application: 08.04.2015
(73) Proprietor: VIB vzw, 9052 Ghent (BE); Vrije Universiteit Brussel, 1050 Brussel (BE)
(72) Inventor: VAN GINDERACHTER, Jo, 9400 Ninove (BE); DE BAETSELIER, Patrick, 2600 Berchem (BE); DEVOOGDT, Nick, 1980 Zemst (BE); LAHOUTTE, Tony, 1083 Ganshoren (BE); LAOUI, Damya, 1300 Limal (BE); MOVAHEDI, Kiavash, 60596 Frankfurt am Main (DE); RAES, Geert, 1640 Sint-Genesius-Rode (BE); SCHOONOOGHE, Steve, 3010 Kessel-Lo (BE)
(86) International application number: PCT/EP2013/055427
(87) International publication number: WO 2013/174537

(56) References cited:
- US-A1- 2011 262 348
- US-A1- 2012 301 394
- DENARDA DANGAJ ET AL: "Mannose Receptor (MR) Engagement by Mesothelin GPI Anchor Polarizes Tumor-Associated Macrophages and Is Blocked by Anti-MR Human Recombinant Antibody", PLOS ONE, PUBLIC LIBRARY OF SCIENCE, US, vol. 6, no. 12, 1 December 2011 (2011-12-01), pages e28386-1, XP009170212, ISSN: 1932-6203
- P. B. KANG: "The human macrophage mannose receptor directs Mycobacterium tuberculosis lipoarabinomannan-mediated phagosome biogenesis", JOURNAL OF EXPERIMENTAL MEDICINE, vol. 202, no. 7, 3 October 2005 (2005-10-03), pages 987-999, XP055065055, ISSN: 0022-1007, DOI: 10.1084/jem.20051239
- RUDIKOFF S ET AL: "Single amino acid substitution altering antigen-binding specificity", PROCEEDINGS OF THE NATIONAL ACADEMY OF SCIENCES, NATIONAL ACADEMY OF SCIENCES, US, vol. 79, 1 March 1982 (1982-03-01), pages 1979-1983, XP007901436, ISSN: 0027-8424, DOI: 10.1073/PNAS.79.6.1979
- Uniprot: "Alignment human and mouse MMR", , 14 June 2016 (2016-06-14), XP55280468, Retrieved from the Internet: URL:http://www.uniprot.org/align/A20160614 ACFE4208EAFA842A78A1B3BA7138A93DA8EBB2B [retrieved on 2016-06-14]
- SHAW S-Y ET AL: "A spontaneous variant of an antidigoxin hybridoma antibody with increased affinity arises from a heavy chain signal peptide mutation", MOLECULAR IMMUNOLOGY, PERGAMON, GB, vol. 29, no. 4, 1 April 1992 (1992-04-01), pages 525-529, XP023681905, ISSN: 0161-5890, DOI: 10.1016/0161-5890(92)90010-U [retrieved on 1992-04-01]
- PANKA D J ET AL: "VARIABLE REGION FRAMEWORK DIFFERENCES RESULT IN DECREASED OR INCREASED AFFINITY OF VARIANT ANTI-DIGOXIN ANTIBODIES", PROCEEDINGS NATIONAL ACADEMY OF SCIENCES PNAS, NATIONAL ACADEMY OF SCIENCES, US, vol. 85, no. 9, 1 May 1988 (1988-05-01), pages 3080-3084, XP000611718, ISSN: 0027-8424, DOI: 10.1073/PNAS.85.9.3080

## Description

### FIELD OF THE INVENTION

The present invention relates to immunoglobulin single variable domains directed against human macrophage mannose receptor (MMR) and their uses in the field of oncology. More specifically, it concerns immunoglobulin single variable domains, including nanobodies, against human MMR and their use in targeting and in vivo imaging of tumor-associated macrophages, with applications in the field of cancer diagnostics and therapeutics and monitoring of the disease.

### BACKGROUND

Non-invasive molecular imaging is a powerful technique aimed at tracking cellular and molecular events in their native environment in the intact living subject. In its broadest sense, molecular imaging entails the administration of a tracer molecule labeled with a contrast reagent for visualization. Primarily, radioactively labeled tracers are used in combination with positron-emission tomography (PET) or single photon-emission computed tomography (SPECT)-based imaging techniques (Pysz et al. 2010, Clin Radiol 65: 500-16). In the clinic, the majority of cancer imaging is currently still performed based on detection of enhanced metabolism in cancer cells using ¹⁸F radiolabeled deoxyglucose (Coenen et al. 2010, Nucl Med Biol 37: 727-40), while ^{99m}Tc-labeled human serum albumin is used for lymphoscintigraphic mapping of the draining lymph nodes in cancer (Kim et al. 2001, Int J Oncol 19: 991-6). Although useful, these tracers do not target a specific molecule or receptor on the surface of the cells involved in the disease process. Therefore, there is a need for probes that allow a more specific molecular characterization of inflamed or diseased tissue using disease related membrane antigens. These specific markers can help to define the phenotype of a disease and can be targeted by specific agents like monoclonal antibodies (MAbs). In this context, the choice of the targeted molecular markers will be a critical factor in determining whether it is possible to acquire in-depth molecular information on the underlying disease process.

Several FDA approved MAbs directed against tumor-associated antigens (TAAs) on malignant cells are being applied for diagnosis and treatment of cancer, with a few of the most commonly used MAbs being human epidermal growth factor receptor 2 (HER2)-specific Trastuzumab (Dijkers et al. 2010, Clin Pharmacol Ther 87: 586-92), carcinoembryonic antigen (CEA)-specific Arcitumomab (Hong et al. 2008, Biomark Insights, 3: 435-451) and prostate-specific membrane antigen (PSMA)-specific Capromab (Aparici et al. 2012, Am J Nucl Med Mol Imaging, 2: 48-54). Yet, although the direct targeting of antibody moieties to TAAs on malignant cells is a potent tool that has reached clinical maturity, the non-transformed cells present within the tumor microenvironment can also provide useful biomarkers for molecular imaging, as an alternative or complement to markers on the inherently genetically instable transformed cells. Indeed, tumors should be considered as organ-like structures featuring a complex bidirectional interplay between transformed (cancer) and non-transformed (stromal) cells, whereby stromal cells can critically contribute to tumor initiation, growth and metastasis. Hence, targeting these tumor-associated stroma cells for imaging could provide additional information on the state of the tumor or response to therapy.

In particular, tumor-associated macrophages (TAMs) are an important component of the tumor stroma, both in murine models and human patients (Pollard 2004, Nat Rev Cancer 4: 71-8). TAMs can promote tumor-growth by affecting angiogenesis, immune suppression and invasion and metastasis (Lin et al. 2006, Cancer Res 66: 11238-46). The plasticity of macrophages offers perspectives for using them as *in vivo* sensors for the tumor microenvironment they are exposed to. As a matter of fact, at the tumor site, these cells are confronted with different tumor microenvironments, leading to different TAM subsets with specialized functions and distinct molecular profiles (Laoui et al. 2011, Int. J. Dev. Biol., 55: 861-867). For example in mammary tumors, at least two distinct TAM subpopulations have been described, based on a differential expression of markers such as the macrophage mannose receptor (MMR or MHC II), differences in pro-angiogenic or immunosuppressive properties and intratumoral localization (normoxic/perivascular tumor areas versus hypoxic regions). In particular, the association of MMR-high TAMs with hypoxic regions in the tumor (Movahedi et al. 2010, Cancer Res, 70: 5728-5739) offers perspectives for image-guided radiotherapy.

Full-sized MAbs have a number of disadvantages that have so far limited their effective use in the clinic. MAbs are macromolecules with a relatively poor penetration into solid and isolated tissues such as tumors (Hughes et al. 2000, J. Clin. Oncol., 18: 363-370). In addition, complete MAbs feature a long residence time in the body and a potential increase in background signals because of binding to Fc receptors on non-target cells, making them less suitable for molecular imaging applications. Indeed, for imaging the most important properties of a tracer are: rapid interaction with the target, fast clearing of unbound molecules from the body and low non-specific accumulation, especially around the area of interest. These requirements have led to the development of a myriad of antibody derived probe formats, like Fabs and scFvs, trying to combine specificity with a small size for favorable pharmacokinetics (Kaur et al. 2012, Cancer Lett., 315: 97-111).

A novel approach for generating small and high-affinity antigen-binding moieties focuses on the use of single-domain VHH antibody fragments, named nanobodies, derived from the heavy-chain only antibodies found in camelid species (Hamers-Casterman et al. 2003, Nature 363: 446-448). For instance, US2012301394 discloses immunoglobulin single variable domains against markers of TAMs and methods for in vivo imaging of tumor cells, as well as cancer diagnostics and therapeutics. Also Dangaj et al., (2011. PLoS One. 6(12):e28386) revealed anti-CRD4-MR scFv able to block tumor antigen GPI anchor/CD206 interactions, to prevent tumor-induced TAM polarization. Nanobodies, conveniently labeled with ^{99m}Tc at their carboxy-terminal hexahistidine-tail, have by now a solid track record for SPECT-based molecular imaging in preclinical animal models (reviewed in: Vaneycken et al. 2011, Curr Opin Biotechnol 22: 877-881), with rapid blood clearance of unbound probes and high signal-to-noise ratios as early as a few hours after inoculation. In particular, US20110262348 demonstrates the usefulness of ^{99m}Tc-labeled mouse-specific anti-MMR nanobodies for targeting MMR-positive TAMs in mice models that spontaneously develop carcinomas. These results offer perspectives for applications of anti-MMR nanobodies in image-guided radiotherapy, whereby the distribution of radiation is adapted in function of localized risk factors such as hypoxia (Bentzen 2005, Lancet Oncol, 6: 112-117). Moreover, as has been documented for nanobodies targeting the HER2 tumor antigen, nanobodies exhibiting effective tumor targeting can be converted from an imaging probe in a radioimmunotherapeutic compound by coupling it to a therapeutic radionuclide (D'Huyvetter et al. 2012, Contrast Media Mol. Imaging, 7: 254-264).

However, there is still a need for specific probes that can be used both in the clinic and in preclinical animal models, with applications including improved diagnosis, prognosis, treatment and therapy monitoring.

### SUMMARY OF THE INVENTION

The applicants have found that MMR-positive TAMs can be detected in intratumoral hypoxic zones of human samples, as illustrated in human breast cancer samples, demonstrating the clinical relevance of targeting MMR-positive TAM subpopulations in the tumor stroma. Therefore, immunoglobulin single variable domains, in particular nanobodies, were generated that specifically recognize human MMR. Several of these nanobodies were found to be cross-reactive with mouse MMR, which is of advantage for diagnostic and/or therapeutic development, since it allows the same immunoglobulin single variable domain to be tested in pre-clinical disease models as well as in clinical settings.

The present invention thus provides for immunoglobulin single variable domains, including nanobodies, directed against the human macrophage mannose receptor, and their usefulness for selective in vivo targeting and imaging of MMR-positive TAM subpopulations in the tumor stroma. Evidence is provided that MMR-positive TAMs can be efficiently targeted in vivo using these anti-MMR immunoglobulin single variable domains in preclinical animal models, as illustrated in murine models.

Accordingly, a first aspect of the invention relates to an immunoglobulin single variable domain that specifically binds to human macrophage mannose receptor (SEQ ID NO: 1), wherein the immunoglobulin single variable domain comprises an amino acid sequence that comprises 4 framework regions (FR) and 3 complementarity determining regions (CDR) according to the following formula (1):

FR1-CDR1-FR2-CDR2-FR3-CDR3-FR4 (1);

and
wherein CDR1 consists of SEQ ID NO: 67, CDR2 consists of SEQ ID NO: 127, and CDR3 consists of SEQ ID NO: 187;
and wherein the framework regions (FRs) have an amino acid sequence of SEQ ID N0: 37 (FR1), SEQ ID N0: 97 (FR2), SEQ ID NO: 157 (FR3), SEQ ID NO: 217 (FR4).

According to a preferred embodiment, the immunoglobulin single variable domain is a Nanobody with SEQ ID NO: 7.

It is particularly envisaged that the immunoglobulin single variable domains as described above are fused to a detectable label, such as a radionuclide. The immunoglobulin single variable domain as described above may also be fused to a functional moiety, preferably a therapeutically active agent.

Also encompassed are polypeptides comprising one or more of any of the above described immunoglobulin single variable domains, as well as nucleic acids encoding an immunoglobulin single variable domain or a polypeptide as described above.

According to another aspect, the invention also relates to a pharmaceutical composition comprising an immunoglobulin single variable domain as described above, or a polypeptide as described above, and optionally at least one of a pharmaceutically acceptable carrier, adjuvant or diluent.

Further aspects of the invention relate to an immunoglobulin single variable domain as described above or a polypeptide as described above for use as contrast agent in non-invasive in vivo medical imaging; for use in diagnosis, prognosis and/or treatment of cancer; for use in monitoring the efficacy of cancer therapy. The immunoglobulin single variable domains and the uses as described are based on the characteristic that these immunoglobulin single variable domains specifically target MMR-positive tumor-associated macrophages (TAMs) inside a tumor.

Then, also envisaged is a method for producing an immunoglobulin single variable domain as described above or a polypeptide as described above, said method comprising the steps of:
- expressing, in a suitable host cell or a suitable expression system, a nucleic acid sequence encoding an immunoglobulin single variable domain or a polypeptide as described above; and optionally
- isolating and/or purifying the immunoglobulin single variable domain or the polypeptide.

Objects of the present invention will be clear from the description that follows.

### BRIEF DESCRIPTION OF THE DRAWINGS

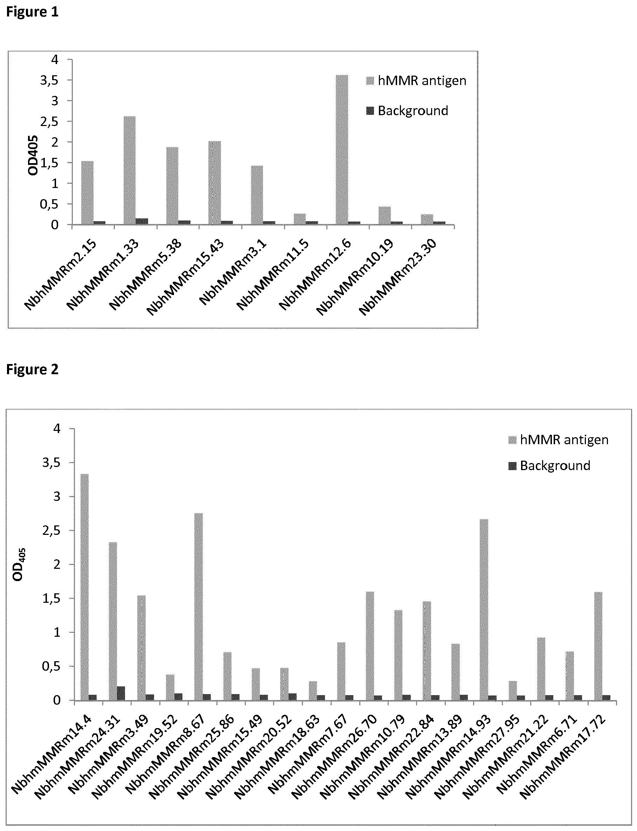

**Figure 1****. PE-ELISA on human MMR.** Summary of the selected anti-human MMR Nb clones. A clone was selected when the OD405nm was at least 3 times higher on specific antigen as compared to irrelevant milk blocking proteins.
**Figure 2****. PE-ELISA on human MMR.** Summary of the selected anti-human/mouse MMR cross-reactive Nb clones. A clone was selected when the OD405nm was at least 3 times higher on specific antigen as compared to irrelevant milk blocking proteins.

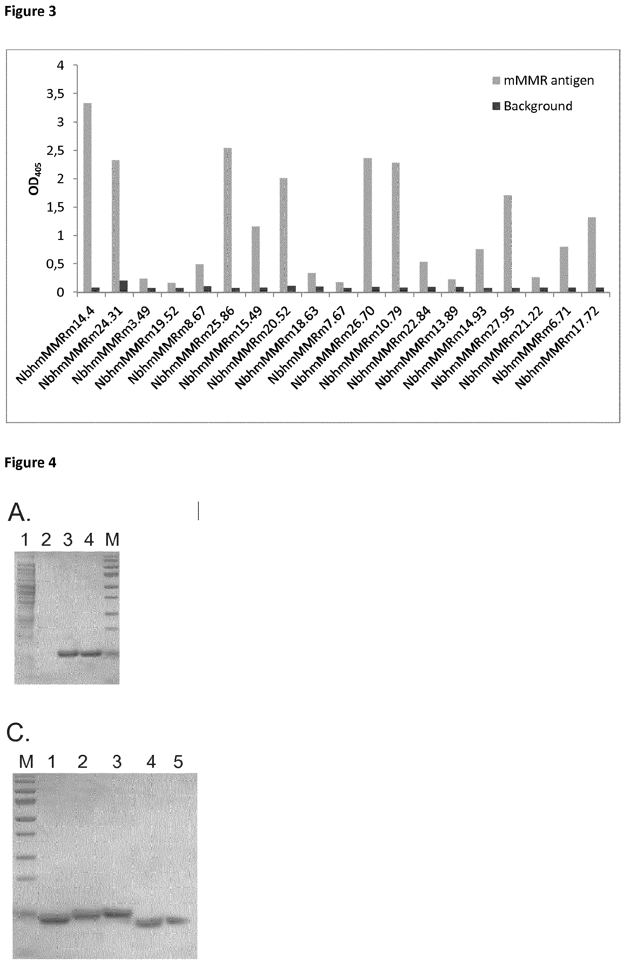

**Figure 3****. PE-ELISA on mouse MMR.** Summary of the selected anti-human/mouse MMR cross-reactive Nb clones. A clone was selected when the OD405nm was at least 2 times higher on specific antigen as compared to irrelevant milk blocking proteins.

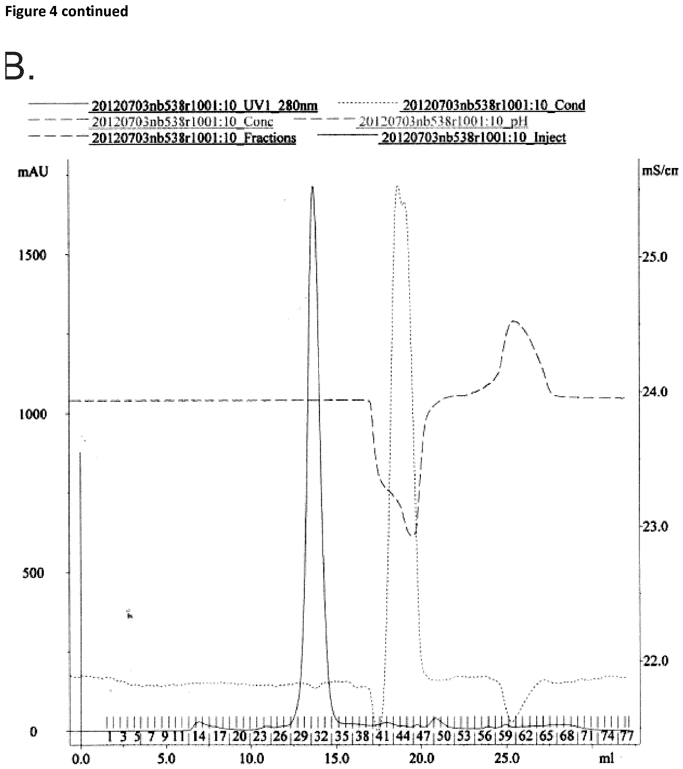

**Figure 4****. Purification of a number of selected Nb clones. A.** Coomassie stained 12% SDS-PAGE gel loaded with protein fractions after IMAC purification of NbhmMMRm5.38 periplasmic extract. Lane 1, column flowthrough, lane 2, wash fraction, lane 3 and 4, elution fractions, M indicates a molecular weight ladder. **B.** Chromatogram of IMAC purified Nb5.38 samples run on a S75 gelfiltration column in PBS. Solid line depicts the OD 280nm, dotted line depicts conductivity, dashed line depicts pH. Only fractions in the main Nb peak around fraction 30 were withheld for further experiments. **C.** Coomassie stained 12% SDS-PAGE gel loaded with protein fractions after gelfiltration of NbhmMMRm3.1 (lane 1), NbhmMMRm14.4 (lane 2), NbhmMMRm5.38 (lane 3), NbhmMMRm26.70 (lane 4) and NbhmMMRm3.49 (lane 5). M indicates a molecular weight ladder. All Nbs were confirmed to be >95% pure and have sizes of 13-15 kDa.

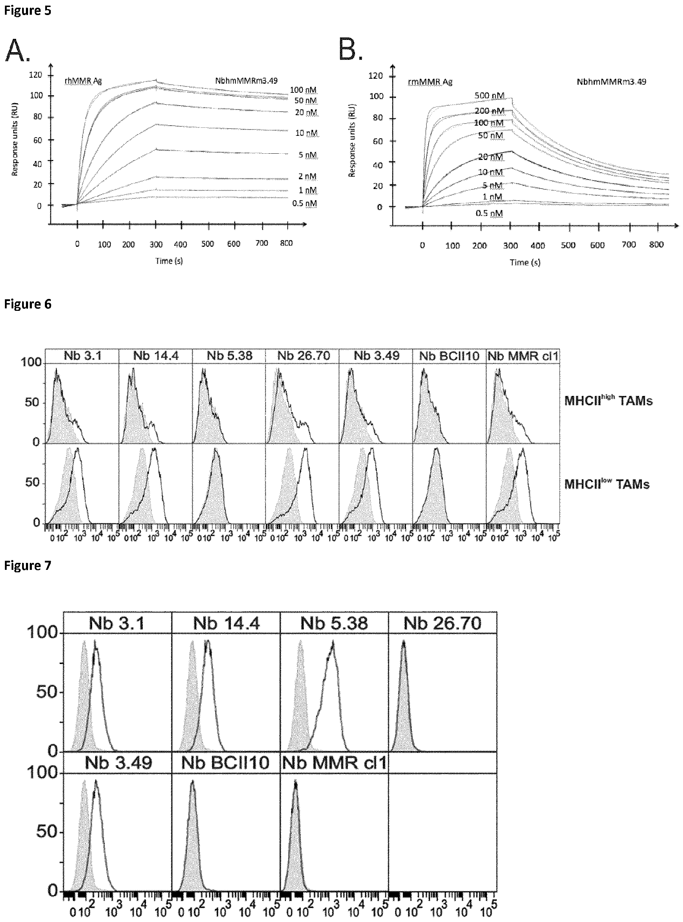

**Figure 5****. Surface Plasmon resonance sensograms of NbhmMMRm3.49 binding to recombinant human and mouse MMR.** NbhmMMRm3.49 was injected in multiple concentrations at 30 µl/min over a CM5 sensorchip coated with 3500RU of recombinant human **(A)** or mouse **(B)** MMR. The sonograms depict the association and dissociation phase over a period of 800s.
**Figure 6****: MMR specific Nbs bind to mouse MMR expressed on ex vivo isolated macrophages.** 3LL-R tumors were induced by injecting 3x10⁶ cancer cells subcutaneously in C57BI/6 mice. After 15 days of tumor growth, the tumors were isolated and single cell suspensions were prepared to be analyzed by flow cytometry. The CD11b+Ly6G- tumor associated macrophages (TAM) were further gated on MHCII expression. The histograms depict MMR expression as defined by Nb binding on MHCII^{low} and MHCII^{high} TAMs. Shaded histograms depict binding of the negative control Nb BCII10. The anti-mouse MMR Nanobody clone 1 (SEQ ID NO: 247) was used as a positive control.
**Figure 7****: MMR specific Nbs bind to human MMR expressed on induced dendritic cells.** Anti-hMMR Nbs bind to CD11c+ subsets in human iDC single-cell suspensions. Shaded histograms depict binding of the negative control Nb BCII10. As expected, the anti-mouse MMR Nanobody clone 1 (SEQ ID NO: 247) does not bind to human MMR.

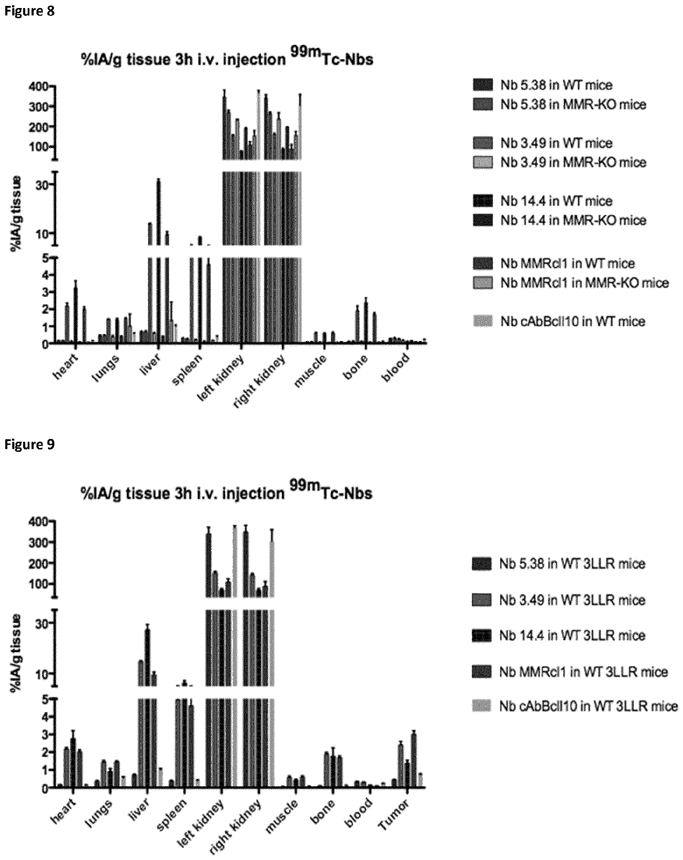

**Figure 8****: Tissue distribution of MMR Nbs in WT versus MMR Knock out C57/bl6 mice.** Anti-MMR Nbs were labelled with ^{99m}Tc and injected in the tail vein of C57/bl6 mice (n=3). After 3h, the mice were dissected and radioactivity was measured in the major organs. The uptake values for the negative control Nb cAbBcII10 served as a measure for general aspecific Nb distribution. The anti-mouse MMR Nanobody clone 1 (SEQ ID NO: 247) was used as a positive control.
**Figure 9****: Tissue distribution of MMR Nbs in 3LL tumor bearing C57/bl6 mice.** 3LL-R tumors were induced by injecting 3x10⁶ cancer cells subcutaneously in C57BI/6 mice. Anti-MMR Nbs were labelled with ^{99m}Tc and injected in the tail vein of the mice (n=3). After 3h, the mice were dissected and radioactivity was measured in the major organs. The uptake values for the negative control Nb cAbBcII10 served as a measure for general aspecific Nb distribution. The anti-mouse MMR Nanobody clone 1 (SEQ ID NO: 247) was used as a positive control.

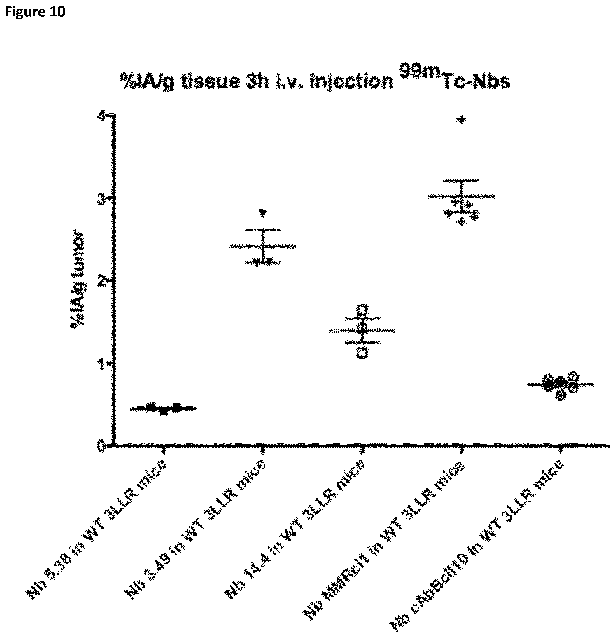

**Figure 10****: Tumor targeting of MMR Nbs in 3LL tumor bearing C57/bl6 mice.** 3LL-R tumors were induced by injecting 3x10⁶ cancer cells subcutaneously in C57BI/6 mice. Anti-MMR Nbs were labelled with ^{99m}Tc and injected in the tail vein of the mice (n=3). After 3h, the mice were dissected and radioactivity of the dissected tumor was measured. The uptake values for the negative control Nb cAbBcII10 served as a measure for general aspecific Nb distribution. The anti-mouse MMR Nanobody clone 1 (SEQ ID NO: 247) was used as a positive control.

### DETAILED DESCRIPTION

The present application describes particular embodiments and refers to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Unless otherwise defined herein, scientific and technical terms and phrases used in connection with the described matter herein shall have the meanings that are commonly understood by those of ordinary skill in the art. Generally, nomenclatures used in connection with, and techniques of molecular and cellular biology, structural biology, biophysics, pharmacology, genetics and protein and nucleic acid chemistry described herein are those well-known and commonly used in the art. The methods and techniques of the specification are generally performed according to conventional methods well known in the art and as described in various general and more specific references that are cited and discussed throughout the present specification unless otherwise indicated. See, for example, Sambrook et al. Molecular Cloning: A Laboratory Manual, 3th ed., Cold Spring Harbor Laboratory Press, Cold Spring Harbor, N.Y. (2001); Ausubel et al., Current Protocols in Molecular Biology, Greene Publishing Associates (1992, and Supplements to 2002); Rup, Biomolecular crystallography: principles, Practice and Applications to Structural Biology, 1st edition, Garland Science, Taylor & Francis Group, LLC, an informa Business, N.Y. (2009); Limbird, Cell Surface Receptors, 3d ed., Springer (2004).

As used herein, the terms "polypeptide", "protein", "peptide" are used interchangeably herein, and refer to a polymeric form of amino acids of any length, which can include coded and non-coded amino acids, chemically or biochemically modified or derivatized amino acids, and polypeptides having modified peptide backbones.

As used herein, the terms "nucleic acid molecule", "polynucleotide", "polynucleic acid", "nucleic acid" are used interchangeably and refer to a polymeric form of nucleotides of any length, either deoxyribonucleotides or ribonucleotides, or analogs thereof. Polynucleotides may have any three-dimensional structure, and may perform any function, known or unknown. Non-limiting examples of polynucleotides include a gene, a gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, control regions, isolated RNA of any sequence, nucleic acid probes, and primers. The nucleic acid molecule may be linear or circular.

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. Determining the percentage of sequence identity can be done manually, or by making use of computer programs that are available in the art. Examples of useful algorithms are PILEUP (Higgins & Sharp, CABIOS 5:151 (1989), BLAST and BLAST 2.0 (Altschul et al. J. Mol. Biol. 215: 403 (1990). Software for performing BLAST analyses is publicly available through the National Center for Biotechnology Information (http://www.ncbi.nlm.nih.gov/).

A "deletion" is defined here as a change in either amino acid or nucleotide sequence in which one or more amino acid or nucleotide residues, respectively, are absent as compared to an amino acid sequence or nucleotide sequence of a parental polypeptide or nucleic acid. Within the context of a protein, a deletion can involve deletion of about 2, about 5, about 10, up to about 20, up to about 30 or up to about 50 or more amino acids. A protein or a fragment thereof may contain more than one deletion.

An "insertion" or "addition" is that change in an amino acid or nucleotide sequences which has resulted in the addition of one or more amino acid or nucleotide residues, respectively, as compared to an amino acid sequence or nucleotide sequence of a parental protein. "Insertion" generally refers to addition to one or more amino acid residues within an amino acid sequence of a polypeptide, while "addition" can be an insertion or refer to amino acid residues added at an N- or C-terminus, or both termini. Within the context of a protein or a fragment thereof, an insertion or addition is usually of about 1, about 3, about 5, about 10, up to about 20, up to about 30 or up to about 50 or more amino acids. A protein or fragment thereof may contain more than one insertion.

A "substitution", as used herein, results from the replacement of one or more amino acids or nucleotides by different amino acids or nucleotides, respectively as compared to an amino acid sequence or nucleotide sequence of a parental protein or a fragment thereof. It is understood that a protein or a fragment thereof may have conservative amino acid substitutions which have substantially no effect on the protein's activity. By conservative substitutions is intended combinations such as gly, ala; val, ile, leu, met; asp, glu; asn, gln; ser, thr; lys, arg; cys, met; and phe, tyr, trp.

A first aspect of the invention relates to an immunoglobulin single variable domain that is directed against and/or specifically binds to human macrophage mannose receptor (SEQ ID NO: 1).

The term "macrophage mannose receptor" (MMR), as used herein, is known in the art and refers to a type I transmembrane protein, first identified in mammalian tissue macrophages and later in dendritic cells and a variety of endothelial and epithelial cells. Macrophages are central actors of the innate and adaptive immune responses. They are disseminated throughout most organs to protect against entry of infectious agents by internalizing and most of the time, killing them. Among the surface receptors present on macrophages, the mannose receptor recognizes a variety of molecular patterns generic to microorganisms. The MMR is composed of a single subunit with N- and O-linked glycosylations and consists of five domains: an N-terminal cysteine-rich region, which recognizes terminal sulfated sugar residues; a fibronectin type II domain with unclear function; a series of eight C-type, lectin-like carbohydrate recognition domains (CRDs) involved in Ca²⁺-dependent recognition of mannose, fucose, or *N*-acetylglucosamine residues on the envelop of pathogens or on endogenous glycoproteins with CRDs 4-8 showing affinity for ligands comparable with that of intact MR; a single transmembrane domain; and a 45 residue-long cytoplasmic tail that contains motifs critical for MR-mediated endocytosis and sorting in endosomes (Chieppa et al. 2003, J Immunol 171:4552-60). In particular, the human macrophage mannose receptor is known as Mrc1 or CD206 (accession number nucleotide sequence: NM_002438.2; accession number protein sequence: NP_002429.1 and as in SEQ ID NO: 1).

The specification in its broadest sense is not particularly limited to or defined by a specific antigenic determinant, epitope, part, domain, subunit or conformation of human MMR (SEQ ID NO: 1) against which the described immunoglobulin single variable domains are directed. It is expected that the immunoglobulin single variable domains according to the specification will generally bind to all naturally occurring or synthetic analogs, variants, mutants, alleles of the MMRs mentioned herein.

The human macrophage mannose receptor as referred to in the present specification includes fragments of the full length human MMR protein. A non-limiting example of a fragment of the full length MMR protein includes the ectodomain of a particular MMR. The "ectodomain" as used herein, refers to a fragment of the MMR containing an N-terminus that is cysteine-rich, followed by a fibronectin type II domain and eight carbohydrate recognition domains (CRDs). All of the eight CRDs are particularly well conserved, especially CRD4. The ectodomain of the human macrophage mannose receptor is defined as the AA 19 - AA 1383 fragment (SEQ ID NO: 5) of the corresponding full length mouse MMR amino acid sequence as defined in NP_002429.1 (SEQ ID NO: 1), see also Table 7. Thus, the specification describes immunoglobulin single variable domains that specifically bind to the ectodomain of the human macrophage mannose receptor (SEQ ID NO: 5).

As used herein, the term "specifically recognizing" or "specifically binding to" or simply "specific for" refers to the ability of an immunoglobulin single variable domain to preferentially bind to a particular antigen that is present in a homogeneous mixture of different antigens and does not necessarily imply high affinity (as defined further herein). The specification describes a specific binding interaction that will discriminate between desirable and undesirable antigens in a sample, and the specification describes that the specific binding interaction of more than about 10 to 100-fold or more (e.g., more than about 1000- or 10,000-fold). The terms "specifically bind", "selectively bind", "preferentially bind", and grammatical equivalents thereof, are used interchangeably herein.

The term "affinity", as used herein, refers to the degree to which an immunoglobulin single variable domain binds to an antigen so as to shift the equilibrium of antigen and immunoglobulin single variable domain toward the presence of a complex formed by their binding. Thus, for example, where an antigen and antibody (fragment) are combined in relatively equal concentration, an antibody (fragment) of high affinity will bind to the available antigen so as to shift the equilibrium toward high concentration of the resulting complex. The dissociation constant is commonly used to describe the affinity between the antibody (fragment) and the antigenic target. Typically, the dissociation constant is lower than 10⁻⁵ M. Preferably, the dissociation constant is lower than 10⁻⁶ M, more preferably, lower than 10⁻⁷ M. Most preferably, the dissociation constant is lower than 10⁻⁸ M.

An immunoglobulin single variable domain that can specifically bind to and/or that has affinity for a specific antigen or antigenic determinant (e.g. epitope) is said to be "against" or "directed against" said antigen or antigenic determinant. An immunoglobulin single variable domain as described herein is said to be "cross-reactive" for two different antigens or antigenic determinants (such as macrophage mannose receptor from two different species of mammal, such as human MMR and mouse MMR) if it is specific for both these different antigens or antigenic determinants.

It will be appreciated that, as described herein, immunoglobulin single variable domains that are directed against the human macrophage mannose receptor from one species may or may not show cross-reactivity with the macrophage mannose receptor from another species. For example, immunoglobulin single variable domains directed against human MMR, in particular human MMR (SEQ ID NO: 1) may or may not show cross-reactivity with MMR from one or more other species of animals that are often used in animal models for diseases (for example, mouse, rat, rabbit, pig or dog). It will be clear to the skilled person that such cross-reactivity, when present, may have advantages for diagnostic and/or therapeutic development, since it allows the immunoglobulin single variable domains to be tested in such disease models.

A non-limiting example of a non-human MMR includes the mouse MMR (synonyms: MRC1 or CD206; accession number nucleotide sequence: NM_008625.2; accession number protein sequence: NP_032651.2 and as in SEQ ID NO: 3). Also, a non-limiting example of a fragment of a non-human MMR includes the ectodomain of the mouse macrophage mannose receptor, which is defined as the AA 19 - AA 1388 fragment (SEQ ID NO: 6) of the corresponding full length mouse MMR amino acid sequence as defined in NP_032651.2 (SEQ ID NO: 3). The deduced amino acid sequence of mouse mannose receptor has an overall 82% homology with the human mannose receptor, as can be easily measured in a BLASTp alignment (Altschul et al. 1990, J Mol Biol 215: 403-10).

The term "immunoglobulin single variable domain" defines molecules wherein the antigen binding site is present on, and formed by, a single immunoglobulin domain (which is different from conventional immunoglobulins or their fragments, wherein typically two immunoglobulin variable domains interact to form an antigen binding site). It should however be clear that the term "immunoglobulin single variable domain" does comprise fragments of conventional immunoglobulins wherein the antigen binding site is formed by a single variable domain.

Generally, an immunoglobulin single variable domain will have an amino acid sequence comprising 4 framework regions (FR1 to FR4) and 3 complementarity determining regions (CDR1 to CDR3), preferably according to the following formula (1): FR1-CDR1-FR2-CDR2-FR3-CDR3-FR4 (1), or any suitable fragment thereof (which will then usually contain at least some of the amino acid residues that form at least one of the complementarity determining regions).

Immunoglobulin single variable domains comprising 4 FRs and 3 CDRs are known to the person skilled in the art and have been described, as a non-limiting example, in Wesolowski et al. 2009, Med Microbiol Immunol 198: 157-174.

Typical, but non-limiting, described are examples of immunoglobulin single variable domains including light chain variable domain sequences (e.g. a VL domain sequence) or a suitable fragment thereof, or heavy chain variable domain sequences (e.g. a VH domain sequence or VHH domain sequence) or a suitable fragment thereof, as long as it is capable of forming a single antigen binding unit. Thus, the specification describes immunoglobulin single variable domain being a light chain variable domain sequence (e.g. a VL domain sequence) or a heavy chain variable domain sequence (e.g. a VH domain sequence); more specifically, the immunoglobulin single variable domain being a heavy chain variable domain sequence that is derived from a conventional four-chain antibody or a heavy chain variable domain sequence that is derived from a heavy chain antibody. The immunoglobulin single variable domain may be a domain antibody, or a single domain antibody, or a "dAB" or dAb, or a Nanobody (as defined herein), or another immunoglobulin single variable domain, or any suitable fragment of any one thereof. For a general description of single domain antibodies, reference is made to the following book: "Single domain antibodies", Methods in Molecular Biology, Eds. Saerens and Muyldermans, 2012, Vol 911. The immunoglobulin single variable domains, generally comprise a single amino acid chain that can be considered to comprise 4 "framework sequences" or FR's and 3 "complementary determining regions" or CDR's (as defined hereinbefore). It should be clear that framework regions of immunoglobulin single variable domains may also contribute to the binding of their antigens (Desmyter et al 2002, J Biol Chem 277: 23645-50; Korotkov et al. 2009, Structure 17: 255-65). The delineation of the CDR sequences (and thus also the FR sequences) can be based on the IMGT unique numbering system for V-domains and V-like domains (Lefranc et al. 2003, Develop Comparat Immunol 27: 55-77). Alternatively, the delineation of the FR and CDR sequences can be done by using the Kabat numbering system as applied to VHH domains from Camelids in the article of Riechmann and Muyldermans 2000, J Immunol Methods 240: 185-195.

It should be noted that the immunoglobulin single variable domains as binding domain moiety in their broadest sense are not limited to a specific biological source or to a specific method of preparation. The term "immunoglobulin single variable domain" encompasses variable domains of different origin, comprising mouse, rat, rabbit, donkey, human, shark, camelid variable domains. According to the specification, the immunoglobulin single variable domains are derived from shark antibodies (the so-called immunoglobulin new antigen receptors or IgNARs), more specifically from naturally occurring heavy chain shark antibodies, devoid of light chains, and are known as VNAR domain sequences. Preferably described herein, the immunoglobulin single variable domains are derived from camelid antibodies. More preferably, the immunoglobulin single variable domains are derived from naturally occurring heavy chain camelid antibodies, devoid of light chains, and are known as VHH domain sequences or Nanobodies.

The term "Nanobody" (Nb), as used herein, is a single domain antigen binding fragment. It particularly refers to a single variable domain derived from naturally occurring heavy chain antibodies and is known to the person skilled in the art. Nanobodies are usually derived from heavy chain only antibodies (devoid of light chains) seen in camelids (Hamers-Casterman et al. 1993, Nature 363: 446-448; Desmyter et al. 1996, Nat Struct Biol 803-811) and consequently are often referred to as VHH antibody or VHH sequence. Camelids comprise old world camelids (*Camelus bactrianus* and *Camelus dromedarius*) and new world camelids (for example *Lama paccos, Lama glama, Lama guanicoe* and *Lama vicugna*)*.* Nanobody® and Nanobodies® are registered trademarks of Ablynx N.V. (Belgium). For a further description of VHH's or Nanobodies, reference is made to the book "Single domain antibodies", Methods in Molecular Biology, Eds. Saerens and Muyldermans, 2012, Vol 911, in particular to the Chapter by Vincke and Muyldermans (2012), as well as to a non-limiting list of patent applications, which are mentioned as general background art, and include: WO 94/04678, WO 95/04079, WO 96/34103 of the Vrije Universiteit Brussel; WO 94/25591, WO 99/37681, WO 00/40968, WO 00/43507, WO 00/65057, WO 01/40310, WO 01/44301, EP 1 134 231 and WO 02/48193 of Unilever; WO 97/49805, WO 01/21817, WO 03/035694, WO 03/054016 and WO 03/055527 of the Vlaams Instituut voor Biotechnologie (VIB); WO 04/041867, WO 04/041862, WO 04/041865, WO 04/041863, WO 04/062551, WO 05/044858, WO 06/40153, WO 06/079372, WO 06/122786, WO 06/122787 and WO 06/122825, by Ablynx N.V. and the further published patent applications by Ablynx N.V. The small size and unique biophysical properties of Nbs excel conventional antibody fragments for the recognition of uncommon or hidden epitopes and for binding into cavities or active sites of protein targets. Further, Nbs can be designed as multi-specific and multivalent antibodies (as defined further herein) or attached to reporter molecules (Conrath et al. 2011, Antimicrob Agents Chemother 45: 2807-2812). Nbs are stable, survive the gastro-intestinal system and can easily be manufactured. Therefore, Nbs can be used in many applications including drug discovery and therapy, but also as a versatile and valuable tool for purification, functional study and crystallization of proteins (Saerens et al. 2008, Curr Opin Pharmacol 8: 600-608).

The Nanobodies described herein generally comprise a single amino acid chain that can be considered to comprise 4 "framework regions" or FR's and 3 "complementarity determining regions" or CDR's, according to formula (1) (as define above). The term "complementarity determining region" or "CDR" refers to variable regions in nanobodies and contains the amino acid sequences capable of specifically binding to antigenic targets. These CDR regions account for the basic specificity of the Nanobody for a particular antigenic determinant structure. Such regions are also referred to as "hypervariable regions." The nanobodies have 3 CDR regions, each non-contiguous with the others (termed CDR1, CDR2, CDR3). The delineation of the FR and CDR sequences is often based on the IMGT unique numbering system for V-domains and V-like domains (Lefranc et al. 2003, Develop Comparat Immunol 27: 55-77). Alternatively, the delineation of the FR and CDR sequences can be done by using the Kabat numbering system as applied to V_{H}H domains from Camelids in the article of Riechmann and Muyldermans 2000, J Immunol Methods 240: 185-195. As will be known by the person skilled in the art, the nanobodies can in particular be characterized by the presence of one or more Camelidae hallmark residues in one or more of the framework sequences (according to Kabat numbering), as described for example in WO 08/020079, on page 75, Table A-3.

The specification describes an immunoglobulin single variable domain immunoglobulin single variable domain that is directed against and/or that specifically binds to human macrophage mannose receptor (SEQ ID NO: 1), wherein the immunoglobulin single variable domain comprises an amino acid sequence that comprises 4 framework regions (FR) and 3 complementarity determining regions (CDR) according to the following formula (1):

FR1-CDR1-FR2-CDR2-FR3-CDR3-FR4 (1),

and wherein CDR1 is chosen from the group consisting of:
a. SEQ ID NOs: 67-96,
b. Polypeptides that have at least 80% amino acid identity with SEQ ID NOs: 67-96,
c. Polypeptides that have 1, 2 or 3 amino acid difference with SEQ ID NOs: 67-96,
and wherein CDR2 is chosen from the group consisting of:
a. SEQ ID NOs: 127-156,
b. Polypeptides that have at least 80% amino acid identity with SEQ ID NOs: 127-156,
c. Polypeptides that have 1, 2 or 3 amino acid difference with SEQ ID NOs: 127-156,
and wherein CDR3 is chosen from the group consisting of:
a. SEQ ID NOs: 187-216,
b. Polypeptides that have at least 80% amino acid identity with SEQ ID NOs: 187-216,
c. Polypeptides that have 1, 2 or 3 amino acid difference with SEQ ID NOs: 187-216,

More specifically, the framework regions (FRs) of the immunoglobulin single variable domains as described hereinabove have an amino acid sequence identity of more than 80% with the FRs of SEQ ID Nos: 37-66 (FR1), SEQ ID Nos: 97-126 (FR2), SEQ ID Nos: 157-186 (FR3), SEQ ID Nos: 217-246 (FR4).

Non-limiting examples of immunoglobulin single variable domains are as described herein and include anti-human and cross-reactive anti-human/anti-mouse MMR nanobodies, for example in Table 1, in particular SEQ ID Nos: 8, 10-29; in Table 2, in particular SEQ ID NOs: 7, 9, 20-36. The specification also describes the nanobodies that may comprise at least one of the complementarity determining regions (CDRs) as described herein, for example CDRs with an amino acid sequence selected from SEQ ID NOs: 67-96, 127-156, 187-216 (see Table 6). Preferably, the nanobodies as described herein comprise a CDR1, a CDR2 and a CDR3 selected from the group consisting of SEQ ID NOs: 67-96, 127-156, 187-216, according to the above described formula (1). Preferably, a nanobody is provided comprising an amino acid sequence according to formula (1) with a CDR1 consisting of SEQ ID NO: 67, a CDR2 consisting of SEQ ID NO: 127, a CDR3 consisting of SEQ ID NO: 187, or with polypeptides that have at least 80% amino acid identity with SEQ ID NO: 67, SEQ ID NO: 127, SEQ ID NO: 187. More specifically, the nanobodies can be selected from the group comprising SEQ ID NOs: 7-36, or a functional fragment thereof. A "functional fragment" or a "suitable fragment", as used herein, may for example comprise one of the CDR loops. Preferably, said functional fragment comprises CDR3. More specifically described herein, said nanobodies consist of any of SEQ ID NOs: 7-36, preferably SEQ ID NOs: 7, 8, 9, 10, most preferably SEQ ID NO: 7.

It should be noted that the term Nanobody as used herein in its broadest sense is not limited to a specific biological source or to a specific method of preparation. For example, the nanobodies described herein can generally be obtained: (1) by isolating the V_{H}H domain of a naturally occurring heavy chain antibody; (2) by expression of a nucleotide sequence encoding a naturally occurring V_{H}H domain; (3) by "humanization" of a naturally occurring V_{H}H domain or by expression of a nucleic acid encoding a such humanized V_{H}H domain; (4) by "camelization" of a naturally occurring VH domain from any animal species, and in particular from a mammalian species, such as from a human being, or by expression of a nucleic acid encoding such a camelized VH domain; (5) by "camelisation" of a "domain antibody" or "Dab" as described in the art, or by expression of a nucleic acid encoding such a camelized VH domain; (6) by using synthetic or semi-synthetic techniques for preparing proteins, polypeptides or other amino acid sequences known per se; (7) by preparing a nucleic acid encoding a Nanobody using techniques for nucleic acid synthesis known per se, followed by expression of the nucleic acid thus obtained; and/or (8) by any combination of one or more of the foregoing.

One described class of nanobodies corresponds to the V_{H}H domains of naturally occurring heavy chain antibodies directed against a macrophage mannose receptor, preferably against a human macrophage mannose receptor. As further described herein, such V_{H}H sequences can generally be generated or obtained by suitably immunizing a species of Camelid with a desired MMR, (i.e. so as to raise an immune response and/or heavy chain antibodies directed against a desired MMR), by obtaining a suitable biological sample from said Camelid (such as a blood sample, or any sample of B-cells), and by generating V_{H}H sequences directed against the MMR, starting from said sample, using any suitable technique known per se. Such techniques will be clear to the skilled person. Alternatively, such naturally occurring V_{H}H domains against MMR can be obtained from naive libraries of Camelid V_{H}H sequences, for example by screening such a library using MMR or at least one part, fragment, antigenic determinant or epitope thereof using one or more screening techniques known per se. Such libraries and techniques are for example described in WO9937681, WO0190190, WO03025020 and WO03035694. Alternatively, improved synthetic or semi-synthetic libraries derived from naive V_{H}H libraries may be used, such as V_{H}H libraries obtained from naive V_{H}H libraries by techniques such as random mutagenesis and/or CDR shuffling, as for example described in WO0043507. Yet another technique for obtaining V_{H}H sequences directed against a desired MMR involves suitably immunizing a transgenic mammal that is capable of expressing heavy chain antibodies (i.e. so as to raise an immune response and/or heavy chain antibodies directed against a MMR), obtaining a suitable biological sample from said transgenic mammal (such as a blood sample, or any sample of B-cells), and then generating V_{H}H sequences directed against the MMR starting from said sample, using any suitable technique known per se. For example, for this purpose, the heavy chain antibody-expressing mice and the further methods and techniques described in WO02085945 and in WO04049794 can be used.

Accordingly, the specification encompasses methods of generating immunoglobulin single variable domains as described herein. As a non-limiting example, a method is provided of generating nanobodies directed against or specifically binding to the human macrophage mannose receptor (as described herein), comprising
(i) immunizing an animal with a MMR, in particular a human MMR (e.g. SEQ ID NOs: 1 or 2), or a fragment thereof (e.g. SEQ ID NO: 5); and
(ii) screening for nanobodies specifically binding to human MMR.

For the immunization of an animal with a MMR, the MMR may be produced and purified using conventional methods that may employ expressing a recombinant form of the MMR in a host cell, and purifying the MMR using affinity chromatography and/or antibody-based methods. Any suitable animal, e.g., a warm-blooded animal, in particular a mammal such as a rabbit, mouse, rat, camel, sheep, cow, shark, or pig or a bird such as a chicken or turkey, may be immunized using any of the techniques well known in the art suitable for generating an immune response. The screening for nanobodies, as a non-limiting example, specifically binding to a MMR may for example be performed by screening a set, collection or library of cells that express heavy chain antibodies on their surface (e.g. B-cells obtained from a suitably immunized Camelid), or bacteriophages that display a fusion of genlll and Nanobody at their surface, by screening of a (naive or immune) library of V_{H}H sequences or Nanobody sequences, or by screening of a (naive or immune) library of nucleic acid sequences that encode VHH sequences or Nanobody sequences, which may all be performed in a manner known per se, and which method may optionally further comprise one or more other suitable steps, such as, for example and without limitation, a step of affinity maturation, a step of expressing the desired amino acid sequence, a step of screening for binding and/or for activity against the desired antigen (in this case, the MMR), a step of determining the desired amino acid sequence or nucleotide sequence, a step of introducing one or more humanizing substitutions, a step of formatting in a suitable multivalent and/or multispecific format, a step of screening for the desired biological and/or physiological properties (i.e. using a suitable assay known in the art), and/or any combination of one or more of such steps, in any suitable order.

A particularly preferred class of immunoglobulin single variable domains described herein comprises nanobodies with an amino acid sequence that corresponds to the amino acid sequence of a naturally occurring V_{H}H domain, but that has been "humanized", i.e. by replacing one or more amino acid residues in the amino acid sequence of said naturally occurring V_{H}H sequence (and in particular in the framework sequences) by one or more of the amino acid residues that occur at the corresponding position(s) in a VH domain from a conventional 4-chain antibody from a human being. This can be performed in a manner known per se, which will be clear to the skilled person, on the basis of the further description herein and the prior art on humanization. Again, it should be noted that such humanized nanobodies as described herein can be obtained in any suitable manner known per se (i.e. as indicated under points (1) - (8) above) and thus are not strictly limited to polypeptides that have been obtained using a polypeptide that comprises a naturally occurring V_{H}H domain as a starting material. Humanized nanobodies may have several advantages, such as a reduced immunogenicity, compared to the corresponding naturally occurring V_{H}H domains. Such humanization generally involves replacing one or more amino acid residues in the sequence of a naturally occurring V_{H}H with the amino acid residues that occur at the same position in a human VH domain, such as a human VH3 domain. The humanizing substitutions should be chosen such that the resulting humanized nanobodies still retain the favourable properties of nanobodies as defined herein. The skilled person will be able to select humanizing substitutions or suitable combinations of humanizing substitutions which optimize or achieve a desired or suitable balance between the favourable properties provided by the humanizing substitutions on the one hand and the favourable properties of naturally occurring V_{H}H domains on the other hand.

Another particularly preferred class of immunoglobulin single variable domains as described herein comprises nanobodies with an amino acid sequence that corresponds to the amino acid sequence of a naturally occurring VH domain, but that has been "camelized", i.e. by replacing one or more amino acid residues in the amino acid sequence of a naturally occurring VH domain from a conventional 4-chain antibody by one or more of the amino acid residues that occur at the corresponding position(s) in a VHH domain of a heavy chain antibody. Such "camelizing" substitutions are preferably inserted at amino acid positions that form and/or are present at the VH-VL interface, and/or at the so-called Camelidae hallmark residues, as defined herein (see for example WO 9404678, WO 08/020079). Preferably, the VH sequence that is used as a starting material or starting point for generating or designing the camelized Nanobody is preferably a VH sequence from a mammal, more preferably the VH sequence of a human being, such as a VH3 sequence. However, it should be noted that such camelized nanobodies as described herein can be obtained in any suitable manner known per se (i.e. as indicated under points (1) - (8) above) and thus are not strictly limited to polypeptides that have been obtained using a polypeptide that comprises a naturally occurring VH domain as a starting material.

For example, both "humanization" and "camelization" can be performed by providing a nucleotide sequence that encodes a naturally occurring V_{H}H domain or VH domain, respectively, and then changing, in a manner known per se, one or more codons in said nucleotide sequence in such a way that the new nucleotide sequence encodes a "humanized" or "camelized" Nanobody as described herein, respectively. This nucleic acid can then be expressed in a manner known per se, so as to provide the desired Nanobody as described herein. Alternatively, based on the amino acid sequence of a naturally occurring VHH domain or VH domain, respectively, the amino acid sequence of the desired humanized or camelized Nanobody as described herein, respectively, can be designed and then synthesized de novo using techniques for peptide synthesis known per se. Also, based on the amino acid sequence or nucleotide sequence of a naturally occurring V_{H}H domain or VH domain, respectively, a nucleotide sequence encoding the desired humanized or camelized Nanobody as described herein, respectively, can be designed and then synthesized de novo using techniques for nucleic acid synthesis known per se, after which the nucleic acid thus obtained can be expressed in a manner known per se, so as to provide the desired Nanobody as described herein. Other suitable methods and techniques for obtaining the nanobodies as disclosed in this specification and/or nucleic acids encoding the same, starting from naturally occurring VH sequences or preferably VHH sequences, will be clear from the skilled person, and may for example comprise combining one or more parts of one or more naturally occurring VH sequences (such as one or more FR sequences and/or CDR sequences), one or more parts of one or more naturally occurring V_{H}H sequences (such as one or more FR sequences or CDR sequences), and/or one or more synthetic or semi-synthetic sequences, in a suitable manner, so as to provide a Nanobody as described herein or a nucleotide sequence or nucleic acid encoding the same.

Also provided herein are natural or synthetic analogs, mutants, variants, alleles, homologs and orthologs (herein collectively referred to as "variants") of the immunoglobulin single variable domains as disclosed herein. Some particularly preferred, but non-limiting examples of immunoglobulin single variable domains are described herein, as well as combinations of CDR sequences are mentioned in Table 6, which lists the CDR sequences that are present in a number of preferred, but non-limiting immunoglobulin single variable domains as disclosed herein. Thus, the specification describes the term "immunoglobulin single variable domain as disclosed herein" in its broadest sense also covering such variants, in particular variants of the nanobodies of SEQ ID NOs: 7-36 (see Table 1, Table 2). Generally, in such variants, one or more amino acid residues may have been replaced, deleted and/or added, compared to the nanobodies as defined herein. Such substitutions, insertions or deletions may be made in one or more of the framework regions and/or in one or more of the CDR's, and in particular variants of the CDR's of the nanobodies of SEQ ID NOs: 7-36, said CDR's corresponding to SEQ ID NOs: 67-96 (CDR1), SEQ ID Nos: 127-156, SEQ ID Nos: 187-216 (CDR3) (Table 6). Variants, as used herein, are sequences wherein each or any framework region and each or any complementarity determining region shows at least 80% identity, preferably at least 85% identity, more preferably 90% identity, even more preferably 95% identity or, still even more preferably 99% identity with the corresponding region in the reference sequence (i.e. FR1_variant versus FR1_reference, CDR1_variant versus CDR1_reference, FR2_variant versus FR2_reference, CDR2_variant versus CDR2_reference, FR3_variant versus FR3_reference, CDR3_variant versus CDR3_reference, FR4_variant versus FR4_reference), as can be measured electronically by making use of algorithms such as PILEUP and BLAST (Altschul et al. 1990, J Mol Biol 215: 403; Higgins & Sharp 1989, CABIOS 5: 151). Software for performing BLAST analyses is publicly available through the National Center for Biotechnology Information (http://www/ncbi.nlm.nih.gov/). Such variants of immunoglobulin single variable domains may be of particular advantage since they may have improved potency or other desired properties.

By means of non-limiting examples, a substitution may for example be a conservative substitution (as described herein) and/or an amino acid residue may be replaced by another amino acid residue that naturally occurs at the same position in another V_{H}H domain. Thus, any one or more substitutions, deletions or insertions, or any combination thereof, that either improve the properties of the Nanobody as disclosed herein or that at least do not detract too much from the desired properties or from the balance or combination of desired properties of the Nanobody as disclosed herein (i.e. to the extent that the Nanobody is no longer suited for its intended use) are included within the specification as described herein. A skilled person will generally be able to determine and select suitable substitutions, deletions or insertions, or suitable combinations of thereof, based on the disclosure herein and optionally after a limited degree of routine experimentation, which may for example involve introducing a limited number of possible substitutions and determining their influence on the properties of the nanobodies thus obtained.

The specification further describes variants of the immunoglobulin single variable domains, in particular the nanobodies as disclosed herein may have a substitution, deletion or insertion, of 1, 2 or 3 amino acids in one, two or three of the CDRs, more specifically (i) in CDR1 or CDR2 or CDR3; (ii) in CDR1 and CDR2, or, in CDR1 and CDR3, or, in CDR2 and CDR3; (iii) in CDR1 and CDR2 and CDR3, as listed in Table 6. More preferably, variants of the immunoglobulin single variable domains, in particular the nanobodies, as described herein, may have a conservative substitution (as defined herein) of 1, 2 or 3 amino acids in one, two or three of the CDRs, more specifically (i) in CDR1 or CDR2 or CDR3; (ii) in CDR1 and CDR2, or, in CDR1 and CDR3, or, in CDR2 and CDR3; (iii) in CDR1 and CDR2 and CDR3, as listed in Table 6.

Further, depending on the host organism used to express the immunoglobulin single variable domain as disclosed herein, such deletions and/or substitutions may be designed in such a way that one or more sites for post-translational modification (such as one or more glycosylation sites) are removed, as will be within the ability of the person skilled in the art. Alternatively, substitutions or insertions may be designed so as to introduce one or more sites for attachment of functional groups (as described herein), for example to allow site-specific pegylation.

Examples of modifications, as well as examples of amino acid residues within the immunoglobulin single variable domain, preferably the Nanobody sequence, that can be modified (i.e. either on the protein backbone but preferably on a side chain), methods and techniques that can be used to introduce such modifications and the potential uses and advantages of such modifications will be clear to the skilled person. For example, such a modification may involve the introduction (e.g. by covalent linking or in another suitable manner) of one or more functional groups, residues or moieties into or onto the immunoglobulin single variable domain as described herein, and in particular of one or more functional groups, residues or moieties that confer one or more desired properties or functionalities to the immunoglobulin single variable domain disclosed herein. Examples of such functional groups and of techniques for introducing them will be clear to the skilled person, and can generally comprise all functional groups and techniques mentioned in the general background art cited hereinabove as well as the functional groups and techniques known per se for the modification of pharmaceutical proteins, and in particular for the modification of antibodies or antibody fragments (including ScFv's and single domain antibodies), for which reference is for example made to Remington's Pharmaceutical Sciences, 16th ed., Mack Publishing Co., Easton, PA (1980). Such functional groups may for example be linked directly (for example covalently) to a immunoglobulin single variable domain disclosed herein, or optionally via a suitable linker or spacer, as will again be clear to the skilled person. One of the most widely used techniques for increasing the half-life and/or reducing immunogenicity of pharmaceutical proteins comprises attachment of a suitable pharmacologically acceptable polymer, such as poly(ethyleneglycol) (PEG) or derivatives thereof (such as methoxypoly(ethyleneglycol) or mPEG). Generally, any suitable form of pegylation can be used, such as the pegylation used in the art for antibodies and antibody fragments (including but not limited to (single) domain antibodies and ScFv's); reference is made to for example Chapman, Nat. Biotechnol., 54, 531-545 (2002); by Veronese and Harris, Adv. Drug Deliv. Rev. 54, 453-456 (2003), by Harris and Chess, Nat. Rev. Drug. Discov., 2, (2003) and in WO04060965. Various reagents for pegylation of proteins are also commercially available, for example from Nektar Therapeutics, USA. Preferably, site-directed pegylation is used, in particular via a cysteine-residue (see for example Yang et al., Protein Engineering, 16, 10, 761-770 (2003)). For example, for this purpose, PEG may be attached to a cysteine residue that naturally occurs in a Nanobody as disclosed herein, a Nanobody as defined herein may be modified so as to suitably introduce one or more cysteine residues for attachment of PEG, or an amino acid sequence comprising one or more cysteine residues for attachment of PEG may be fused to the N- and/or C-terminus of a Nanobody as described in the specification, all using techniques of protein engineering known per se to the skilled person. Preferably, for the immunoglobulin single variable domains and proteins as described herein, a PEG is used with a molecular weight of more than 5000, such as more than 10,000 and less than 200,000, such as less than 100,000; for example in the range of 20,000-80,000. Another, usually less preferred modification comprises N-linked or O-linked glycosylation, usually as part of co-translational and/or post-translational modification, depending on the host cell used for expressing the immunoglobulin single variable domain or polypeptide as disclosed herein. Another technique for increasing the half-life of an immunoglobulin single variable domain may comprise the engineering into bifunctional constructs (for example, one Nanobody against the target MMR and one against a serum protein such as albumin) or into fusions of immunoglobulin single variable domains with peptides (for example, a peptide against a serum protein such as albumin).

Yet another modification may comprise the introduction of one or more detectable labels or other signal-generating groups or moieties, depending on the intended use of the labelled Nanobody. Suitable labels and techniques for attaching, using and detecting them will be clear to the skilled person, and for example include, but are not limited to, fluorescent labels (such as IRDye800, VivoTag800, fluorescein, isothiocyanate, rhodamine, phycoerythrin, phycocyanin, allophycocyanin, o-phthaldehyde, and fluorescamine and fluorescent metals such as Eu or others metals from the lanthanide series), phosphorescent labels, chemiluminescent labels or bioluminescent labels (such as luminal, isoluminol, theromatic acridinium ester, imidazole, acridinium salts, oxalate ester, dioxetane or GFP and its analogs ), radio-isotopes, metals, metals chelates or metallic cations or other metals or metallic cations that are particularly suited for use in in vivo, in vitro or in situ diagnosis and imaging, as well as chromophores and enzymes (such as malate dehydrogenase, staphylococcal nuclease, delta-V-steroid isomerase, yeast alcohol dehydrogenase, alpha-glycerophosphate dehydrogenase, triose phosphate isomerase, biotinavidin peroxidase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholine esterase). Other suitable labels will be clear to the skilled person, and for example include moieties that can be detected using NMR or ESR spectroscopy. Such labelled Nanobodies and polypeptides as disclosed herein may for example be used for in vitro, in vivo or in situ assays (including immunoassays known per se such as ELISA, RIA, EIA and other "sandwich assays", etc.) as well as in vivo diagnostic and imaging purposes, depending on the choice of the specific label. As will be clear to the skilled person, another modification may involve the introduction of a chelating group, for example to chelate one of the metals or metallic cations referred to above. Suitable chelating groups for example include, without limitation, 2,2',2"-(10-(2-((2,5-dioxopyrrolidin-1-yl)oxy)-2-oxoethyl)-1,4,7,10-tetraazacyclododecane-1,4,7-triyl)triacetic acid (DOTA), 2,2'-(7-(2-((2,5-dioxopyrrolidin-1-yl)oxy)-2-oxoethyl)-1,4,7-triazonane-1,4-diyl)diacetic acid (NOTA), diethyl- enetriaminepentaacetic acid (DTPA) or ethylenediaminetetraacetic acid (EDTA). Yet another modification may comprise the introduction of a functional group that is one part of a specific binding pair, such as the biotin-(strept)avidin binding pair. Such a functional group may be used to link the Nanobody as described herein to another protein, polypeptide or chemical compound that is bound to the other half of the binding pair, i.e. through formation of the binding pair. For example, a Nanobody as disclosed herein may be conjugated to biotin, and linked to another protein, polypeptide, compound or carrier conjugated to avidin or streptavidin. For example, such a conjugated Nanobody may be used as a reporter, for example in a diagnostic system where a detectable signal-producing agent is conjugated to avidin or streptavidin. Such binding pairs may for example also be used to bind the Nanobody as disclosed herein to a carrier, including carriers suitable for pharmaceutical purposes. One non-limiting example are the liposomal formulations described by Cao and Suresh, Journal of Drug Targetting, 8, 4, 257 (2000). Such binding pairs may also be used to link a therapeutically active agent to the Nanobody as disclosed herein.

Thus, according to a preferred embodiment, the immunoglobulin single variable domain as used in the present invention is coupled or fused to a detectable label, either directly or through a linker. Preferably, the detectable label is a radio-isotope, in particular a radioactive tracer suitable for medical applications, such as in *in vivo* nuclear imaging. Examples include, without the purpose of being limitative, technetium 99m (^{99m}Tc), iodium 123 (¹²³I), zirconium 89 (⁸⁹Zr), iodium 125 (¹²⁵I), indium 111 (¹¹¹In), fluor 18 (¹⁸F), copper 64 (⁶⁴Cu), gallium 67 (⁶⁷Ga), gallium 68 (⁶⁸Ga), and any other radio-isotope which can be used in animals, in particular mouse, rabbit or human. The specification also describes that the detectable label is ^{99m}Tc.

In another preferred embodiment, the immunoglobulin single variable domain as used in the present invention is coupled to or fused to a functional moiety, in particular a therapeutically active agent, either directly or through a linker. As used herein, a "therapeutically active agent" means any molecule that has or may have a therapeutic effect (i.e. curative or stabilizing effect) in the context of treatment of a disease (as described further herein).

The specification further describes that a therapeutically active agent is a disease-modifying agent, which can be a cytotoxic agent, such as a toxin, or a cytotoxic drug, or an enzyme capable of converting a prodrug into a cytotoxic drug, or a radionuclide, or a cytotoxic cell, or which can be a non-cytotoxic agent. Even more preferably, a therapeutically active agent has a curative effect on the disease. Specific, but non-limiting examples of such moieties are described in the Example section. The specification alternatively describes that the therapeutically active agent is not a cytotoxic agent.

As used herein, "linker molecules" or "linkers" are peptides of 1 to 200 amino acids length, and are typically, but not necessarily, chosen or designed to be unstructured and flexible. For instance, one can choose amino acids that form no particular secondary structure. Or, amino acids can be chosen so that they do not form a stable tertiary structure. Or, the amino acid linkers may form a random coil. Such linkers include, but are not limited to, synthetic peptides rich in Gly, Ser, Thr, Gln, Glu or further amino acids that are frequently associated with unstructured regions in natural proteins (Dosztányi, Z., Csizmok, V., Tompa, P., & Simon, I. (2005). IUPred: web server for the prediction of intrinsically unstructured regions of proteins based on estimated energy content. Bioinformatics (Oxford, England), 21(16), 3433-4.).

Thus, according to specific embodiments, the amino acid (AA) linker sequence is a peptide of between 0 and 200 AA, between 0 and 150 AA, between 0 and 100 AA, between 0 and 90 AA, between 0 and 80 AA, between 0 and 70 AA, between 0 and 60 AA, between 0 and 50 AA, between 0 and 40 AA, between 0 and 30 amino acids, between 0 and 20 AA, between 0 and 10 amino acids, between 0 and 5 amino acids. Non-limiting examples of suitable linker sequences include (GS)₅ (GSGSGSGSGS; SEQ ID NO: 248), (GS)₁₀ (GSGSGSGSGSGSGSGSGSGS; SEQ ID NO: 249), (G₄S)₃ (GGGGSGGGGSGGGGS; SEQ ID NO: 250), llama IgG2 hinge (AHHSEDPSSKAPKAPMA; SEQ ID NO: 251) or human IgA hinge (SPSTPPTPSPSTPPAS; SEQ ID NO: 252) linkers. Examples of sequences of short linkers include, but are not limited to, PPP, PP or GS.

For certain applications, it may be advantageous that the linker molecule comprises or consists of one or more particular sequence motifs. For example, a proteolytic cleavage site can be introduced into the linker molecule such that detectable label or moiety can be released. Useful cleavage sites are known in the art, and include a protease cleavage site such as Factor Xa cleavage site having the sequence IEGR (SEQ ID NO: 253), the thrombin cleavage site having the sequence LVPR (SEQ ID NO: 254), the enterokinase cleaving site having the sequence DDDDK (SEQ ID NO: 255), or the PreScission cleavage site LEVLFQGP (SEQ ID NO: 256).

Alternatively, in case the immunoglobulin single variable domain is linked to a detectable label or moiety using chemoenzymatic methods for protein modification, the linker moiety may exist of different chemical entities, depending on the enzymes or the synthetic chemistry that is used to produce the covalently coupled molecule *in vivo* or *in vitro* (reviewed in: Rabuka 2010, Curr Opin Chem Biol 14: 790-796).

The specification further describes the immunoglobulin single variable domains disclosed herein are in a "multivalent" form and are formed by bonding, chemically or by recombinant DNA techniques, together two or more monovalent immunoglobulin single variable domains. Non-limiting examples of multivalent constructs include "bivalent" constructs, "trivalent" constructs, "tetravalent" constructs, and so on. The immunoglobulin single variable domains comprised within a multivalent construct may be identical or different. The specification describes immunoglobulin single variable domains as disclosed herein that are in a "multi-specific" form and are formed by bonding together two or more immunoglobulin single variable domains, of which at least one with a different specificity. Non-limiting examples of multi-specific constructs include "bi-specific" constructs, "tri-specific" constructs, "tetra-specific" constructs, and so on. To illustrate this further, any multivalent or multispecific (as defined herein) immunoglobulin single variable domain disclosed herein may be suitably directed against two or more different epitopes on the same antigen, for example against two or more different parts of the MMR ectodomain; or may be directed against two or more different antigens, for example against MMR and one or more other marker. Preferably, a monovalent immunoglobulin single variable domain as disclosed herein is such that it will bind to the MMR (as described herein) with an affinity less than 500 nM, preferably less than 200 nM, more preferably less than 10 nM, such as less than 500 pM. Multivalent or multispecific immunoglobulin single variable domains disclosed herein may also have (or be engineered and/or selected for) increased avidity and/or improved selectivity for the desired MMR, and/or for any other desired property or combination of desired properties that may be obtained by the use of such multivalent or multispecific immunoglobulin single variable domains.

In a further aspect, the specification provides a polypeptide comprising any of the immunoglobulin single variable domains disclosed herein, either in a monovalent, multivalent or multi-specific form. Thus, polypeptides comprising monovalent, multivalent or multi-specific nanobodies are included here as non-limiting examples.

Another aspect of the specification relates to a nucleic acid sequence encoding an immunoglobulin single variable domain, in particular a Nanobody, or a polypeptide , as described hereinbefore. Further, the specification also envisages expression vectors comprising nucleic acid sequences encoding any of the above immunoglobulin single variable domains or polypeptides, as well as host cells expressing such expression vectors. Suitable expression systems include constitutive and inducible expression systems in bacteria or yeasts, virus expression systems, such as baculovirus, semliki forest virus and lentiviruses, or transient transfection in insect or mammalian cells. Suitable host cells include *E. coli, Lactococcus lactis, Saccharomyces cerevisiae, Schizosaccharomyces pombe, Pichia pastoris,* and the like. Suitable animal host cells include HEK 293, COS, S2, CHO, NSO, DT40 and the like. The cloning, expression and/or purification of the nanobodies can be done according to techniques known by the skilled person in the art.

The specification further relates to a pharmaceutical composition comprising a immunoglobulin single variable domain as disclosed herein, and at least one of a pharmaceutically acceptable carrier, adjuvant or diluent. Preferably, the pharmaceutical composition comprises a therapeutically effective amount of an immunoglobulin single variable domain disclosed herein, and at least one of a pharmaceutically acceptable carrier, adjuvant or diluent.

A 'carrier', or 'adjuvant', in particular a 'pharmaceutically acceptable carrier' or 'pharmaceutically acceptable adjuvant' is any suitable excipient, diluent, carrier and/or adjuvant which, by themselves, do not induce the production of antibodies harmful to the individual receiving the composition nor do they elicit protection. So, pharmaceutically acceptable carriers are inherently non-toxic and nontherapeutic, and they are known to the person skilled in the art. Suitable carriers or adjuvantia typically comprise one or more of the compounds included in the following non-exhaustive list: large slowly metabolized macromolecules such as proteins, polysaccharides, polylactic acids, polyglycolic acids, polymeric amino acids, amino acid copolymers and inactive virus particles. Carriers or adjuvants may be, as a non limiting example, Ringer's solution, dextrose solution or Hank's solution. Non aqueous solutions such as fixed oils and ethyl oleate may also be used. A preferred excipient is 5% dextrose in saline. The excipient may contain minor amounts of additives such as substances that enhance isotonicity and chemical stability, including buffers and preservatives.

As used herein, the terms "therapeutically effective amount", "therapeutically effective dose" and "effective amount" mean the amount needed to achieve the desired result or results. As used herein, "pharmaceutically acceptable" means a material that is not biologically or otherwise undesirable, i.e., the material may be administered to an individual along with the compound without causing any undesirable biological effects or interacting in a deleterious manner with any of the other components of the pharmaceutical composition in which it is contained.

Certain of the above-described immunoglobulin single variable domains may have diagnostic, prognostic and/or therapeutic utility. More specifically, the specification also envisages immunoglobulin single variable domains as disclosed herein for use in diagnosis, prognosis, prevention and/or treatment of cancer, as well as for monitoring or assessing the impact of a therapy.

As used herein, the term "diagnosing" or grammatically equivalent wordings, means determining whether or not a subject suffers from a particular disease or disorder. As used herein, "prognosing" or grammatically equivalent wordings, means determining whether or not a subject has a risk of developing a particular disease or disorder.

As used herein, the term "preventing cancer" means inhibiting or reversing the onset of the disease, inhibiting or reversing the initial signs of the disease, inhibiting the appearance of clinical symptoms of the disease. As used herein, "treating cancer" or "treating a subject or individual having cancer" includes substantially inhibiting the disease, substantially slowing or reversing the progression of the disease, substantially ameliorating clinical symptoms of the disease or substantially preventing the appearance of clinical symptoms of the disease. In particular, it includes inhibition of the replication of cancer cells, inhibition of the spread of cancer, reduction in tumor size, lessening or reducing the number of cancerous cells in the body, and/or amelioration or alleviation of the symptoms of cancer. A treatment is considered therapeutic if there is a decrease in mortality and/or morbidity, and may be performed prophylactically, or therapeutically. A variety of subjects or individuals are treatable. Generally the "subjects" are mammals or mammalian, where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many embodiments, the subjects will be humans.

As used herein, the term "cancer" refers to any neoplastic disorder, including such cellular disorders as, for example, renal cell cancer, Kaposi's sarcoma, chronic leukemia, breast cancer, sarcoma, ovarian carcinoma, rectal cancer, throat cancer, melanoma, colon cancer, bladder cancer, mastocytoma, lung cancer, mammary adenocarcinoma, pharyngeal squamous cell carcinoma, and gastrointestinal or stomach cancer.

Accordingly, the specification provides for a method of preventing and/or treating cancer, comprising administering a pharmaceutically effective amount of an immunoglobulin single variable domain as disclosed herein or a pharmaceutical composition derived thereof to a subject in need thereof.

Specifically, the specification provides for a method of inhibiting tumor growth or tumor metastases in a subject in need thereof comprising selectively targeting TAM subpopulations linked to different intratumoral regions, such as hypoxic or normoxic regions of a solid tumor. The specification also describes the above method comprising administering to said subject a pharmaceutically effective amount of an immunoglobulin single variable domain or a pharmaceutical composition or a polypeptide as disclosed herein, in particular an immunoglobulin single variable domain fused to a toxin, or to a cytotoxic drug, or to an enzyme capable of converting a prodrug into a cytotoxic drug, or to a radionuclide, or coupled to a cytotoxic cell, and the like (see also Example section).

As used herein, "TAM subpopulations" refer to distinct subsets of tumor-associated macrophages (TAMs) that are present in a tumor environment, which are characterized by the differential expression of molecular markers, as listed in Table 1 on p. 5733 of Movahedi et al. 2010, Cancer Res 70: 5728-39. For example, the macrophage mannose receptor (MMR) is one of the molecular markers which is specifically expressed on a TAM subpopulation which resides predominantly in the hypoxic regions of a tumor. The specification describes that a TAM subpopulation can be defined as MHC II ^{low} or MHC II ^{hi}. And preferably, the TAM subpopulation is defined as MHC II ^{low}. Even more preferably, a TAM subpopulation is defined as a MMR-positive TAM subpopulation. The term "MMR-positive TAMs" means tumor-associated macrophages that express the macrophage mannose receptor at a high amount on their surface and predominantly reside in the hypoxic region of a tumor, in contrast to "MMR-negative TAMs", which do not or only poorly express the macrophage mannose receptor and mainly reside in the normoxic regions of a tumor (see also Movahedi et al. 2010, Cancer Res 70: 5728-39).

The immunoglobulin single variable domain and/or pharmaceutical composition may be administered by any suitable method within the knowledge of the skilled man. The administration of an immunoglobulin single variable domain as described above or a pharmaceutically acceptable salt thereof may be by way of oral, inhaled or parenteral administration. Particularly, the immunoglobulin single variable domain is delivered through intrathecal or intracerebroventricular administration. The active compound may be administered alone or preferably formulated as a pharmaceutical composition. An amount effective to treat a certain disease or disorder that express the antigen recognized by the immunoglobulin single variable domain depends on the usual factors such as the nature and severity of the disorder being treated and the weight of the mammal. However, a unit dose will normally be in the range of 0.01 to 50 mg, for example 0.01 to 10 mg, or 0.05 to 2 mg of immunoglobulin single variable domain or a pharmaceutically acceptable salt thereof. Unit doses will normally be administered once or more than once a day, for example 2, 3, or 4 times a day, more usually 1 to 3 times a day, such that the total daily dose is normally in the range of 0.0001 to 1 mg/kg; thus a suitable total daily dose for a 70 kg adult is 0.01 to 50 mg, for example 0.01 to 10 mg or more usually 0.05 to 10 mg. It is greatly preferred that the immunoglobulin single variable domain or a pharmaceutically acceptable salt thereof is administered in the form of a unit-dose composition, such as a unit dose oral, parenteral, or inhaled composition. Such compositions are prepared by admixture and are suitably adapted for oral, inhaled or parenteral administration, and as such may be in the form of tablets, capsules, oral liquid preparations, powders, granules, lozenges, reconstitutable powders, injectable and infusable solutions or suspensions or suppositories or aerosols. Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well-known methods in the art. Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate. These solid oral compositions may be prepared by conventional methods of blending, filling, tabletting or the like. Repeated blending operations may be used to distribute the active agent throughout those compositions employing large quantities of fillers. Such operations are, of course, conventional in the art. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents. Oral formulations also include conventional sustained release formulations, such as tablets or granules having an enteric coating. Preferably, compositions for inhalation are presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns, for example between 1 and 5 microns, such as between 2 and 5 microns. A favored inhaled dose will be in the range of 0.05 to 2 mg, for example 0.05 to 0.5 mg, 0.1 to 1 mg or 0.5 to 2 mg. For parenteral administration, fluid unit dose forms are prepared containing a compound as described herein and a sterile vehicle. The active compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound. Where appropriate, small amounts of bronchodilators for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included. As is common practice, the compositions will usually be accompanied by written or printed directions for use in the medical treatment concerned. All these medicaments can be intended for human or veterinary use.

The efficacy of the immunoglobulin single variable domains as disclosed herein, and of compositions comprising the same, can be tested using any suitable in vitro assay, cell-based assay, in vivo assay and/or animal model known per se, or any combination thereof, depending on the specific disease or disorder involved.

Specifically, it should be clear that the therapeutic method provided in the specificiation against cancer can also be used in combination with any other cancer therapy known in the art such as irradiation, chemotherapy or surgery.

Reliable hypoxia tracers that can be used for non-invasive tumor imaging are currently unavailable or limiting. The availability of such tracers would represent a significant progress in the field of radiotherapy, since they would allow the radiotherapist to adapt the radiation dose, depending on the targeted tumor region (hypoxic versus normoxic). The identification of tumor-associated macrophage (TAM) subsets that are situated in hypoxic/normoxic environments allows for the identification of macrophage-specific biomarkers that can be used for non-invasive imaging of hypoxic/normoxic areas in tumors. For example, MMR represents such a marker, since it is preferentially expressed on the hypoxic MHC II^{low} TAMs. Due to their small size and high tumor penetrance, immunoglobulin single variable domains, in particular Nanobodies, are the ideal format for non-invasive imaging. immunoglobulin single variable domains raised against markers that are preferentially expressed on the hypoxic MHC II^{low} TAMs can be used for the imaging of hypoxia in tumors. The immunoglobulin single variable domains against human MMR (or crossreactive against human/mouse MMR) can be used in this respect.

Other applications of TAM subset-specific immunoglobulin single variable domains coupled to tracers for imaging (for example Near Infrared Fluorescent or NIRF tracers), include but are not limited to (i) accurately quantifying the amount of TAM or TAM subsets inside any given tumor, which can be of prognostic value, (ii) assessing the impact of therapy - including TAM-directed therapies as presently claimed - on the amount and/or the activation state of TAM, (iii) visualizing hypoxic/normoxic regions within the tumor.

Accordingly, in a further aspect, the present invention provides immunoglobulin single variable domains for use as contrast agent in non-invasive in vivo medical imaging. Preferably, nuclear imaging is envisaged using the immunoglobulin single variable domains as disclosed herein, whereby MMR-positive tumor-associated macrophages are targeted inside a tumor. The specification also provides immunoglobulin single variable domains for use in monitoring the relative percentage of MMR-positive TAMs and/or the evolution in function of time of the relative percentage of MMR-positive TAMs.

As used herein, the term "medical imaging" refers to the technique and process that is used to visualize the inside of an organism's body (or parts and/or functions thereof), for clinical purposes (e.g. disease diagnosis, prognosis or therapy monitoring) or medical science (e.g. study of anatomy and physiology). Examples of medical imaging methods include invasive techniques, such as intravascular ultrasound (IVUS), as well as non-invasive techniques, such as magnetic resonance imaging (MRI), ultrasound (US) and nuclear imaging. Examples of nuclear imaging include positron emission tomography (PET) and single photon emission computed tomography (SPECT).

The specification also encompasses a method of in vivo imaging tumor cells in a subject, the method comprising the step of:
- administering to the subject an immunoglobulin single variable domain as disclosed herein fused to a detectable label.

As used herein, "tumor cells" or simply "tumor" refers to the tumor tissue as a whole, including different cell types that are present in a tumor environment. Tumor cells include cancer cells but also non-transformed host cells, or tumor-associated stroma cells. Examples of tumor-associated stroma cells include myeloid cells, in particular tumor-associated macrophages.

Preferably, the above described method may further comprise one or more of the following steps of:
- selectively targeting and/or visualizing MMR-positive TAMs linked to a hypoxic region of a solid tumor;
- determining a relative percentage of the MMR-positive TAMs, and optionally assessing the impact of a cancer therapy on the relative percentage of the MMR-positive TAMs,

Further, in still another aspect, the specification describes a method of diagnosing cancer or prognosing cancer aggressiveness in a subject suffering from or suspected to suffer from cancer comprising the steps of:
- administering to the subject an immunoglobulin single variable domain as disclosed herein, and
- determining the presence and/or relative percentage of MMR-positive TAMs in the subject, and
- diagnosing cancer or prognosing cancer aggressiveness in the subject according to the relative percentage of the MMR-positive TAMs.

Particularly, said method comprises the steps of (i) providing a sample from said individual comprising cancer cells or suspected to comprise cancer cells; (ii) determining in said sample the presence and/or relative percentage of MMR-positive TAMs; (iii) classifying said individual as having a good/prognosis or diagnosing said individual as having cancer according to the results of step (ii).

A sample may comprise any clinically relevant tissue sample, such as a tumor biopsy or fine needle aspirate, or a sample of bodily fluid, such as blood, plasma, serum, lymph, ascitic fluid, cystic fluid, urine or nipple exudate. The sample may be taken from a human, or, in a veterinary context, from non-human animals such as ruminants, horses, swine or sheep, or from domestic companion animals such as felines and canines. The sample may also be paraffin-embedded tissue sections. It is understood that the cancer tissue includes the primary tumor tissue as well as an organ-specific or tissue-specific metastasis tissue.

In the context of the specification as used herein, prognosing an individual suffering from or suspected to suffer from cancer refers to a prediction of the survival probability of individual having cancer or relapse risk which is related to the invasive or metastatic behavior (i.e. malignant progression) of tumor tissue or cells. As used herein, "good prognosis" means a desired outcome. For example, in the context of cancer, a good prognosis may be an expectation of no recurrences or metastasis within two, three, four, five years or more of initial diagnosis of cancer. "Poor prognosis" means an undesired outcome. For example, in the context of cancer, a poor prognosis may be an expectation of a recurrence or metastasis within two, three, four, or five years of initial diagnosis of cancer. Poor prognosis of cancer may indicate that a tumor is relatively aggressive, while good prognosis may indicate that a tumor is relatively nonaggressive.

As used herein, the terms "determining," "measuring," "assessing," and "assaying" are used interchangeably and include both quantitative and qualitative determinations. In particular, ways to determine the presence and/or relative percentage of TAM subpopulations, in particular MMR-positive TAMs, are known to the person skilled in the art, for example by using flow cytometry, and is illustrated into more detail, but without the purpose of being limitative, e.g. in US20110262348 and in Movahedi et al. 2010, Cancer Res 70: 5728-39).

Next, it is commonly known that finding tumor-specific markers for antibody-based targeting remains a difficult task. This is especially true when targeting the tumor stroma, since stromal antigens are typically not restricted to tumors. This may hamper the usefulness of these tools both for diagnostic and therapeutic applications. Therefore, it will often be desired to block extratumoral binding sites without competing for free binding sites in the tumor.

The specification further provides for any of the above described methods for in vivo imaging, diagnosis/prognosis or treatment of cancer comprising an additional step of co-administering a monovalent labeled immunoglobulin single variable domain as disclosed herein and an unlabeled bivalent form of an immunoglobulin single variable domain directed against the same target (macrophage mannose receptor) to block extratumoral binding sites. Preferably, the unlabeled bivalent form of the anti-MMR immunoglobulin single variable domain may comprise two identical or two different immunoglobulin single variable domains, as long as at least one of the immunoglobulin single variable domains is directed against the same target (macrophage mannose receptor). As used herein, "unlabeled" refers to the absence of a detectable label, in particular a radio-isotope or radio-active tracer as defined hereinbefore. It should be clear that this does not exclude the absence of another modification (as defined hereinbefore).

A further aspect of the invention relates to a method for producing an immunoglobulin single variable domain as disclosed herein or a polypeptide comprising an immunoglobulin single variable domain as disclosed herein, said method comprising the steps of:
- expressing, in a suitable host cell or expression system, a nucleic acid sequence encoding an immunoglobulin single variable domain or a polypeptide comprising an immunoglobulin single variable domain as disclosed herein; and optionally
- isolating and/or purifying said immunoglobulin single variable domain or said polypeptide.

Suitable expression systems include constitutive and inducible expression systems in bacteria or yeasts, virus expression systems, such as baculovirus, semliki forest virus and lentiviruses, or transient transfection in insect or mammalian cells. Suitable host cells include *E. coli, Lactococcus lactis, Saccharomyces cerevisiae, Schizosaccharomyces pombe, Pichia pastoris,* and the like. Suitable animal host cells include HEK 293, COS, S2, CHO, NSO, DT40 and the like. The cloning, expression and/or purification of the immunoglobulin single variable domains can be done according to techniques known by the skilled person in the art.

The following examples more fully illustrate preferred features of the specification, but are not intended to limit the invention in any way. Those having ordinary skill in the art and access to the teachings herein will recognize additional modifications and embodiments within the scope thereof. Therefore, the present invention is limited only by the claims attached herein. All of the starting materials and reagents disclosed below are known to those skilled in the art, and are available commercially or can be prepared using well-known techniques.

### EXAMPLES

### Material and methods to the Examples

### Mice and cell lines

Female Balb/c and C57BL/6 mice for biodistribution experiments in naive animals were purchased from Harlan. C57BL/6 MMR⁻ deficient mice were provided by Etienne Pays (Universite Libre de Bruxelles). All animal studies were approved by and performed according to the guidelines of the institutional review board. The 3LL-R clone of the C57BL/6 Lewis Lung Carcinoma was injected subcutaneously (sc) in the flank (3x10⁶ cells). 12-14 days after inoculation, 3LL-R tumor-bearing mice were imaged.

### Generation of anti-human MMR and anti-human/mouse MMR cross-reactive Nanobodies.

The anti-human macrophage mannose receptor (MMR) and anti-human/mouse MMR cross-reactive nanobodies (Nbs) were isolated from an immune phage library in a similar way as described before (Saerens et al. 2008, Current Opin Pharmacol 8: 600-608; Saerens et al. 2004, J Biol Chem 279: 51965-72; Saerens et al. 2008, Immunol Methods 329: 138-50). However, in order to generate cross-reactive Nbs, an alternating immunisation schedule was carried out. An alpaca (Vicugna pacos) was immunised with 100µg human MMR (R&D Systems #2534) followed by 100 µg mouse MMR (R&D Systems #2535) one week later. This alternating schedule was maintained for a total of 6 weeks and both proteins were mixed with the Gerbu adjuvant before injection. After immunisation, blood was collected and the peripheral blood lymphocytes were isolated. mRNA was extracted from these cells using TRIzol (Invitrogen) and was reverse-transcribed with oligo(dT) and SuperScript II RT (Invitrogen), following the manufacturer's instructions. The gene sequences encoding the variable domains (VHHs) were PCR amplified, with the leader sequence specific CALL001 (5'-GTC CTG GCT CTC TTC TAC AAG G-3'; SEQ ID NO: 257) and CH2 exon specific CALL002 (5'-GGT ACG TGC TGT TGA ACT GTT CC-3'; SEQ ID NO: 258) primers. After 1% agarose gel separation, the 600 bp fragment VHH-CH2 fragment was isolated from gel and re-amplified using the nested primers A6E (5'- GAT GTG CAG CTG CAG GAG TCT GGR GGA GG-3'; SEQ ID NO: 259) and PMCF (5'- CTA GTG CGG CCG CTG AGG AGA CGG TGA CCT GGG T -3'; SEQ ID NO: 260) specific for the framework-1 and framework-4 regions, respectively. These PCR fragments were ligated into the phagemid vector pMECS, a variant of pHEN4 (Arbabi Ghahroudi et al. 1997, FEBS Lett 414: 521-6), after digestion with the Pstl and Notl restriction enzymes. The pMECS differs from the pHEN4 in coding for a HA (YPYDVPDYGS; SEQ ID NO: 261) and 6xhistidine tag fusion at the C-terminus of the Nb instead of a HA tag only fusion. Ligated material was transformed in freshly prepared E. coli TG1 cells and plated on LB plates with ampicillin. The colonies were scraped from the plates, washed and stored at - 80 °C in LB-medium supplemented with glycerol (50% final concentration). Using M13VCS helper phage infection, the VHH library was expressed on phages. Specific Nanobody-phages were enriched by several consecutive rounds of *in vitro* selection on antigen coated to wells of microtiter plates (Nunc). For isolation of human/mouse MMR cross-reactive Nbs, screening was performed using human and mouse MMR alternatingly. Bound phage particles were eluted with 100 mM triethylamine (pH 11.0), immediately neutralized with 1 M Tris-HCI (pH 7.4) and used to infect E. coli TG1 cells. Individual colonies were picked and expression of recombinant Nanobody-M13 protein III by addition of 1 mM isopropyl-β-D-thiogalac-topyranoside (IPTG). The periplasmic extract of each clone was subsequently tested in ELISA for human MMR recognition with non-specific antigen coated wells serving as a negative control. Human/mouse MMR cross-reactive Nbs were also screened in a similar fashion against mouse MMR, only clones reactive with both human and mouse antigens were withheld as cross-reactive Nbs. Each ELISA was performed on plates coated with 1 µg/ml MMR in 100 mM NaHCO₃ buffer pH = 8.8. After coating the plates are washed with PBS +0.05% Tween-20 (PBST) and blocked for 2h with PBS + 0.05% Tween-20 + 2% non-fat dry milkpowder (Nestle) (PBSM). The PE extracts are then incubated for 1h on the plate and then washed with PBST followed by 1h incubation of 0.5 µg/ml mouse anti-HA tag antibody (16B12, Covance) in PBSM. After washing with PBST, 1.5 µg/ml alkaline phosphatase conjugated anti-mouse antibody (Sigma) in PBSM in added to the plate for 1h followed by PBST washing. Finally, the ELISA is developed using 2 mg/ml alkaline phosphatise substrate (Sigma) in AP-buffer (100 mM NaCl, 50 mM MgCl₂, 100 mM Tris pH=9.5) and the optical density signal at 405 nm is measured.

### Expression and purification of anti-human MMR and anti-human/mouse MMR cross-reactive Nanobodies.

The pMECS-Nb plasmids of the clones that scored positive in ELISA were transformed into *E. coli* WK6 cells. These cells stop translation at the TAG codon and therefore express the Nbs without a phage protein fusion. Production of recombinant VHH was performed in shaker flasks by growing the bacteria in Terrific Broth supplemented with 0.1% glucose and ampicillin until an absorbance at 600 nm between 0.6 and 0.9 was reached. VHH expression was then induced with 1 mM IPTG for 16 h at 28 °C. After pelleting the cells, the periplasmic proteins were extracted by osmotic shock. This periplasmic extract was loaded on a nickel-nitrilotriacetic acid (Thermo Scientific), and after washing, the bound proteins were eluted in PBS with 500 mM imidazol. The eluted fraction was dialysed to Vivaspin 2 centrifugal concentrators (Sartorius). The final purity of the protein was checked by SDS-PAGE. The final yield was determined from UV absorption at 280 nm using the calculated theoretical extinction coefficient of the VHH.

A HA tag is useful for detection of Nanobodies via flow cytometry, but has been shown to interfere with 99mTc labeling on adjacent His tags. Therefore, for experimental tests involving 99mTc labelling, the Nanobodies were recloned to the pHEN6c vector. This removes the HA tag and only fuses a 6xHis tag at the C-terminus of the Nanobody. In addition, after periplasmic expression and IMAC purification, Nanobodies to be used in experiments involving 99mTc labelling were subjected to an additional purification step via size exclusion chromatography (SEC) on Superdex 75 HR 10/30 (Pharmacia, Gaithersburg, MD) in phosphate buffered saline pH 7.4 (PBS) (Figure 4B and 4C).

### Surface plasmon resonance

Affinity analysis was performed using a BIAcore T100 (GE Healthcare) with HEPES-buffered saline running buffer (10 mM HEPES with 0.15 M NaCl, 3.4 mM EDTA and 0.005% surfactant P20 at pH 7.4). MRR was immobilized on a CM5 chip in acetate buffer 50 mM (pH 5,0), resulting in 2100 RU MMR coated on the chip. A second channel on the same chip was activated/deactivated in a similar way and served as a negative control. The MMR Nanobodies were used as analytes in 11 different concentrations, ranging from 1 to 2000 nM, at a flow rate of 10 ml/min. Glycine-HCI 50 mM (pH 2.0) was used for elution. The kinetic and equilibrium parameters (kd, ka and K_{D}) values were calculated from the combined sensogram of all concentrations using BIAcore T100 evaluation software 2.02 (GE Healthcare).

### Cell preparation and flow cytometry

The Nanobodies used for flow cytometry staining were produced from the original pMECS phage vector and therefore each Nanobody possesses a C-terminal HA and 6xHis tag.

For examining specific binding of the anti-MMR Nanobodies to mouse MMR, 3LL-R tumors were induced by injecting 3E6 cancer cells subcutaneously in C57BI/6 mice. After 15 days of tumor growth, the tumors were isolated, chopped and incubated for 25 minutes (37°C) with 10 U/ml Collagenase typel, 400 U/ml Collagenase typelV and 30 U/ml DNAsel (Worthington). Density gradients (Axis-Shield) were used to remove tissue debris and dead cells. Nanobodies were added at 10 µg/ml to 1E6 cells per tube. After at least one hour of incubation with anti-MMR Nanobody or control Nanobody, cells were washed two times with ice-cold Hank's Buffered Salt Solution (HBSS) buffer (containing 0,74 g/l EDTA and 0,5% (v/v) heat inactivated fetal calf serum) and incubated with 0.5 µg/ml Alexa fluor 488 conjugated anti-HA tag monoclonal antibody (clone 16B12, Invitrogen). Commercial antibodies used for cell surface stainings were Alexa Fluor 647 conjugated anti-mouse Ly6C monoclonal antibody (clone ER-MP20, AbD Serotec), PerCPCy5.5 conjugated anti-mouse MHCII monoclonal antibody (clone M5/114.15.2, Biolegend), Phycoerythrin conjugated anti-mouse Ly6G monoclonal antibody (clone 1A8, BD Biosciences). For flow cytometry measurements, CD11b+Ly6G- tumor associated macrophages were further gated on MHCII expression, as the MHCII^{low} TAMs express MMR to a high degree. Binding profiles of anti-MMR Nanobodies were recorded.

In order to examine binding of the Nanobodies to human MMR, human immature dendritic cells were used. Cryopreserved immature dendritic cells derived from healthy human donor monocytes were a kind gift of Dr. Karine Breckpot (Vrije Universiteit Brussel, Jette, Belgium). To prepare the immature dendritic cells, peripheral blood mononuclear cells were removed from the blood via leukapheresis and monocytes were separated by adherence to plastic Nunclon dishes (Nunc, Biotech Line, Slangerup, Denmark). After removal of the non-adherent cells, immature dendritic cells were in vitro generated during a six days differentiation from monocytes in RPMI 1640 medium supplemented with 500 U/ml IL-4 (Invitrogen) and 1000 U/ml GM-CSF (Gentaur). Cells were harvested at day 6, counted and aliquoted at 1E7 cells/vial. The cells were cryopreserved in 85% autologous serum, 10% DMSO (Sigma-Aldrich) and 5% Glucosteril 40% (Fresenius, Albertslund, Denmark). For flow cytometry analysis, cells were thawed on ice and incubated for more than one hour at room temperature with precooled RPMI 1640 medium supplemented with 500 U/ml IL-4 (Invitrogen) and 1000 U/ml GM-CSF (Gentaur). Next, 10% normal rabbit serum was added to prevent aspecific Fc mediated binding of antibodies. After half an hour the Nanobodies were added at 10 µg/ml to 2E5 cells per tube. After at least one hour of incubation with anti-MMR Nanobody or control Nanobody, cells were washed two times with ice-cold HBSS buffer supplemented with 1% normal rabbit serum (Eppendorf 5810-R Centrifuge, 8 minutes, 1400 rpm, 4°C) and incubated with 0.5 µg/ml Alexa fluor 488 conjugated anti-HA tag monoclonal antibody (clone 16B12, Invitrogen). Allophycocyanin conjugate (APC) conjugated anti-human CD11c monoclonal antibody (clone B-ly6, BD Biosciences) was used for CD11c staining. Stained cells were washed once more with ice-cold HBSS buffer supplemented with 1% normal rabbit serum (Eppendorf 5810-R Centrifuge, 8 minutes, 1400 rpm, 4°C) and analysed by flow cytometry.

### Nanobody labeling and in vitro characterization of ^{99m}Tc-labeled Nanobodies

Nanobodies were labeled with ^{99m}Tc at their hexahistidine tail. For the labeling, [^{99m}Tc(H₂0)₃(CO)₃]⁺ was synthesized by adding 1 mL of ^{99m}TcO4⁻ (0.74-3.7 GBq) to an Isolink kit (Mallinckrodt Medical BV) containing 4.5 mg of sodium boranocarbonate, 2.85 mg of sodium tetraborate.10H₂O, 8.5 mg of sodium tartrate.2H₂O, and 7.15 mg of sodium carbonate, pH 10.5. The vial was incubated at 100°C in a boiling bath for 20 min. The freshly prepared [^{99m}Tc(H₂O)₃(CO)₃]⁺ was allowed to cool at room temperature for 5 min and neutralized with 125 µL of 1 M HCl to pH 7-8. [^{99m}Tc(H₂O)₃(CO)₃]⁺ was added to 50 µL of 1 mg/mL monovalent Nanobody or 2 mg/ml bivalent Nanobody, together with 50 µL of carbonate buffer, pH 8. The mixture was incubated for 90 min at 52°C in a water bath. The labeling efficiency was determined by instant thin-layer chromatography in acetone as mobile phase and analyzed using a radiometric chromatogram scanner (VCS-201; Veenstra). When the labeling yield was less than 90%, the ^{99m}Tc-Nanobody solution was purified on a NAP-5 column (GE Healthcare) pre-equilibrated with phosphate-buffered saline (PBS) and passed through a 0.22 µm Millipore filter to eliminate possible aggregates.

### Pinhole SPECT-microCT imaging procedure

Mice were intravenously injected with 100-200 µl 45-155 MBq (about 5-10 µg) of ^{99m}Tc-Nanobody, with or without an excess of concentrated monovalent or bivalent unlabeled Nanobody. Mice were anesthetized with a mixture of 18.75 mg/kg ketamine hydrochloride (Ketamine 1000®, CEVA, Brussels, Belgium) and 0.5 mg/kg medetomidin hydrochloride (Domitor®, Pfizer, Brussels, Belgium) 10-15 min before pinhole SPECT acquisition.

MicroCT imaging was followed by pinhole SPECT on separate imaging systems. MicroCT was performed using a dual source CT scanner (Skyscan 1178, Skyscan, Aartselaar, Belgium) with 60 kV and 615 mA at a resolution of 83 µm. The total body scan time was 2 minutes. Image reconstruction was performed using filtered backprojection (Nrecon, Skyscan, Aartselaar, Belgium). Total body pinhole SPECT was performed at 60 min or 180 min post-injection (p.i.) using a dual headed gamma camera (e.cam¹⁸⁰ Siemens Medical Solutions, IL, USA), mounted with two multi-pinhole collimators (3 pinholes of 1.5 mm in each collimator, 200 mm focal length, 80 mm radius of rotation). Images were acquired over 360 degrees in 64 projections of 10 s into 128 x 128 matrices resulting in a total imaging time of 14 min. The SPECT images were reconstructed using an iterative reconstruction algorithm (OSEM) modified for the three pinhole geometry and automatically reoriented for fusion with CT based on six ⁵⁷Co landmarks.

### Image analysis

Image viewing and quantification was performed using AMIDE Medical Image Data Examiner software. Ellipsoid regions of interest (ROIs) were drawn around the tumor and major organs. Uptake was calculated as the counts in the tissue divided by the injected activity counts and normalized for the ROI size (%IA/cm³). High-resolution image 3D-reconstructions were generated using OsiriX Imaging Software.

### Biodistribution analysis

30 min after microCT/SPECT acquisition, mice were sacrificed with a lethal dose of pentobarbital (Nembutal; CEVA). Tumor, kidneys, liver, lungs, muscle, spleen, lymph nodes, bone, heart, and blood were removed and weighed, and the radioactivity was measured using an automated y-counter (Cobra II Inspector 5003; Canberra-Packard). Tissue and organ uptake was calculated as percentage of injected activity per gram of tissue (%IA/g), corrected for decay.

### Statistics

Statistical significance was determined by the Student's *t* test, using Microsoft Excel or GraphPad Prism 4.0 software. Differences were considered significant when *P* ≤ 0.05. Geometric means and confidence intervals were determined using Microsoft Excel.

Where multiple comparisons are made (9-10 different organs), the p-values of the student's t test were adjusted by Holm's procedure (Holm 1979, Scand J Stat 6: 65-70). The R environment (Ihaka and Gentleman 1996, J Comput Graph Stat 5: 299-314) and the multitest package (Pollard et al. 2011, available from [cited; Available from: http://CRAN.Rproject.org/package=multtest) were used for statistical analyses and figures. The significance of the student ttests and corrections for multiple testing was set to 0.05.

### Example 1. Relevance of MMR as a marker for tumor-promoting TAMs in human tumors.

In order to test the relevancy of MMR as a marker for tumor-promoting TAMs in human tumors, we assessed MMR and CD68 (as human macrophage marker) expression in paraffin-embedded sections of human breast cancer samples (VUB-UZ Brussel). Using immunohistochemistry on consecutive slides of the same specimen and one double staining on a single slide, we could demonstrate the presence of CD68 positive macrophages in both tumor and fibrotic foci within the tumor region. Immunostaining for MMR clearly shows that the macrophages found in fibrotic foci do co-express MMR (data not shown). Since fibrotic foci within the tumor region is known to be a marker of hypoxia and worse prognosis (Colpaert et al. 2003, Breast Cancer Res Treat 81: 137-47), the presence of MMR⁺ macrophages could function as an indicator of severe hypoxia in human tumors as well, similar to what was shown before for mouse tumors (US20110262348). In conclusion, these studies shows that in human breast cancer samples, MMR⁺ TAMS are clearly detected and are enriched in fibrotic foci which are known to be a marker for intratumoral hypoxia and correlate with a poor prognosis.

### Example 2. Selection of anti-human MMR Nbs

Anti-human MMR nanobodies were generated (see Material and Method section). After 4 panning rounds of an anti-human/anti mouse MMR phage bank on human MMR, up to 100 fold enrichments for hMMR reactive phages were observed per panning round. Therefore, 188 colonies from all rounds were selected for PE-expression. These PE-extracts were used in PE-ELISAs to determine which clones react effectively to hMMR. In total 100 clones were selected based on these results (Figure 1). Additionally, the DNA and protein sequence of the selected clones was determined (Table 1) and double clones or premature stopping clones were discarded.

### Example 3. Selection of anti-human/mouse MMR cross-reactive Nbs

Next, anti-human/mouse MMR cross-reactive nanobodies were generated (see also Material and Method section). The anti-human/anti mouse MMR phage bank was alternatingly screened on human and mouse MMR for a total of 4 rounds, resulting in up to 100 fold enrichments for hMMR/mMMR reactive phages from the second panning round. Therefore, 188 colonies from the second and third rounds were selected for PE-expression. These PE-extracts were used in PE-ELISAs to determine which clones react effectively to MMR, clones were selected after the ELISA on hMMR (Figure 2). These clones were then screened for binding on mouse MMR (Figure 3). Only clones (42) that reacted to both antigens were withheld as true cross-reactive Nbs. These clones were sequenced (Table 2) and divided into families based on their CDR3 regions.

### Example 4. Production of representative set of anti-human or anti-human/mouse MMR Nbs

A set of representative clones was selected for Nb production in E. Coli: (1) anti-human Nbs: NbhMMRm1.33, NbhMMRm10.19, NbhMMRm23.30, NbhMMRm2.15, NbhMMRm3.1, NbhMMRm5.38, NbhMMRm12.6, NbhMMRm11.5, NbhMMRm15.43, NbhMMRm16.95; (2) anti-human/mouse Nbs: NbhmMMRm14.4, NbhmMMRm6.71, NbhmMMRm24.31, NbhmMMRm20.52, NbhmMMRm3.49, NbhmMMRm22.84, NbhmMMRm19.52, NbhMMRm21.22, NbhmMMRm14.93, NbhmMMRm15.49, NbhmMMRm17.72, NbhmMMRm10.79, NbhmMMRm7.67, NbhMMRm4.83 Each clone was grown in a two litre culture. After expression and osmotic shock, the resulting extract was purified on 1 ml of Ni-NTA resin. The resulting 5 ml of eluted Nb was dialysed to PBS after which the concentration was determined using a Nanodrop device and purity was assessed on Coomassie stained SDS-PAGE gels. The nanobodies all produced between 0.7 and 9 mg Nb/I E coli culture (Table 3).

### Example 5. Determination of kinetic rate constants of a representative set of anti-human or anti-human/mouse MMR Nbs via surface plasmon resonance (SPR)

The binding characteristics and affinity of selected Nbs towards the recombinant hMMR and recombinant mMMR antigen was examined in further detail using surface plasmon resonance. A combined sensogram was recorded for each Nb (example for NbhmMMRm3.49 in Figure 5) and the kinetic and equilibrium parameters (kd, ka and KD) values were calculated (Table 4 and Table 5). Most but not all results on binding to mouse or human rMMR obtained via this SPR analysis are in agreement with the results obtained by PE-ELISA.

Based on the kinetic and equilibrium parameters (kd, ka and KD) values five among the cross-reactive anti-hmMMR Nbs were selected for further analysis (indicated in bold in Table 4 and Table 5). These five Nbs (NbhmMMRm3.1, NbhmMMRm14.4, NbhmMMRm5.38, NbhmMMRm26.70 and NbhmMMRm3.49) displayed rather low dissociation rate constants, which makes them suitable for in vivo imaging. The corresponding KD values for these Nanobodies ranged from 68 nM to 2 nM. It can clearly be seen from the data in Table 4 and 5 that the Nbs have a preferred MMR antigen: NbhmMMRm3.1, NbhmMMRm14.4, NbhmMMRm5.38 and NbhmMMRm3.49 have a higher affinity for the hMMR Ag compared to the mMMR Ag. In contrast, NbhmMMRm26.70 binds better to mMMR Ag as compared to hMMR Ag, even though the first rounds of immunization and panning were performed using the hMMR antigen.

### Example 6. Determination of binding of a representative set of anti-human or anti-human/mouse MMR Nbs on MMR expressed on cells via flow cytometry

In order to confirm the binding specificity of the 5 selected Nbs to MMR expressed on cells, flow cytometric analysis was performed.

Binding to cell-expressed mouse MMR was determined on tumor associated macrophages derived from a preclinical mouse tumor model, making use of the previously documented finding that TAMs contain molecularly and functionally distinct subsets differing in expression of MMR: MMR is highly expressed on MHC II^{low}TAMs, whereas MMR expression is lower for MHC II^{high} TAMs (Movahedi et al., 2010). As shown in Figure 6, clear shifts in fluorescence intensity, comparable to the shift of the anti-mMMR Nb clone 1, could be detected on MHCII^{low} TAMs for NbhMMRm3.1, NbhmMMRm14.4, NbhmMMRm26.70 and NbhMMRm3.49. Remarkably, binding of NbhmMMRm5.38 to TAMs could not be detected.

In order to investigate the binding specificity of the selected Nbs to human MMR, human immature monocyte-derived dendritic cells were generated and gated on CD11c⁺ cells. As shown in Figure 7, binding of NbhMMRm3.1, NbhmMMRm14.4, NbhmMMRm5.38 and NbhmMMRm3.49 to hMMR expressed on immature dendritic cells was clearly detected, whereas no significant shift in fluorescence intensity could be detected for NbhmMMRm26.70.

Overall, the flow cytometry analysis indicates that NbhmMMRm5.38 binds on cell expressed human MMR, but not mouse MMR. In contrast, NbhmMMRm26.70 has a similar binding pattern to the orginal anti-mouse MMR clone 1 and binds to mouse but not human MMR. NbhMMRm3.1, NbhmMMRm14.4 and NbhMMRm3.49 bind to both mouse and human MMR expressed on cells.

### Example 7. Tissue distribution experiments with a representative set of anti-human or anti-human/mouse MMR nanobodies in 3LL tumor bearing mice.

In a next step, we wished to assess whether selected anti-human MMR Nbs could be used for in vivo targeting of MMR-expressing cells. Since the flow cytometry analysis on human immature dendritic cells had revealed that NbhmMMRm26.70 does not bind to human MMR, it was not analysed at this time. Since NbhmMMRm3.1 and NbhmMMRm3.49 share the same CDR3 loop, but NbhmMMRm3.49 has a better affinity for recombinant MMR as compared to NbhmMMRm3.1, among those two Nanobodies, NbhmMMRm3.49 was selected for the in vivo targeting. Also NbhmMMRm14.4 and NbhmMMRm5.38 were included in the selection to be used for this example. Since the latter did not bind to mouse MMR according to the flow cytometric analysis, it could be used to exclude aspecific binding and accumulation in tissues.

The selected Nanobodies were labeled with ^{99m}Tc and injected intravenously in 3LL tumor bearing C57BL/6 mice. 3 hours post injection, the mice were dissected and radioactivity was measured in the major organs. As shown in Figure 8, NbhmMMRm14.4 and NbhmMMRm3.49 exhibited a similar pattern of tissue distribution as the positive control anti-mouse MMR Nanobody clone 1 (SEQ ID NO: 247), with high uptake in organs such as lungs, spleen and liver. Hereby, NbhmMMRm14.4 exhibited an even higher uptake in these organs as compared to NbhmMMRm3.49 and the anti-mouse MMR Nanobody clone 1. In contrast, the negative controls NbhmMMRm5.38 and Nb cAbBcII10 mainly showed high tracer uptake in the kidneys, indicative of renal clearance. The MMR nanobodies were also inoculated in MMR knockout mice where the uptake in liver and spleen dropped below 1% IA/g (Figure 8). These data indicate that the accumulation of NbhmMMRm14.4, NbhmMMRm3.49 and the anti-mouse MMR Nanobody clone 1 in organs such as liver and spleen is related to MMR expression and therefore reflects specific targeting to endogenous MMR expressed in these organs.

As shown in Figures 9 and 10, NbhmMMRm3.49 has similar tumor targeting potential as the positive control anti-mouse MMR Nanobody clone 1 (SEQ ID NO: 247). Remarkably, the tumor-targeting potential of NbhmMMRm14.4, which showed enhanced targeting to endogenous MMR in organs such as liver and spleen, was lower as compared to NbhmMMRm3.49 or the anti-mouse MMR Nanobody clone 1.

**Table 1. Anti-human MMR Nbs selected after ELISA on human MMR of PE-extracts from single Nb clones isolated from phage display. In addition to the Nb sequence sensu strictu depicted here, all clones also carry a C-terminal extension containing a HA and 6xHis tag (AAAYPYDVPDYGSHHHHHH; SEQ ID NO: 262). FRs and CDRs are listed separately in Table 6.**

| Name | SEQ ID NO: | Sequence |
|---|---|---|
| NbhMMRm3.1 | 8 | |
| NbhMMRm5.38 | 10 | |
| NbhMMRm1.33 | 11 | |
| NbhMMRm10.19 | 12 | |
| NbhMMRm23.30 | 13 | |
| NbhMMRm2.15 | 14 | |
| NbhMMRm12.6 | 15 | |
| NbhMMRm11.5 | 16 | |
| NbhMMRm15.43 | 17 | |
| NbhMMRm16.95 | 18 | |
| NbhMMRm4.83 | 19 | |

**Table 2. Anti-human/mouse MMR cross-reactive Nbs selected after ELISA on human MMR and mouse MMR of PE-extracts from single Nb clones isolated from phage display. In addition to the Nb sequence sensu strictu depicted here, all clones also carry a C-terminal extension containing a HA and 6xHis tag (AAAYPYDVPDYGSHHHHHH; SEQ ID NO: 262). FRs and CDRs are listed separately in Table 6.**

| Name | SEQ ID NO: | Sequence |
|---|---|---|
| NbhmMMRm3.49 | 7 | |
| NbhmMMRm14.4 | 9 | |
| NbhmMMRm6.71 | 20 | |
| NbhmMMRm24.31 | 21 | |
| NbhmMMRm20.52 | 22 | |
| NbhmMMRm22.84 | 23 | |
| NbhmMMRm19.52 | 24 | |
| NbhmMMRm21.22 | 25 | |
| NbhmMMRm14.93 | 26 | |
| NbhmMMRm15.49 | 27 | |
| NbhmMMRm17.72 | 28 | |
| NbhmMMRm10.79 | 29 | |
| NbhmMMRm7.67 | 30 | |
| NbhmMMRm8.67 | 31 | |
| NbhmMMRm13.89 | 32 | |
| NbhmMMRm18.63 | 33 | |
| NbhmMMRm25.86 | 34 | |
| NbhmMMRm26.70 | 35 | |
| NbhmMMRm27.95 | 36 | |

**Table 3. Production yields and physico-chemical characteristics of the anti-human MMR and anti-human/mouse MMR cross-reactive Nbs. All Nbs produce between 0.7 and 9 mg/l E coli culture. T.B.D.: to be determined. The number of amino acids (A.A.) and molecular weight (MW) indicated in the table include the HA and 6xHis tag.**

| Name | number of A.A. | MW (dalton) | Theoretical pi | Extinction coefficient (assuming all Cys form cystines) | Estimated production capacity (g/l *E*. *Coli)* |
|---|---|---|---|---|---|
| anti-human MMR Nbs | | | | | |
| NbhMMRm1.33 | 152 | 16545 | 6.30 | 30620 | 0.7 |
| NbhMMRm10.19 | 140 | 15188 | 6.63 | 31525 | 3.7 |
| NbhMMRm23.30 | 144 | 16150 | 5.71 | 63035 | 2.3 |
| NbhMMRm2.15 | 146 | 16095 | 5.58 | 29130 | 1.6 |
| NbhMMRm3.1 | 137 | 14961 | 6.63 | 30620 | 1.1 |
| NbhMMRm5.38 | 150 | 16535 | 5.51 | 36120 | 1.2 |
| NbhMMRm12.6 | 138 | 15011 | 6.13 | 23045 | 1.7 |
| NbhMMRm11.5 | 139 | 15106 | 7.17 | 26025 | 6.8 |
| NbhMMRm15.43 | 131 | 14266 | 8.00 | 30035 | 6.2 |
| NbhMMRm16.95 | 140 | 15025 | 7.17 | 26025 | 5.6 |
| NbhMMRm4.83 | 149 | 16395 | 6.70 | 36120 | 3.0 |

| anti-human/anti-mouse MMR Nbs | | | | | |
|---|---|---|---|---|---|
| NbhmMMRm14.4 | 141 | 15275 | 6.29 | 26025 | 1.6 |
| NbhmMMRm6.71 | 144 | 15295 | 5.70 | 24660 | 2.4 |
| NbhmMMRm24.31 | 144 | 15793 | 8.00 | 26025 | 1.0 |
| NbhmMMRm20.52 | 143 | 15431 | 8.00 | 30035 | 5.4 |
| NbhmMMRm3.49 | 137 | 14875 | 6.63 | 29130 | 1.6 |
| NbhmMMRm22.84 | 149 | 16628 | 7.25 | 35995 | 4.2 |
| NbhmMMRm19.52 | 136 | 14986 | 8.59 | 31525 | 4.1 |
| NbhMMRm21.22 | 137 | 15045 | 5.91 | 26025 | 2.1 |
| NbhmMMRm14.93 | 141 | 15289 | 6.63 | 26025 | 2.6 |
| NbhmMMRm15.49 | 131 | 14226 | 8.00 | 30035 | 4.0 |
| NbhmMMRm17.72 | 138 | 14896 | 7.18 | 24535 | 3.4 |
| NbhmMMRm10.79 | 140 | 15130 | 6.63 | 31525 | T.B.D |
| NbhmMMRm7.67 | 137 | 15153 | 7.18 | 30035 | 4.0 |
| NbhmMMRm8.67 | 151 | 16635 | 6.76 | 40005 | 2.0 |
| NbhmMMRm13.89 | 139 | 15096 | 6.70 | 30035 | 5.4 |
| NbhmMMRm18.63 | 135 | 14393 | 7.18 | 34045 | 9.0 |
| NbhmMMRm25.86 | 135 | 14891 | 6.29 | 24535 | 3.9 |
| NbhmMMRm26.70 | 140 | 15299 | 7.18 | 24535 | 6.0 |
| NbhmMMRm27.95 | 140 | 15392 | 7.22 | 24535 | 1.0 |

**Table 4. SPR kinetic and equilibrium parameters for anti-MMR Nanobodies on mouse MMR.**

| Sample | kₐ (1/Ms) | k_{d} (1/s) | K_{D} | Chi² |
|---|---|---|---|---|
| | | | | |
| | | | | |
| NbMMRm1.33 | NB | NB | NB | |
| NbhMMRm2.15 | NB | NB | NB | |
| **NbhMMRm5.38** | **1.3 E+5** | **3.3 E-3** | **2.5 E-8** | **0.216** |
| NbhMMRm10.19 | 8.4 E+5 | 2.1 E-1 | 2.5 E-7 | 0.280 |
| NbhMMRm11.5 | 1.5 E+5 | 1.9 E-2 | 1.2 E-7 | 0.211 |
| NbhMMRm12.6 | NB | NB | NB | |
| NbhMMRm15.43 | 2.9 E+4 | 1.3 E-3 | 4.4 E-8 | 0.299 |
| NbhMMRm16.95 | NB | NB | NB | |
| NbhMMRm23.30 | NB | NB | NB | |
| **NbhmMMRm3.1** | **2.1 E+5** | **4.0 E-3** | **1.9 E-8** | **0.459** |
| **NbhmMMRm3.49** | **2.9 E+5** | **3.6 E-3** | **1.2 E-8** | **0.451** |
| NbhmMMRm6.71 | NB | NB | NB | |
| NbhmMMRm7.67 | NB | NB | NB | |
| NbhmMMRm10.79 | 1.1 E+5 | 4.2 E-3 | 3.9 E-8 | 0.441 |
| **NbhmMMRm14.4** | **3.3 E+4** | **2.3 E-3** | **6.8 E-8** | **0.0343** |
| NbhmMMRm14.93 | 2.9 E+4 | 2.1 E-3 | 7.4 E-8 | 0.0389 |
| NbhmMMRm15.49 | 2.9 E+4 | 1.3 E-3 | 4.4 E-8 | 0.258 |
| NbhmMMRm17.72 | NB | NB | NB | |
| NbhmMMRm19.52 | 3.7 E+3 | 3.2 E-2 | 8.5 E-6 | 0.204 |
| NbhmMMRm20.52 | 1.6 E+6 | 2.0 E-3 | 1.3 E-9 | 1.10 |
| NbhmMMRm21.22 | NB | NB | NB | |
| NbhmMMRm22.84 | 3.0 E+4 | 4.0 E-3 | 1.3 E-7 | 0.0634 |
| NbhmMMRm24.31 | 2.8 E+4 | 2.1 E-3 | 7.4 E-8 | 0.0389 |
| **NbhmMMRm26.70** | **6.9 E+5** | **1.3 E-3** | **1.9 E-9** | **0.653** |

| | | | | |
|---|---|---|---|---|
| Nb:Nanobody; SE: standard error; NB: no binding. | | | | |

**Table 5. SPR kinetic and equilibrium parameters for anti-MMR Nanobodies on human MMR.**

| Sample | kₐ (1/Ms) | k_{d} (1/s) | K_{D} | Chi² |
|---|---|---|---|---|
| | | | | |
| | | | | |
| NbMMRm1.33 | 2.0 E+5 | 1.5 E-3 | 7.7 E-9 | 0.394 |
| NbhMMRm2.15 | 1.5 E+5 | 1.3 E-3 | 8.6 E-9 | 0.209 |
| **NbhMMRm5.38** | **2.0 E+5** | **6.6 E-4** | **3.3 E-9** | **0.144** |
| NbhMMRm10.19 | 7.5 E+5 | 3.1 E-2 | 5.0 E-8 | 0.240 |
| NbhMMRm11.5 | 4.0 E+5 | 2.2 E-2 | 5.5 E-8 | 0.246 |
| NbhMMRm12.6 | 1.5 E+5 | 1.2 E-3 | 8.2 E-9 | 0.132 |
| NbhMMRm15.43 | 2.2 E+4 | 5.9 E-3 | 2.7 E-7 | 0.201 |
| NbhMMRm16.95 | 6.6 E+4 | 1.4 E-3 | 2.1 E-8 | 0.496 |
| NbhMMRm23.30 | NB | NB | NB | |
| **NbhmMMRm3.1** | **2.2 E+5** | **7.4 E-4** | **3.4 E-9** | **0.157** |
| **NbhmMMRm3.49** | **4.4 E+5** | **8.0 E-4** | **1.8 E-9** | **0.271** |
| NbhmMMRm6.71 | 1.9 E+5 | 1.1 E-3 | 5.6 E-9 | 0.185 |
| NbhmMMRm7.67 | NB | NB | NB | |
| NbhmMMRmlO.79 | 1.6 E+4 | 6.6 E-3 | 4.2 E-7 | 0.122 |
| **NbhmMMRm14.4** | **1.4 E+5** | **1.4 E-3** | **1.0 E-8** | **0.136** |
| NbhmMMRm14.93 | 9.5 E+4 | 1.2 E-3 | 1.3 E-8 | 0.135 |
| NbhmMMRm15.49 | 2.1 E+4 | 6.1 E-3 | 2.9 E-7 | 0.196 |
| NbhmMMRm17.72 | 6.2 E+4 | 1.2 E-3 | 1.9 E-8 | 0.442 |
| NbhmMMRm19.52 | 6.0 E+3 | 1.0 E-2 | 1.7 E-6 | 0.107 |
| NbhmMMRm20.52 | 5.1 E+5 | 1.3 E-1 | 2.6 E-7 | 0.392 |
| NbhmMMRm21.22 | 3.4 E+5 | 1.2 E-3 | 3.6 E-9 | 1.72 |
| NbhmMMRm22.84 | 4.9 E+4 | 1.9 E-3 | 3.8 E-8 | 0.262 |
| NbhmMMRm24.31 | 2.6 E+5 | 6.9 E-4 | 2.7 E-9 | 0.386 |
| **NbhmMMRm26.70** | **5.8 E+5** | **7.3 E-3** | **1.3 E-8** | **1.03** |

| | | | | |
|---|---|---|---|---|
| Nb: Nanobody; SE: standard error; NB: no binding. | | | | |

**Table 6. CDRs of MMR-specific nanobodies**

| **Nanobody reference number** | **SEQ ID NO ¹** | **FR1** | **CDR1** | **FR2** | **CDR2** | **FR3** | **CDR3** | **FR4** |
|---|---|---|---|---|---|---|---|---|
| NbhmMMRm3.49 | 7 | | | | CISYKGGST (SEQ ID NO:127) | | | |
| NbhMMRm3.1 | 8 | | | | CISYKGGST (SEQ ID NO:128) | | | |
| NbhmMMRm14.4 | 9 | | | | AITSGSGST (SEQ ID NO:129) | | | |
| NbhMMRm5.38 | 10 | | | | CISSSDGST (SEQ ID NO:130) | | | |
| NbhMMRm1.33 | 11 | | | | CISSSGGST (SEQ ID NO:131) | | | |
| NbhMMRm10.19 | 12 | | | | AITSGGST (SEQ ID NO:132) | | | |
| NbhMMRm23.30 | 13 | | | | | | | |
| NbhMMRm2.15 | 14 | | | | CISSIGGSA (SEQ ID NO:134) | | | |
| NbhMMRm12.6 | 15 | | | | AITSGGST (SEQ ID NO:135) | | | |
| NbhMMRm11.5 | 16 | | | | AITSGGNT (SEQ ID NO:136) | | | |
| NbhMMRm15.43 | 17 | | | | GITGGNT (SEQ ID NO:137) | | | |
| NbhMMRm16.95 | 18 | | | | AITGSGRT (SEQ ID NO:138) | | | |
| NbhMMRm4.83 | 19 | | | | | | | |
| NbhmMMRm6.71 | 20 | | | | | | | |
| NbhmMMRm24.31 | 21 | | | | SINSSGGST (SEQ ID NO:141) | | | |
| NbhmMMRm20.52 | 22 | | | | AISRSGDST (SEQ ID NO:142) | | | |
| NbhmMMRm22.84 | 23 | | | | | | | |
| NbhmMMRm19.52 | 24 | | | | AIRLSGAR (SEQ ID NO:144) | | | |
| NbhmMMRm21.22 | 25 | | | | | | | |
| NbhmMMRm14.93 | 26 | | | | AITSGSGST (SEQ ID NO:146) | | | |
| NbhmMMRm15.49 | 27 | | | | GITGGNT (SEQ ID NO:147) | | NWGAY (SEQ ID NO:207) | |
| NbhmMMRm17.72 | 28 | | | | LVTGSGRT (SEQ ID NO:148) | | | |
| NbhmMMRm10.79 | 29 | | | | AVSSGGST (SEQ ID NO:149) | | | |
| NbhmMMRm7.67 | 30 | | | | SITSSGLDT (SEQ ID NO:150) | | | |
| NbhmMMRm8.67 | 31 | | | | SITSGGGST (SEQ ID NO:151) | | | |
| NbhmMMRm13.89 | 32 | | | | EITSSGST (SEQ ID NO:152) | | | |
| NbhmMMRm18.63 | 33 | | | | AISSGGST (SEQ ID NO:153) | | | |
| NbhmMMRm25.86 | 34 | | | | DVLPSGST (SEQ ID NO:154) | | | |
| NbhmMMRm26.70 | 35 | | | | | | | |
| NbhmMMRm27.95 | 36 | | | | | | | |

| | | | | | | | | |
|---|---|---|---|---|---|---|---|---|
| ¹ Nanobody sequences without His tag | | | | | | | | |

**Table 7. Amino acid sequences of human and mouse macrophage mannose receptor**

| **Name** | **SEQ ID NO** | **Amino acid sequence** |
|---|---|---|
| Human MMR (MRC1) | 1 | |
| | | |
| Recombinant human MMR (R&D systems) | 2 | |
| Mouse MMR (Mrc1) | 3 | |
| | | |
| Recombinant mouse MMR (R&D systems) | 4 | |
| Human MMR (MRC1) - ectodomain | 5 | |
| | | |
| Mouse MMR (Mrc1) - ectodomain | 6 | |

## Claims

1. An immunoglobulin single variable domain that specifically binds to human macrophage mannose receptor (SEQ ID NO: 1), wherein the immunoglobulin single variable domain comprises an amino acid sequence that comprises 4 framework regions (FR) and 3 complementarity determining regions (CDR) according to the following formula (1):
FR1-CDR1-FR2-CDR2-FR3-CDR3-FR4 (1);
and wherein CDR1 consists of SEQ ID NO: 67, CDR2 consists of SEQ ID NO: 127, and CDR3 consists of SEQ ID NO: 187;
and wherein the framework regions (FRs) have an amino acid sequence of SEQ ID N0: 37 (FR1), SEQ ID N0: 97 (FR2), SEQ ID NO: 157 (FR3), SEQ ID NO: 217 (FR4).

2. The immunoglobulin single variable domain according to claim 1, wherein the immunoglobulin single variable domain consists of an amino acid sequence of SEQ ID NO: 7.

3. The immunoglobulin single variable domain according to any of claims 1 to 2, wherein said immunoglobulin single variable domain is fused to a detectable label.

4. The immunoglobulin single variable domain according to claim 3, wherein said detectable label is a radionuclide.

5. The immunoglobulin single variable domain according to any of claims 1 or 2, wherein said immunoglobulin single variable domain is fused to a functional moiety.

6. The immunoglobulin single variable domain according to claim 5, wherein said functional moiety is a therapeutically active agent.

7. A polypeptide comprising an immunoglobulin single variable domain according to any of claims 1 to 6.

8. A nucleic acid sequence encoding an immunoglobulin single variable domain according to any of claims 1 to 6, or a polypeptide according to claim 7.

9. A pharmaceutical composition comprising the immunoglobulin single variable domain according to any of claims 1 to 6, or the polypeptide according to claim 7, and optionally at least one of a pharmaceutically acceptable carrier, adjuvant or diluent.

10. The immunoglobulin single variable domain according to any of claims 1 to 6, or the polypeptide according to claim 7, for use as a contrast agent in non-invasive in vivo medical imaging.

11. The immunoglobulin single variable domain according to any of claims 1 to 6, or the polypeptide according to claim 7, for use in the diagnosis and prognosis of cancer.

12. The immunoglobulin single variable domain according to any of claims 1 to 6, or the polypeptide according to claim 7, for use in the
treatment of cancer.

13. The immunoglobulin single variable domain according to any of claims 1 to 6, or the polypeptide according to claim 7, for use in monitoring cancer therapy.

14. The immunoglobulin single variable domain according to any of claims 1 to 6, or the polypeptide according to claim 7, wherein the immunoglobulin single variable domain specifically targets MMR-positive tumor-associated macrophages (TAMs) inside a tumor.

15. A method for producing an immunoglobulin single variable domain according to any of claims 1 to 6 or a polypeptide according to claim 7, said method comprising the step(s) of:
i) expressing, in a suitable host cell or a suitable expression system, a nucleic acid sequence according to claim 8; and optionally
ii) isolating and/or purifying the immunoglobulin single variable domain according to any of claims 1 to 6 or a polypeptide according to claim 7.

## Patentansprüche

1. Einzelne variable Immunglobulindomäne, die spezifisch an humanen Makrophagenmannose-Rezeptor (SEQ ID Nr. 1) bindet, wobei die einzelne variable Immunglobulindomäne eine Aminosäuresequenz umfasst, die 4 Gerüstregionen (FR) und 3 komplementaritätsbestimmende Regionen (CDR) gemäß der folgenden Formel (1) umfasst:
FR1-CDR1-FR2-CDR2-FR3-CDR3-FR4 (1);
und wobei CDR1 aus SEQ ID Nr. 67 besteht, CDR2 aus SEQ ID Nr. 127 besteht und CDR3 aus SEQ ID Nr. 187 besteht;
und wobei die Gerüstregionen (FR) eine Aminosäuresequenz von SEQ ID Nr. 37 (FR1), SEQ ID Nr. 97 (FR2), SEQ ID Nr. 157 (FR3), SEQ ID Nr. 217 (FR4) aufweisen.

2. Einzelne variable Immunglobulindomäne nach Anspruch 1, wobei die einzelne variable Immunglobulindomäne aus einer Aminosäuresequenz von SEQ ID Nr. 7 besteht.

3. Einzelne variable Immunglobulindomäne nach einem der Ansprüche 1 bis 2, wobei die einzelne variable Immunglobulindomäne an einen nachweisbaren Marker fusioniert ist.

4. Einzelne variable Immunglobulindomäne nach Anspruch 3, wobei der nachweisbare Marker ein Radionuklid ist.

5. Einzelne variable Immunglobulindomäne nach einem der Ansprüche 1 oder 2, wobei die einzelne variable Immunglobulindomäne an eine funktionelle Einheit fusioniert ist.

6. Einzelne variable Immunglobulindomäne nach Anspruch 5, wobei die funktionelle Einheit ein therapeutisch aktives Agens ist.

7. Polypeptid, das eine einzelne variable Immunglobulindomäne nach einem der Ansprüche 1 bis 6 umfasst.

8. Nukleinsäuresequenz, die eine einzelne variable Immunglobulindomäne nach einem der Ansprüche 1 bis 6 oder ein Polypeptid nach Anspruch 7 kodiert.

9. Pharmazeutische Zusammensetzung, die die einzelne variable Immunglobulindomäne nach einem der Ansprüche 1 bis 6 oder das Polypeptid nach Anspruch 7 und fakultativ mindestens einen bzw. eines von einem pharmazeutisch akzeptablen Trägerstoff, Adjuvans oder Verdünnungsmittel umfasst.

10. Einzelne variable Immunglobulindomäne nach einem der Ansprüche 1 bis 6 oder Polypeptid nach Anspruch 7 zur Verwendung als ein Kontrastmittel bei nichtinvasiver medizinischer In-vivo-Bildgebung.

11. Einzelne variable Immunglobulindomäne nach einem der Ansprüche 1 bis 6 oder Polypeptid nach Anspruch 7 zur Verwendung bei der Diagnose und Prognose von Krebs.

12. Einzelne variable Immunglobulindomäne nach einem der Ansprüche 1 bis 6 oder Polypeptid nach Anspruch 7 zur Verwendung bei der Behandlung von Krebs.

13. Einzelne variable Immunglobulindomäne nach einem der Ansprüche 1 bis 6 oder Polypeptid nach Anspruch 7 zur Verwendung bei der Überwachung einer Krebstherapie.

14. Einzelne variable Immunglobulindomäne nach einem der Ansprüche 1 bis 6 oder Polypeptid nach Anspruch 7, wobei die einzelne variable Immunglobulindomäne spezifisch auf MMR-positive tumorassoziierte Makrophagen (TAM) innerhalb eines Tumors abzielt.

15. Verfahren zur Herstellung einer einzelnen variablen Immunglobulindomäne nach einem der Ansprüche 1 bis 6 oder eines Polypeptids nach Anspruch 7, wobei das Verfahren den bzw. die folgenden Schritte umfasst:
i) Exprimieren einer Nukleinsäuresequenz nach Anspruch 8 in einer geeigneten Wirtszelle oder einem geeigneten Expressionssystem und fakultativ
ii) Isolieren und/oder Aufreinigen der einzelnen variablen Immunglobulindomäne nach einem der Ansprüche 1 bis 6 oder eines Polypeptids nach Anspruch 7.

## Revendications

1. Domaine variable unique d'immunoglobuline qui se fixe spécifiquement au récepteur MMR humain (SEQ ID N° : 1), dans lequel le domaine variable unique d'immunoglobuline comprend une séquence d'acides aminés qui comprend 4 régions de charpente (FR) et 3 régions déterminant la complémentarité (CDR) selon la formule (1) suivante :
FR1-CDR1-FR2-CDR2-FR3-CDR3-FR4 (1) ;
et dans lequel CDR1 est constituée de SEQ ID N° : 67, CDR2 est constituée de SEQ ID N° : 127, et CDR3 est constituée de SEQ ID N° : 187 ;
et dans lequel les régions de charpente (FR) ont une séquence d'acides aminés de SEQ ID N° : 37 (FR1), SEQ ID N° : 97 (FR2), SEQ ID N° : 157 (FR3), SEQ ID N° : 217 (FR4).

2. Domaine variable unique d'immunoglobuline selon la revendication 1, dans lequel le domaine variable unique d'immunoglobuline est constitué d'une séquence d'acides aminés de SEQ ID N° : 7.

3. Domaine variable unique d'immunoglobuline selon l'une quelconque des revendications 1 à 2, dans lequel ledit domaine variable unique d'immunoglobuline est fusionné à un traceur détectable.

4. Domaine variable unique d'immunoglobuline selon la revendication 3, dans lequel ledit traceur détectable est un radionucléide.

5. Domaine variable unique d'immunoglobuline selon l'une quelconque des revendications 1 ou 2, dans lequel ledit domaine variable unique d'immunoglobuline est fusionné à un fragment fonctionnel.

6. Domaine variable unique d'immunoglobuline selon la revendication 5, dans lequel ledit fragment fonctionnel est un agent thérapeutiquement actif.

7. Polypeptide comprenant un domaine variable unique d'immunoglobuline selon l'une quelconque des revendications 1 à 6.

8. Séquence d'acide nucléique codant pour un domaine variable unique d'immunoglobuline selon l'une quelconque des revendications 1 à 6 ou un polypeptide selon la revendication 7.

9. Composition pharmaceutique comprenant le domaine variable unique d'immunoglobuline selon l'une quelconque des revendications 1 à 6 ou le polypeptide selon la revendication 7, et en option au moins un d'un vecteur, adjuvant ou diluant pharmaceutiquement acceptable.

10. Domaine variable unique d'immunoglobuline selon l'une quelconque des revendications 1 à 6 ou polypeptide selon la revendication 7 destiné à être utilisé comme agent de contraste en imagerie médicale in vivo non invasive.

11. Domaine variable unique d'immunoglobuline selon l'une quelconque des revendications 1 à 6 ou polypeptide selon la revendication 7 destiné à être utilisé dans le diagnostic et pronostic du cancer.

12. Domaine variable unique d'immunoglobuline selon l'une quelconque des revendications 1 à 6 ou polypeptide selon la revendication 7 destiné à être utilisé dans le traitement du cancer.

13. Domaine variable unique d'immunoglobuline selon l'une quelconque des revendications 1 à 6 ou polypeptide selon la revendication 7 destiné à être utilisé dans la surveillance de la thérapie cancéreuse.

14. Domaine variable unique d'immunoglobuline selon l'une quelconque des revendications 1 à 6 ou polypeptide selon la revendication 7, dans lequel le domaine variable unique d'immunoglobuline vise spécifiquement des macrophages associés à une tumeur positifs au MMR (TAM) à l'intérieur d'une tumeur.

15. Procédé de production d'un domaine variable unique d'immunoglobuline selon l'une quelconque des revendications 1 à 6 ou d'un polypeptide selon la revendication 7, ledit procédé comprenant la/les étape(s) consistant à :
i) exprimer, dans une cellule hôte appropriée ou un système d'expression approprié, une séquence d'acide nucléique selon la revendication 8 ; et en option
ii) isoler et/ou purifier le domaine variable unique d'immunoglobuline selon l'une quelconque des revendications 1 à 6 ou un polypeptide selon la revendication 7.


### SEQUENCE LISTING

<110> VIB VZW
   VRIJE UNIVERSITEIT BRUSSEL
<120> ANTI-MACROPHAGE MANNOSE RECEPTOR SINGLE VARIABLE DOMAINS FOR TARGETING AND IN VIVO IMAGING OF TUMOR-ASSOCIATED MACROPHAGES
<130> JoVG/TAM/336
<150> US 13/480,350
   <151> 2012-05-24
<160> 262
<170> PatentIn version 3.5
<210> 1
   <211> 1456
   <212> PRT
   <213> Homo sapiens
<400> 1
<210> 2
   <211> 1371
   <212> PRT
   <213> Homo sapiens
<400> 2
   Leu Leu Asp Thr Arg Gln Phe Leu Ile Tyr Asn Glu Asp His Lys Arg
   1               5                   10                  15
   Cys Val Asp Ala Val Ser Pro Ser Ala Val Gln Thr Ala Ala Cys Asn
               20                  25                  30
   Gln Asp Ala Glu Ser Gln Lys Phe Arg Trp Val Ser Glu Ser Gln Ile
           35                  40                  45
   Met Ser Val Ala Phe Lys Leu Cys Leu Gly Val Pro Ser Lys Thr Asp
       50                  55                  60
   Trp Val Ala Ile Thr Leu Tyr Ala Cys Asp Ser Lys Ser Glu Phe Gln
   65                  70                  75                  80
   Lys Trp Glu Cys Lys Asn Asp Thr Leu Leu Gly Ile Lys Gly Glu Asp
                   85                  90                  95
   Leu Phe Phe Asn Tyr Gly Asn Arg Gln Glu Lys Asn Ile Met Leu Tyr
               100                 105                 110
   Lys Gly Ser Gly Leu Trp Ser Arg Trp Lys Ile Tyr Gly Thr Thr Asp
           115                 120                 125
   Asn Leu Cys Ser Arg Gly Tyr Glu Ala Met Tyr Thr Leu Leu Gly Asn
       130                 135                 140
   Ala Asn Gly Ala Thr Cys Ala Phe Pro Phe Lys Phe Glu Asn Lys Trp
   145                 150                 155                 160
   Tyr Ala Asp Cys Thr Ser Ala Gly Arg Ser Asp Gly Trp Leu Trp Cys
                   165                 170                 175
   Gly Thr Thr Thr Asp Tyr Asp Thr Asp Lys Leu Phe Gly Tyr Cys Pro
               180                 185                 190
   Leu Lys Phe Glu Gly Ser Glu Ser Leu Trp Asn Lys Asp Pro Leu Thr
           195                 200                 205
   Ser Val Ser Tyr Gln Ile Asn Ser Lys Ser Ala Leu Thr Trp His Gln
       210                 215                 220
   Ala Arg Lys Ser Cys Gln Gln Gln Asn Ala Glu Leu Leu Ser Ile Thr
   225                 230                 235                 240
   Glu Ile His Glu Gln Thr Tyr Leu Thr Gly Leu Thr Ser Ser Leu Thr
                   245                 250                 255
   Ser Gly Leu Trp Ile Gly Leu Asn Ser Leu Ser Phe Asn Ser Gly Trp
               260                 265                 270
   Gln Trp Ser Asp Arg Ser Pro Phe Arg Tyr Leu Asn Trp Leu Pro Gly
           275                 280                 285
   Ser Pro Ser Ala Glu Pro Gly Lys Ser Cys Val Ser Leu Asn Pro Gly
       290                 295                 300
   Lys Asn Ala Lys Trp Glu Asn Leu Glu Cys Val Gln Lys Leu Gly Tyr
   305                 310                 315                 320
   Ile Cys Lys Lys Gly Asn Thr Thr Leu Asn Ser Phe Val Ile Pro Ser
                   325                 330                 335
   Glu Ser Asp Val Pro Thr His Cys Pro Ser Gln Trp Trp Pro Tyr Ala
               340                 345                 350
   Gly His Cys Tyr Lys Ile His Arg Asp Glu Lys Lys Ile Gln Arg Asp
           355                 360                 365
   Ala Leu Thr Thr Cys Arg Lys Glu Gly Gly Asp Leu Ala Ser Ile His
       370                 375                 380
   Thr Ile Glu Glu Phe Asp Phe Ile Ile Ser Gln Leu Gly Tyr Glu Pro
   385                 390                 395                 400
   Asn Asp Glu Leu Trp Ile Gly Leu Asn Asp Ile Lys Ile Gln Met Tyr
   405 410 415
   Phe Glu Trp Ser Asp Gly Thr Pro Val Thr Phe Thr Lys Trp Leu Arg
   420 425 430
   Gly Glu Pro Ser His Glu Asn Asn Arg Gln Glu Asp Cys Val Val Met
   435 440 445
   Lys Gly Lys Asp Gly Tyr Trp Ala Asp Arg Gly Cys Glu Trp Pro Leu
   450 455 460
   Gly Tyr Ile Cys Lys Met Lys Ser Arg Ser Gln Gly Pro Glu Ile Val
   465 470 475 480
   Glu Val Glu Lys Gly Cys Arg Lys Gly Trp Lys Lys His His Phe Tyr
   485 490 495
   Cys Tyr Met Ile Gly His Thr Leu Ser Thr Phe Ala Glu Ala Asn Gln
   500 505 510
   Thr Cys Asn Asn Glu Asn Ala Tyr Leu Thr Thr Ile Glu Asp Arg Tyr
   515 520 525
   Glu Gln Ala Phe Leu Thr Ser Phe Val Gly Leu Arg Pro Glu Lys Tyr
   530 535 540
   Phe Trp Thr Gly Leu Ser Asp Ile Gln Thr Lys Gly Thr Phe Gln Trp
   545 550 555 560
   Thr Ile Glu Glu Glu Val Arg Phe Thr His Trp Asn Ser Asp Met Pro
   565 570 575
   Gly Arg Lys Pro Gly Cys Val Ala Met Arg Thr Gly Ile Ala Gly Gly
   580 585 590
   Leu Trp Asp Val Leu Lys Cys Asp Glu Lys Ala Lys Phe Val Cys Lys
   595 600 605
   His Trp Ala Glu Gly Val Thr His Pro Pro Lys Pro Thr Thr Thr Pro
   610 615 620
   Glu Pro Lys Cys Pro Glu Asp Trp Gly Ala Ser Ser Arg Thr Ser Leu
   625 630 635 640
   Cys Phe Lys Leu Tyr Ala Lys Gly Lys His Glu Lys Lys Thr Trp Phe
   645 650 655
   Glu Ser Arg Asp Phe Cys Arg Ala Leu Gly Gly Asp Leu Ala Ser Ile
               660                 665                 670
   Asn Asn Lys Glu Glu Gln Gln Thr Ile Trp Arg Leu Ile Thr Ala Ser
           675                 680                 685
   Gly Ser Tyr His Lys Leu Phe Trp Leu Gly Leu Thr Tyr Gly Ser Pro
       690                 695                 700
   Ser Glu Gly Phe Thr Trp Ser Asp Gly Ser Pro Val Ser Tyr Glu Asn
   705                 710                 715                 720
   Trp Ala Tyr Gly Glu Pro Asn Asn Tyr Gln Asn Val Glu Tyr Cys Gly
                   725                 730                 735
   Glu Leu Lys Gly Asp Pro Thr Met Ser Trp Asn Asp Ile Asn Cys Glu
               740                 745                 750
   His Leu Asn Asn Trp Ile Cys Gln Ile Gln Lys Gly Gln Thr Pro Lys
           755                 760                 765
   Pro Glu Pro Thr Pro Ala Pro Gln Asp Asn Pro Pro Val Thr Glu Asp
       770                 775                 780
   Gly Trp Val Ile Tyr Lys Asp Tyr Gln Tyr Tyr Phe Ser Lys Glu Lys
   785                 790                 795                 800
   Glu Thr Met Asp Asn Ala Arg Ala Phe Cys Lys Arg Asn Phe Gly Asp
                   805                 810                 815
   Leu Val Ser Ile Gln Ser Glu Ser Glu Lys Lys Phe Leu Trp Lys Tyr
               820                 825                 830
   Val Asn Arg Asn Asp Ala Gln Ser Ala Tyr Phe Ile Gly Leu Leu Ile
           835                 840                 845
   Ser Leu Asp Lys Lys Phe Ala Trp Met Asp Gly Ser Lys Val Asp Tyr
       850                 855                 860
   Val Ser Trp Ala Thr Gly Glu Pro Asn Phe Ala Asn Glu Asp Glu Asn
   865                 870                 875                 880
   Cys Val Thr Met Tyr Ser Asn Ser Gly Phe Trp Asn Asp Ile Asn Cys
                   885                 890                 895
   Gly Tyr Pro Asn Ala Phe Ile Cys Gln Arg His Asn Ser Ser Ile Asn
               900                 905                 910
   Ala Thr Thr Val Met Pro Thr Met Pro Ser Val Pro Ser Gly Cys Lys
           915                 920                 925
   Glu Gly Trp Asn Phe Tyr Ser Asn Lys Cys Phe Lys Ile Phe Gly Phe
       930                 935                 940
   Met Glu Glu Glu Arg Lys Asn Trp Gln Glu Ala Arg Lys Ala Cys Ile
   945                 950                 955                 960
   Gly Phe Gly Gly Asn Leu Val Ser Ile Gln Asn Glu Lys Glu Gln Ala
                   965                 970                 975
   Phe Leu Thr Tyr His Met Lys Asp Ser Thr Phe Ser Ala Trp Thr Gly
               980                 985                 990
   Leu Asn Asp Val Asn Ser Glu His  Thr Phe Leu Trp Thr  Asp Gly Arg
           995                 1000                 1005
   Gly Val  His Tyr Thr Asn Trp  Gly Lys Gly Tyr Pro  Gly Gly Arg
       1010                 1015                 1020
   Arg Ser  Ser Leu Ser Tyr Glu  Asp Ala Asp Cys Val  Val Ile Ile
       1025                 1030                 1035
   Gly Gly  Ala Ser Asn Glu Ala  Gly Lys Trp Met Asp  Asp Thr Cys
       1040                 1045                 1050
   Asp Ser  Lys Arg Gly Tyr Ile  Cys Gln Thr Arg Ser  Asp Pro Ser
       1055                 1060                 1065
   Leu Thr  Asn Pro Pro Ala Thr  Ile Gln Thr Asp Gly  Phe Val Lys
       1070                 1075                 1080
   Tyr Gly  Lys Ser Ser Tyr Ser  Leu Met Arg Gln Lys  Phe Gln Trp
       1085                 1090                 1095
   His Glu  Ala Glu Thr Tyr Cys  Lys Leu His Asn Ser  Leu Ile Ala
       1100                 1105                 1110
   Ser Ile  Leu Asp Pro Tyr Ser  Asn Ala Phe Ala Trp  Leu Gln Met
       1115                 1120                 1125
   Glu Thr  Ser Asn Glu Arg Val  Trp Ile Ala Leu Asn  Ser Asn Leu
       1130                 1135                 1140
   Thr Asp  Asn Gln Tyr Thr Trp  Thr Asp Lys Trp Arg  Val Arg Tyr
       1145                 1150                 1155
   Thr Asn  Trp Ala Ala Asp Glu  Pro Lys Leu Lys Ser  Ala Cys Val
       1160                 1165                 1170
   Tyr Leu  Asp Leu Asp Gly Tyr  Trp Lys Thr Ala His  Cys Asn Glu
       1175                 1180                 1185
   Ser Phe  Tyr Phe Leu Cys Lys  Arg Ser Asp Glu Ile  Pro Ala Thr
       1190                 1195                 1200
   Glu Pro  Pro Gln Leu Pro Gly  Arg Cys Pro Glu Ser  Asp His Thr
       1205                 1210                 1215
   Ala Trp  Ile Pro Phe His Gly  His Cys Tyr Tyr Ile  Glu Ser Ser
       1220                 1225                 1230
   Tyr Thr  Arg Asn Trp Gly Gln  Ala Ser Leu Glu Cys  Leu Arg Met
       1235                 1240                 1245
   Gly Ser  Ser Leu Val Ser Ile  Glu Ser Ala Ala Glu  Ser Ser Phe
       1250                 1255                 1260
   Leu Ser  Tyr Arg Val Glu Pro  Leu Lys Ser Lys Thr  Asn Phe Trp
       1265                 1270                 1275
   Ile Gly  Leu Phe Arg Asn Val  Glu Gly Thr Trp Leu  Trp Ile Asn
       1280                 1285                 1290
   Asn Ser  Pro Val Ser Phe Val  Asn Trp Asn Thr Gly  Asp Pro Ser
       1295                 1300                 1305
   Gly Glu  Arg Asn Asp Cys Val  Ala Leu His Ala Ser  Ser Gly Phe
       1310                 1315                 1320
   Trp Ser  Asn Ile His Cys Ser  Ser Tyr Lys Gly Tyr  Ile Cys Lys
       1325                 1330                 1335
   Arg Pro  Lys Ile Ile Asp Ala  Lys Pro Thr His Glu  Leu Leu Thr
       1340                 1345                 1350
   Thr Lys  Ala Asp Thr Arg Lys  Met Asp Pro Ser Lys  His His His
       1355                 1360                 1365
   His His  His
       1370
<210> 3
   <211> 1456
   <212> PRT
   <213> Mus musculus
<400> 3
   Met Arg Leu Leu Leu Leu Leu Ala Phe Ile Ser Val Ile Pro Val Ser
   1               5                   10                  15
   Val Gln Leu Leu Asp Ala Arg Gln Phe Leu Ile Tyr Asn Glu Asp His
               20                  25                  30
   Lys Arg Cys Val Asp Ala Leu Ser Ala Ile Ser Val Gln Thr Ala Thr
           35                  40                  45
   Cys Asn Pro Glu Ala Glu Ser Gln Lys Phe Arg Trp Val Ser Asp Ser
       50                  55                  60
   Gln Ile Met Ser Val Ala Phe Lys Leu Cys Leu Gly Val Pro Ser Lys
   65                  70                  75                  80
   Thr Asp Trp Ala Ser Val Thr Leu Tyr Ala Cys Asp Ser Lys Ser Glu
                   85                  90                  95
   Tyr Gln Lys Trp Glu Cys Lys Asn Asp Thr Leu Phe Gly Ile Lys Gly
               100                 105                 110
   Thr Glu Leu Tyr Phe Asn Tyr Gly Asn Arg Gln Glu Lys Asn Ile Lys
           115                 120                 125
   Leu Tyr Lys Gly Ser Gly Leu Trp Ser Arg Trp Lys Val Tyr Gly Thr
       130                 135                 140
   Thr Asp Asp Leu Cys Ser Arg Gly Tyr Glu Ala Met Tyr Ser Leu Leu
   145                 150                 155                 160
   Gly Asn Ala Asn Gly Ala Val Cys Ala Phe Pro Phe Lys Phe Glu Asn
                   165                 170                 175
   Lys Trp Tyr Ala Asp Cys Thr Ser Ala Gly Arg Ser Asp Gly Trp Leu
               180                 185                 190
   Trp Cys Gly Thr Thr Thr Asp Tyr Asp Lys Asp Lys Leu Phe Gly Phe
           195                 200                 205
   Cys Pro Leu His Phe Glu Gly Ser Glu Arg Leu Trp Asn Lys Asp Pro
       210                 215                 220
   Leu Thr Gly Ile Leu Tyr Gln Ile Asn Ser Lys Ser Ala Leu Thr Trp
   225 230 235 240
   His Gln Ala Arg Ala Ser Cys Lys Gln Gln Asn Ala Asp Leu Leu Ser
   245 250 255
   Val Thr Glu Ile His Glu Gln Met Tyr Leu Thr Gly Leu Thr Ser Ser
   260 265 270
   Leu Ser Ser Gly Leu Trp Ile Gly Leu Asn Ser Leu Ser Val Arg Ser
   275 280 285
   Gly Trp Gln Trp Ala Gly Gly Ser Pro Phe Arg Tyr Leu Asn Trp Leu
   290 295 300
   Pro Gly Ser Pro Ser Ser Glu Pro Gly Lys Ser Cys Val Ser Leu Asn
   305 310 315 320
   Pro Gly Lys Asn Ala Lys Trp Glu Asn Leu Glu Cys Val Gln Lys Leu
   325 330 335
   Gly Tyr Ile Cys Lys Lys Gly Asn Asn Thr Leu Asn Pro Phe Ile Ile
   340 345 350
   Pro Ser Ala Ser Asp Val Pro Thr Gly Cys Pro Asn Gln Trp Trp Pro
   355 360 365
   Tyr Ala Gly His Cys Tyr Arg Ile His Arg Glu Glu Lys Lys Ile Gln
   370 375 380
   Lys Tyr Ala Leu Gln Ala Cys Arg Lys Glu Gly Gly Asp Leu Ala Ser
   385 390 395 400
   Ile His Ser Ile Glu Glu Phe Asp Phe Ile Phe Ser Gln Leu Gly Tyr
   405 410 415
   Glu Pro Asn Asp Glu Leu Trp Ile Gly Leu Asn Asp Ile Lys Ile Gln
   420 425 430
   Met Tyr Phe Glu Trp Ser Asp Gly Thr Pro Val Thr Phe Thr Lys Trp
   435 440 445
   Leu Pro Gly Glu Pro Ser His Glu Asn Asn Arg Gln Glu Asp Cys Val
   450 455 460
   Val Met Lys Gly Lys Asp Gly Tyr Trp Ala Asp Arg Ala Cys Glu Gln
   465 470 475 480
   Pro Leu Gly Tyr Ile Cys Lys Met Val Ser Gln Ser His Ala Val Val
                   485                 490                 495
   Pro Glu Gly Ala Asp Lys Gly Cys Arg Lys Gly Trp Lys Arg His Gly
               500                 505                 510
   Phe Tyr Cys Tyr Leu Ile Gly Ser Thr Leu Ser Thr Phe Thr Asp Ala
           515                 520                 525
   Asn His Thr Cys Thr Asn Glu Lys Ala Tyr Leu Thr Thr Val Glu Asp
       530                 535                 540
   Arg Tyr Glu Gln Ala Phe Leu Thr Ser Leu Val Gly Leu Arg Pro Glu
   545                 550                 555                 560
   Lys Tyr Phe Trp Thr Gly Leu Ser Asp Val Gln Asn Lys Gly Thr Phe
                   565                 570                 575
   Arg Trp Thr Val Asp Glu Gln Val Gln Phe Thr His Trp Asn Ala Asp
               580                 585                 590
   Met Pro Gly Arg Lys Ala Gly Cys Val Ala Met Lys Thr Gly Val Ala
           595                 600                 605
   Gly Gly Leu Trp Asp Val Leu Ser Cys Glu Glu Lys Ala Lys Phe Val
       610                 615                 620
   Cys Lys His Trp Ala Glu Gly Val Thr Arg Pro Pro Glu Pro Thr Thr
   625                 630                 635                 640
   Thr Pro Glu Pro Lys Cys Pro Glu Asn Trp Gly Thr Thr Ser Lys Thr
                   645                 650                 655
   Ser Met Cys Phe Lys Leu Tyr Ala Lys Gly Lys His Glu Lys Lys Thr
               660                 665                 670
   Trp Phe Glu Ser Arg Asp Phe Cys Lys Ala Ile Gly Gly Glu Leu Ala
           675                 680                 685
   Ser Ile Lys Ser Lys Asp Glu Gln Gln Val Ile Trp Arg Leu Ile Thr
       690                 695                 700
   Ser Ser Gly Ser Tyr His Glu Leu Phe Trp Leu Gly Leu Thr Tyr Gly
   705                 710                 715                 720
   Ser Pro Ser Glu Gly Phe Thr Trp Ser Asp Gly Ser Pro Val Ser Tyr
                   725                 730                 735
   Glu Asn Trp Ala Tyr Gly Glu Pro Asn Asn Tyr Gln Asn Val Glu Tyr
   740 745 750
   Cys Gly Glu Leu Lys Gly Asp Pro Gly Met Ser Trp Asn Asp Ile Asn
   755 760 765
   Cys Glu His Leu Asn Asn Trp Ile Cys Gln Ile Gln Lys Gly Lys Thr
   770 775 780
   Leu Leu Pro Glu Pro Thr Pro Ala Pro Gln Asp Asn Pro Pro Val Thr
   785 790 795 800
   Ala Asp Gly Trp Val Ile Tyr Lys Asp Tyr Gln Tyr Tyr Phe Ser Lys
   805 810 815
   Glu Lys Glu Thr Met Asp Asn Ala Arg Ala Phe Cys Lys Lys Asn Phe
   820 825 830
   Gly Asp Leu Ala Thr Ile Lys Ser Glu Ser Glu Lys Lys Phe Leu Trp
   835 840 845
   Lys Tyr Ile Asn Lys Asn Gly Gly Gln Ser Pro Tyr Phe Ile Gly Met
   850 855 860
   Leu Ile Ser Met Asp Lys Lys Phe Ile Trp Met Asp Gly Ser Lys Val
   865 870 875 880
   Asp Phe Val Ala Trp Ala Thr Gly Glu Pro Asn Phe Ala Asn Asp Asp
   885 890 895
   Glu Asn Cys Val Thr Met Tyr Thr Asn Ser Gly Phe Trp Asn Asp Ile
   900 905 910
   Asn Cys Gly Tyr Pro Asn Asn Phe Ile Cys Gln Arg His Asn Ser Ser
   915 920 925
   Ile Asn Ala Thr Ala Met Pro Thr Thr Pro Thr Thr Pro Gly Gly Cys
   930 935 940
   Lys Glu Gly Trp His Leu Tyr Lys Asn Lys Cys Phe Lys Ile Phe Gly
   945 950 955 960
   Phe Ala Asn Glu Glu Lys Lys Ser Trp Gln Asp Ala Arg Gln Ala Cys
   965 970 975
   Lys Gly Leu Lys Gly Asn Leu Val Ser Ile Glu Asn Ala Gln Glu Gln
   980 985 990
   Ala Phe Val Thr Tyr His Met Arg  Asp Ser Thr Phe Asn  Ala Trp Thr
           995                 1000                 1005
   Gly Leu  Asn Asp Ile Asn Ala  Glu His Met Phe Leu  Trp Thr Ala
       1010                 1015                 1020
   Gly Gln  Gly Val His Tyr Thr  Asn Trp Gly Lys Gly  Tyr Pro Gly
       1025                 1030                 1035
   Gly Arg  Arg Ser Ser Leu Ser  Tyr Glu Asp Ala Asp  Cys Val Val
       1040                 1045                 1050
   Val Ile  Gly Gly Asn Ser Arg  Glu Ala Gly Thr Trp  Met Asp Asp
       1055                 1060                 1065
   Thr Cys  Asp Ser Lys Gln Gly  Tyr Ile Cys Gln Thr  Gln Thr Asp
       1070                 1075                 1080
   Pro Ser  Leu Pro Val Ser Pro  Thr Thr Thr Pro Lys  Asp Gly Phe
       1085                 1090                 1095
   Val Thr  Tyr Gly Lys Ser Ser  Tyr Ser Leu Met Lys  Leu Lys Leu
       1100                 1105                 1110
   Pro Trp  His Glu Ala Glu Thr  Tyr Cys Lys Asp His  Thr Ser Leu
       1115                 1120                 1125
   Leu Ala  Ser Ile Leu Asp Pro  Tyr Ser Asn Ala Phe  Ala Trp Met
       1130                 1135                 1140
   Lys Met  His Pro Phe Asn Val  Pro Ile Trp Ile Ala  Leu Asn Ser
       1145                 1150                 1155
   Asn Leu  Thr Asn Asn Glu Tyr  Thr Trp Thr Asp Arg  Trp Arg Val
       1160                 1165                 1170
   Arg Tyr  Thr Asn Trp Gly Ala  Asp Glu Pro Lys Leu  Lys Ser Ala
       1175                 1180                 1185
   Cys Val  Tyr Met Asp Val Asp  Gly Tyr Trp Arg Thr  Ser Tyr Cys
       1190                 1195                 1200
   Asn Glu  Ser Phe Tyr Phe Leu  Cys Lys Lys Ser Asp  Glu Ile Pro
       1205                 1210                 1215
   Ala Thr  Glu Pro Pro Gln Leu  Pro Gly Lys Cys Pro  Glu Ser Glu
   1220 1225 1230
   Gln Thr Ala Trp Ile Pro Phe Tyr Gly His Cys Tyr Tyr Phe Glu
   1235 1240 1245
   Ser Ser Phe Thr Arg Ser Trp Gly Gln Ala Ser Leu Glu Cys Leu
   1250 1255 1260
   Arg Met Gly Ala Ser Leu Val Ser Ile Glu Thr Ala Ala Glu Ser
   1265 1270 1275
   Ser Phe Leu Ser Tyr Arg Val Glu Pro Leu Lys Ser Lys Thr Asn
   1280 1285 1290
   Phe Trp Ile Gly Met Phe Arg Asn Val Glu Gly Lys Trp Leu Trp
   1295 1300 1305
   Leu Asn Asp Asn Pro Val Ser Phe Val Asn Trp Lys Thr Gly Asp
   1310 1315 1320
   Pro Ser Gly Glu Arg Asn Asp Cys Val Val Leu Ala Ser Ser Ser
   1325 1330 1335
   Gly Leu Trp Asn Asn Ile His Cys Ser Ser Tyr Lys Gly Phe Ile
   1340 1345 1350
   Cys Lys Met Pro Lys Ile Ile Asp Pro Val Thr Thr His Ser Ser
   1355 1360 1365
   Ile Thr Thr Lys Ala Asp Gln Arg Lys Met Asp Pro Gln Pro Lys
   1370 1375 1380
   Gly Ser Ser Lys Ala Ala Gly Val Val Thr Val Val Leu Leu Ile
   1385 1390 1395
   Val Ile Gly Ala Gly Val Ala Ala Tyr Phe Phe Tyr Lys Lys Arg
   1400 1405 1410
   His Ala Leu His Ile Pro Gln Glu Ala Thr Phe Glu Asn Thr Leu
   1415 1420 1425
   Tyr Phe Asn Ser Asn Leu Ser Pro Gly Thr Ser Asp Thr Lys Asp
   1430 1435 1440
   Leu Met Gly Asn Ile Glu Gln Asn Glu His Ala Ile Ile
   1445 1450 1455
<210> 4
   <211> 1376
   <212> PRT
   <213> Mus musculus
<400> 4
   Leu Leu Asp Ala Arg Gln Phe Leu Ile Tyr Asn Glu Asp His Lys Arg
   1               5                   10                  15
   Cys Val Asp Ala Leu Ser Ala Ile Ser Val Gln Thr Ala Thr Cys Asn
               20                  25                  30
   Pro Glu Ala Glu Ser Gln Lys Phe Arg Trp Val Ser Asp Ser Gln Ile
           35                  40                  45
   Met Ser Val Ala Phe Lys Leu Cys Leu Gly Val Pro Ser Lys Thr Asp
       50                  55                  60
   Trp Ala Ser Val Thr Leu Tyr Ala Cys Asp Ser Lys Ser Glu Tyr Gln
   65                  70                  75                  80
   Lys Trp Glu Cys Lys Asn Asp Thr Leu Phe Gly Ile Lys Gly Thr Glu
                   85                  90                  95
   Leu Tyr Phe Asn Tyr Gly Asn Arg Gln Glu Lys Asn Ile Lys Leu Tyr
               100                 105                 110
   Lys Gly Ser Gly Leu Trp Ser Arg Trp Lys Val Tyr Gly Thr Thr Asp
           115                 120                 125
   Asp Leu Cys Ser Arg Gly Tyr Glu Ala Met Tyr Ser Leu Leu Gly Asn
       130                 135                 140
   Ala Asn Gly Ala Val Cys Ala Phe Pro Phe Lys Phe Glu Asn Lys Trp
   145                 150                 155                 160
   Tyr Ala Asp Cys Thr Ser Ala Gly Arg Ser Asp Gly Trp Leu Trp Cys
                   165                 170                 175
   Gly Thr Thr Thr Asp Tyr Asp Lys Asp Lys Leu Phe Gly Phe Cys Pro
               180                 185                 190
   Leu His Phe Glu Gly Ser Glu Arg Leu Trp Asn Lys Asp Pro Leu Thr
           195                 200                 205
   Gly Ile Leu Tyr Gln Ile Asn Ser Lys Ser Ala Leu Thr Trp His Gln
       210                 215                 220
   Ala Arg Ala Ser Cys Lys Gln Gln Asn Ala Asp Leu Leu Ser Val Thr
   225                 230                 235                 240
   Glu Ile His Glu Gln Met Tyr Leu Thr Gly Leu Thr Ser Ser Leu Ser
                   245                 250                 255
   Ser Gly Leu Trp Ile Gly Leu Asn Ser Leu Ser Val Arg Ser Gly Trp
               260                 265                 270
   Gln Trp Ala Gly Gly Ser Pro Phe Arg Tyr Leu Asn Trp Leu Pro Gly
           275                 280                 285
   Ser Pro Ser Ser Glu Pro Gly Lys Ser Cys Val Ser Leu Asn Pro Gly
       290                 295                 300
   Lys Asn Ala Lys Trp Glu Asn Leu Glu Cys Val Gln Lys Leu Gly Tyr
   305                 310                 315                 320
   Ile Cys Lys Lys Gly Asn Asn Thr Leu Asn Pro Phe Ile Ile Pro Ser
                   325                 330                 335
   Ala Ser Asp Val Pro Thr Gly Cys Pro Asn Gln Trp Trp Pro Tyr Ala
               340                 345                 350
   Gly His Cys Tyr Arg Ile His Arg Glu Glu Lys Lys Ile Gln Lys Tyr
           355                 360                 365
   Ala Leu Gln Ala Cys Arg Lys Glu Gly Gly Asp Leu Ala Ser Ile His
       370                 375                 380
   Ser Ile Glu Glu Phe Asp Phe Ile Phe Ser Gln Leu Gly Tyr Glu Pro
   385                 390                 395                 400
   Asn Asp Glu Leu Trp Ile Gly Leu Asn Asp Ile Lys Ile Gln Met Tyr
                   405                 410                 415
   Phe Glu Trp Ser Asp Gly Thr Pro Val Thr Phe Thr Lys Trp Leu Pro
               420                 425                 430
   Gly Glu Pro Ser His Glu Asn Asn Arg Gln Glu Asp Cys Val Val Met
           435                 440                 445
   Lys Gly Lys Asp Gly Tyr Trp Ala Asp Arg Ala Cys Glu Gln Pro Leu
       450                 455                 460
   Gly Tyr Ile Cys Lys Met Val Ser Gln Ser His Ala Val Val Pro Glu
   465                 470                 475                 480
   Gly Ala Asp Lys Gly Cys Arg Lys Gly Trp Lys Arg His Gly Phe Tyr
   485 490 495
   Cys Tyr Leu Ile Gly Ser Thr Leu Ser Thr Phe Thr Asp Ala Asn His
   500 505 510
   Thr Cys Thr Asn Glu Lys Ala Tyr Leu Thr Thr Val Glu Asp Arg Tyr
   515 520 525
   Glu Gln Ala Phe Leu Thr Ser Leu Val Gly Leu Arg Pro Glu Lys Tyr
   530 535 540
   Phe Trp Thr Gly Leu Ser Asp Val Gln Asn Lys Gly Thr Phe Arg Trp
   545 550 555 560
   Thr Val Asp Glu Gln Val Gln Phe Thr His Trp Asn Ala Asp Met Pro
   565 570 575
   Gly Arg Lys Ala Gly Cys Val Ala Met Lys Thr Gly Val Ala Gly Gly
   580 585 590
   Leu Trp Asp Val Leu Ser Cys Glu Glu Lys Ala Lys Phe Val Cys Lys
   595 600 605
   His Trp Ala Glu Gly Val Thr Arg Pro Pro Glu Pro Thr Thr Thr Pro
   610 615 620
   Glu Pro Lys Cys Pro Glu Asn Trp Gly Thr Thr Ser Lys Thr Ser Met
   625 630 635 640
   Cys Phe Lys Leu Tyr Ala Lys Gly Lys His Glu Lys Lys Thr Trp Phe
   645 650 655
   Glu Ser Arg Asp Phe Cys Lys Ala Ile Gly Gly Glu Leu Ala Ser Ile
   660 665 670
   Lys Ser Lys Asp Glu Gln Gln Val Ile Trp Arg Leu Ile Thr Ser Ser
   675 680 685
   Gly Ser Tyr His Glu Leu Phe Trp Leu Gly Leu Thr Tyr Gly Ser Pro
   690 695 700
   Ser Glu Gly Phe Thr Trp Ser Asp Gly Ser Pro Val Ser Tyr Glu Asn
   705 710 715 720
   Trp Ala Tyr Gly Glu Pro Asn Asn Tyr Gln Asn Val Glu Tyr Cys Gly
   725 730 735
   Glu Leu Lys Gly Asp Pro Gly Met Ser Trp Asn Asp Ile Asn Cys Glu
   740 745 750
   His Leu Asn Asn Trp Ile Cys Gln Ile Gln Lys Gly Lys Thr Leu Leu
   755 760 765
   Pro Glu Pro Thr Pro Ala Pro Gln Asp Asn Pro Pro Val Thr Ala Asp
   770 775 780
   Gly Trp Val Ile Tyr Lys Asp Tyr Gln Tyr Tyr Phe Ser Lys Glu Lys
   785 790 795 800
   Glu Thr Met Asp Asn Ala Arg Ala Phe Cys Lys Lys Asn Phe Gly Asp
   805 810 815
   Leu Ala Thr Ile Lys Ser Glu Ser Glu Lys Lys Phe Leu Trp Lys Tyr
   820 825 830
   Ile Asn Lys Asn Gly Gly Gln Ser Pro Tyr Phe Ile Gly Met Leu Ile
   835 840 845
   Ser Met Asp Lys Lys Phe Ile Trp Met Asp Gly Ser Lys Val Asp Phe
   850 855 860
   Val Ala Trp Ala Thr Gly Glu Pro Asn Phe Ala Asn Asp Asp Glu Asn
   865 870 875 880
   Cys Val Thr Met Tyr Thr Asn Ser Gly Phe Trp Asn Asp Ile Asn Cys
   885 890 895
   Gly Tyr Pro Asn Asn Phe Ile Cys Gln Arg His Asn Ser Ser Ile Asn
   900 905 910
   Ala Thr Ala Met Pro Thr Thr Pro Thr Thr Pro Gly Gly Cys Lys Glu
   915 920 925
   Gly Trp His Leu Tyr Lys Asn Lys Cys Phe Lys Ile Phe Gly Phe Ala
   930 935 940
   Asn Glu Glu Lys Lys Ser Trp Gln Asp Ala Arg Gln Ala Cys Lys Gly
   945 950 955 960
   Leu Lys Gly Asn Leu Val Ser Ile Glu Asn Ala Gln Glu Gln Ala Phe
   965 970 975
   Val Thr Tyr His Met Arg Asp Ser Thr Phe Asn Ala Trp Thr Gly Leu
   980 985 990
   Asn Asp Ile Asn Ala Glu His Met Phe Leu Trp Thr Ala Gly Gln Gly
   995 1000 1005
   Val His Tyr Thr Asn Trp Gly Lys Gly Tyr Pro Gly Gly Arg Arg
   1010 1015 1020
   Ser Ser Leu Ser Tyr Glu Asp Ala Asp Cys Val Val Val Ile Gly
   1025 1030 1035
   Gly Asn Ser Arg Glu Ala Gly Thr Trp Met Asp Asp Thr Cys Asp
   1040 1045 1050
   Ser Lys Gln Gly Tyr Ile Cys Gln Thr Gln Thr Asp Pro Ser Leu
   1055 1060 1065
   Pro Val Ser Pro Thr Thr Thr Pro Lys Asp Gly Phe Val Thr Tyr
   1070 1075 1080
   Gly Lys Ser Ser Tyr Ser Leu Met Lys Leu Lys Leu Pro Trp His
   1085 1090 1095
   Glu Ala Glu Thr Tyr Cys Lys Asp His Thr Ser Leu Leu Ala Ser
   1100 1105 1110
   Ile Leu Asp Pro Tyr Ser Asn Ala Phe Ala Trp Met Lys Met His
   1115 1120 1125
   Pro Phe Asn Val Pro Ile Trp Ile Ala Leu Asn Ser Asn Leu Thr
   1130 1135 1140
   Asn Asn Glu Tyr Thr Trp Thr Asp Arg Trp Arg Val Arg Tyr Thr
   1145 1150 1155
   Asn Trp Gly Ala Asp Glu Pro Lys Leu Lys Ser Ala Cys Val Tyr
   1160 1165 1170
   Met Asp Val Asp Gly Tyr Trp Arg Thr Ser Tyr Cys Asn Glu Ser
   1175 1180 1185
   Phe Tyr Phe Leu Cys Lys Lys Ser Asp Glu Ile Pro Ala Thr Glu
   1190 1195 1200
   Pro Pro Gln Leu Pro Gly Lys Cys Pro Glu Ser Glu Gln Thr Ala
   1205 1210 1215
   Trp Ile Pro Phe Tyr Gly His Cys Tyr Tyr Phe Glu Ser Ser Phe
   1220 1225 1230
   Thr Arg Ser Trp Gly Gln Ala Ser Leu Glu Cys Leu Arg Met Gly
   1235 1240 1245
   Ala Ser Leu Val Ser Ile Glu Thr Ala Ala Glu Ser Ser Phe Leu
   1250 1255 1260
   Ser Tyr Arg Val Glu Pro Leu Lys Ser Lys Thr Asn Phe Trp Ile
   1265 1270 1275
   Gly Met Phe Arg Asn Val Glu Gly Lys Trp Leu Trp Leu Asn Asp
   1280 1285 1290
   Asn Pro Val Ser Phe Val Asn Trp Lys Thr Gly Asp Pro Ser Gly
   1295 1300 1305
   Glu Arg Asn Asp Cys Val Val Leu Ala Ser Ser Ser Gly Leu Trp
   1310 1315 1320
   Asn Asn Ile His Cys Ser Ser Tyr Lys Gly Phe Ile Cys Lys Met
   1325 1330 1335
   Pro Lys Ile Ile Asp Pro Val Thr Thr His Ser Ser Ile Thr Thr
   1340 1345 1350
   Lys Ala Asp Gln Arg Lys Met Asp Pro Gln Pro Lys Gly Ser Ser
   1355 1360 1365
   Lys Ala His His His His His His
   1370 1375
<210> 5
   <211> 1365
   <212> PRT
   <213> Homo sapiens
<400> 5
   Leu Leu Asp Thr Arg Gln Phe Leu Ile Tyr Asn Glu Asp His Lys Arg
   1 5 10 15
   Cys Val Asp Ala Val Ser Pro Ser Ala Val Gln Thr Ala Ala Cys Asn
   20 25 30
   Gln Asp Ala Glu Ser Gln Lys Phe Arg Trp Val Ser Glu Ser Gln Ile
   35 40 45
   Met Ser Val Ala Phe Lys Leu Cys Leu Gly Val Pro Ser Lys Thr Asp
   50 55 60
   Trp Val Ala Ile Thr Leu Tyr Ala Cys Asp Ser Lys Ser Glu Phe Gln
   65 70 75 80
   Lys Trp Glu Cys Lys Asn Asp Thr Leu Leu Gly Ile Lys Gly Glu Asp
   85 90 95
   Leu Phe Phe Asn Tyr Gly Asn Arg Gln Glu Lys Asn Ile Met Leu Tyr
   100 105 110
   Lys Gly Ser Gly Leu Trp Ser Arg Trp Lys Ile Tyr Gly Thr Thr Asp
   115 120 125
   Asn Leu Cys Ser Arg Gly Tyr Glu Ala Met Tyr Thr Leu Leu Gly Asn
   130 135 140
   Ala Asn Gly Ala Thr Cys Ala Phe Pro Phe Lys Phe Glu Asn Lys Trp
   145 150 155 160
   Tyr Ala Asp Cys Thr Ser Ala Gly Arg Ser Asp Gly Trp Leu Trp Cys
   165 170 175
   Gly Thr Thr Thr Asp Tyr Asp Thr Asp Lys Leu Phe Gly Tyr Cys Pro
   180 185 190
   Leu Lys Phe Glu Gly Ser Glu Ser Leu Trp Asn Lys Asp Pro Leu Thr
   195 200 205
   Ser Val Ser Tyr Gln Ile Asn Ser Lys Ser Ala Leu Thr Trp His Gln
   210 215 220
   Ala Arg Lys Ser Cys Gln Gln Gln Asn Ala Glu Leu Leu Ser Ile Thr
   225 230 235 240
   Glu Ile His Glu Gln Thr Tyr Leu Thr Gly Leu Thr Ser Ser Leu Thr
   245 250 255
   Ser Gly Leu Trp Ile Gly Leu Asn Ser Leu Ser Phe Asn Ser Gly Trp
   260 265 270
   Gln Trp Ser Asp Arg Ser Pro Phe Arg Tyr Leu Asn Trp Leu Pro Gly
   275 280 285
   Ser Pro Ser Ala Glu Pro Gly Lys Ser Cys Val Ser Leu Asn Pro Gly
   290 295 300
   Lys Asn Ala Lys Trp Glu Asn Leu Glu Cys Val Gln Lys Leu Gly Tyr
   305 310 315 320
   Ile Cys Lys Lys Gly Asn Thr Thr Leu Asn Ser Phe Val Ile Pro Ser
   325 330 335
   Glu Ser Asp Val Pro Thr His Cys Pro Ser Gln Trp Trp Pro Tyr Ala
   340 345 350
   Gly His Cys Tyr Lys Ile His Arg Asp Glu Lys Lys Ile Gln Arg Asp
   355 360 365
   Ala Leu Thr Thr Cys Arg Lys Glu Gly Gly Asp Leu Thr Ser Ile His
   370 375 380
   Thr Ile Glu Glu Leu Asp Phe Ile Ile Ser Gln Leu Gly Tyr Glu Pro
   385 390 395 400
   Asn Asp Glu Leu Trp Ile Gly Leu Asn Asp Ile Lys Ile Gln Met Tyr
   405 410 415
   Phe Glu Trp Ser Asp Gly Thr Pro Val Thr Phe Thr Lys Trp Leu Arg
   420 425 430
   Gly Glu Pro Ser His Glu Asn Asn Arg Gln Glu Asp Cys Val Val Met
   435 440 445
   Lys Gly Lys Asp Gly Tyr Trp Ala Asp Arg Gly Cys Glu Trp Pro Leu
   450 455 460
   Gly Tyr Ile Cys Lys Met Lys Ser Arg Ser Gln Gly Pro Glu Ile Val
   465 470 475 480
   Glu Val Glu Lys Gly Cys Arg Lys Gly Trp Lys Lys His His Phe Tyr
   485 490 495
   Cys Tyr Met Ile Gly His Thr Leu Ser Thr Phe Ala Glu Ala Asn Gln
   500 505 510
   Thr Cys Asn Asn Glu Asn Ala Tyr Leu Thr Thr Ile Glu Asp Arg Tyr
   515 520 525
   Glu Gln Ala Phe Leu Thr Ser Phe Val Gly Leu Arg Pro Glu Lys Tyr
   530 535 540
   Phe Trp Thr Gly Leu Ser Asp Ile Gln Thr Lys Gly Thr Phe Gln Trp
   545 550 555 560
   Thr Ile Glu Glu Glu Val Arg Phe Thr His Trp Asn Ser Asp Met Pro
   565 570 575
   Gly Arg Lys Pro Gly Cys Val Ala Met Arg Thr Gly Ile Ala Gly Gly
   580 585 590
   Leu Trp Asp Val Leu Lys Cys Asp Glu Lys Ala Lys Phe Val Cys Lys
   595 600 605
   His Trp Ala Glu Gly Val Thr His Pro Pro Lys Pro Thr Thr Thr Pro
   610 615 620
   Glu Pro Lys Cys Pro Glu Asp Trp Gly Ala Ser Ser Arg Thr Ser Leu
   625 630 635 640
   Cys Phe Lys Leu Tyr Ala Lys Gly Lys His Glu Lys Lys Thr Trp Phe
   645 650 655
   Glu Ser Arg Asp Phe Cys Arg Ala Leu Gly Gly Asp Leu Ala Ser Ile
   660 665 670
   Asn Asn Lys Glu Glu Gln Gln Thr Ile Trp Arg Leu Ile Thr Ala Ser
   675 680 685
   Gly Ser Tyr His Lys Leu Phe Trp Leu Gly Leu Thr Tyr Gly Ser Pro
   690 695 700
   Ser Glu Gly Phe Thr Trp Ser Asp Gly Ser Pro Val Ser Tyr Glu Asn
   705 710 715 720
   Trp Ala Tyr Gly Glu Pro Asn Asn Tyr Gln Asn Val Glu Tyr Cys Gly
   725 730 735
   Glu Leu Lys Gly Asp Pro Thr Met Ser Trp Asn Asp Ile Asn Cys Glu
   740 745 750
   His Leu Asn Asn Trp Ile Cys Gln Ile Gln Lys Gly Gln Thr Pro Lys
   755 760 765
   Pro Glu Pro Thr Pro Ala Pro Gln Asp Asn Pro Pro Val Thr Glu Asp
   770 775 780
   Gly Trp Val Ile Tyr Lys Asp Tyr Gln Tyr Tyr Phe Ser Lys Glu Lys
   785 790 795 800
   Glu Thr Met Asp Asn Ala Arg Ala Phe Cys Lys Arg Asn Phe Gly Asp
                   805                 810                 815
   Leu Val Ser Ile Gln Ser Glu Ser Glu Lys Lys Phe Leu Trp Lys Tyr
               820                 825                 830
   Val Asn Arg Asn Asp Ala Gln Ser Ala Tyr Phe Ile Gly Leu Leu Ile
           835                 840                 845
   Ser Leu Asp Lys Lys Phe Ala Trp Met Asp Gly Ser Lys Val Asp Tyr
       850                 855                 860
   Val Ser Trp Ala Thr Gly Glu Pro Asn Phe Ala Asn Glu Asp Glu Asn
   865                 870                 875                 880
   Cys Val Thr Met Tyr Ser Asn Ser Gly Phe Trp Asn Asp Ile Asn Cys
                   885                 890                 895
   Gly Tyr Pro Asn Ala Phe Ile Cys Gln Arg His Asn Ser Ser Ile Asn
               900                 905                 910
   Ala Thr Thr Val Met Pro Thr Met Pro Ser Val Pro Ser Gly Cys Lys
           915                 920                 925
   Glu Gly Trp Asn Phe Tyr Ser Asn Lys Cys Phe Lys Ile Phe Gly Phe
       930                 935                 940
   Met Glu Glu Glu Arg Lys Asn Trp Gln Glu Ala Arg Lys Ala Cys Ile
   945                 950                 955                 960
   Gly Phe Gly Gly Asn Leu Val Ser Ile Gln Asn Glu Lys Glu Gln Ala
                   965                 970                 975
   Phe Leu Thr Tyr His Met Lys Asp Ser Thr Phe Ser Ala Trp Thr Gly
               980                 985                 990
   Leu Asn Asp Val Asn Ser Glu His  Thr Phe Leu Trp Thr  Asp Gly Arg
           995                 1000                1005
   Gly Val  His Tyr Thr Asn Trp  Gly Lys Gly Tyr Pro  Gly Gly Arg
       1010                1015                1020
   Arg Ser  Ser Leu Ser Tyr Glu  Asp Ala Asp Cys Val  Val Ile Ile
       1025                1030                1035
   Gly Gly  Ala Ser Asn Glu Ala  Gly Lys Trp Met Asp  Asp Thr Cys
       1040                1045                1050
   Asp Ser  Lys Arg Gly Tyr Ile  Cys Gln Thr Arg Ser  Asp Pro Ser
       1055                 1060                 1065
   Leu Thr  Asn Pro Pro Ala Thr  Ile Gln Thr Asp Gly  Phe Val Lys
       1070                 1075                 1080
   Tyr Gly  Lys Ser Ser Tyr Ser  Leu Met Arg Gln Lys  Phe Gln Trp
       1085                 1090                 1095
   His Glu  Ala Glu Thr Tyr Cys  Lys Leu His Asn Ser  Leu Ile Ala
       1100                 1105                 1110
   Ser Ile  Leu Asp Pro Tyr Ser  Asn Ala Phe Ala Trp  Leu Gln Met
       1115                 1120                 1125
   Glu Thr  Ser Asn Glu Arg Val  Trp Ile Ala Leu Asn  Ser Asn Leu
       1130                 1135                 1140
   Thr Asp  Asn Gln Tyr Thr Trp  Thr Asp Lys Trp Arg  Val Arg Tyr
       1145                 1150                 1155
   Thr Asn  Trp Ala Ala Asp Glu  Pro Lys Leu Lys Ser  Ala Cys Val
       1160                 1165                 1170
   Tyr Leu  Asp Leu Asp Gly Tyr  Trp Lys Thr Ala His  Cys Asn Glu
       1175                 1180                 1185
   Ser Phe  Tyr Phe Leu Cys Lys  Arg Ser Asp Glu Ile  Pro Ala Thr
       1190                 1195                 1200
   Glu Pro  Pro Gln Leu Pro Gly  Arg Cys Pro Glu Ser  Asp His Thr
       1205                 1210                 1215
   Ala Trp  Ile Pro Phe His Gly  His Cys Tyr Tyr Ile  Glu Ser Ser
       1220                 1225                 1230
   Tyr Thr  Arg Asn Trp Gly Gln  Ala Ser Leu Glu Cys  Leu Arg Met
       1235                 1240                 1245
   Gly Ser  Ser Leu Val Ser Ile  Glu Ser Ala Ala Glu  Ser Ser Phe
       1250                 1255                 1260
   Leu Ser  Tyr Arg Val Glu Pro  Leu Lys Ser Lys Thr  Asn Phe Trp
       1265                 1270                 1275
   Ile Gly  Leu Phe Arg Asn Val  Glu Gly Thr Trp Leu  Trp Ile Asn
       1280                 1285                 1290
   Asn Ser Pro Val Ser Phe Val Asn Trp Asn Thr Gly Asp Pro Ser
   1295 1300 1305
   Gly Glu Arg Asn Asp Cys Val Ala Leu His Ala Ser Ser Gly Phe
   1310 1315 1320
   Trp Ser Asn Ile His Cys Ser Ser Tyr Lys Gly Tyr Ile Cys Lys
   1325 1330 1335
   Arg Pro Lys Ile Ile Asp Ala Lys Pro Thr His Glu Leu Leu Thr
   1340 1345 1350
   Thr Lys Ala Asp Thr Arg Lys Met Asp Pro Ser Lys
   1355 1360 1365
<210> 6
   <211> 1370
   <212> PRT
   <213> Mus musculus
<400> 6
   Leu Leu Asp Ala Arg Gln Phe Leu Ile Tyr Asn Glu Asp His Lys Arg
   1 5 10 15
   Cys Val Asp Ala Leu Ser Ala Ile Ser Val Gln Thr Ala Thr Cys Asn
   20 25 30
   Pro Glu Ala Glu Ser Gln Lys Phe Arg Trp Val Ser Asp Ser Gln Ile
   35 40 45
   Met Ser Val Ala Phe Lys Leu Cys Leu Gly Val Pro Ser Lys Thr Asp
   50 55 60
   Trp Ala Ser Val Thr Leu Tyr Ala Cys Asp Ser Lys Ser Glu Tyr Gln
   65 70 75 80
   Lys Trp Glu Cys Lys Asn Asp Thr Leu Phe Gly Ile Lys Gly Thr Glu
   85 90 95
   Leu Tyr Phe Asn Tyr Gly Asn Arg Gln Glu Lys Asn Ile Lys Leu Tyr
   100 105 110
   Lys Gly Ser Gly Leu Trp Ser Arg Trp Lys Val Tyr Gly Thr Thr Asp
   115 120 125
   Asp Leu Cys Ser Arg Gly Tyr Glu Ala Met Tyr Ser Leu Leu Gly Asn
   130 135 140
   Ala Asn Gly Ala Val Cys Ala Phe Pro Phe Lys Phe Glu Asn Lys Trp
   145                 150                 155                 160
   Tyr Ala Asp Cys Thr Ser Ala Gly Arg Ser Asp Gly Trp Leu Trp Cys
                   165                 170                 175
   Gly Thr Thr Thr Asp Tyr Asp Lys Asp Lys Leu Phe Gly Phe Cys Pro
               180                 185                 190
   Leu His Phe Glu Gly Ser Glu Arg Leu Trp Asn Lys Asp Pro Leu Thr
           195                 200                 205
   Gly Ile Leu Tyr Gln Ile Asn Ser Lys Ser Ala Leu Thr Trp His Gln
       210                 215                 220
   Ala Arg Ala Ser Cys Lys Gln Gln Asn Ala Asp Leu Leu Ser Val Thr
   225                 230                 235                 240
   Glu Ile His Glu Gln Met Tyr Leu Thr Gly Leu Thr Ser Ser Leu Ser
                   245                 250                 255
   Ser Gly Leu Trp Ile Gly Leu Asn Ser Leu Ser Val Arg Ser Gly Trp
               260                 265                 270
   Gln Trp Ala Gly Gly Ser Pro Phe Arg Tyr Leu Asn Trp Leu Pro Gly
           275                 280                 285
   Ser Pro Ser Ser Glu Pro Gly Lys Ser Cys Val Ser Leu Asn Pro Gly
       290                 295                 300
   Lys Asn Ala Lys Trp Glu Asn Leu Glu Cys Val Gln Lys Leu Gly Tyr
   305                 310                 315                 320
   Ile Cys Lys Lys Gly Asn Asn Thr Leu Asn Pro Phe Ile Ile Pro Ser
                   325                 330                 335
   Ala Ser Asp Val Pro Thr Gly Cys Pro Asn Gln Trp Trp Pro Tyr Ala
               340                 345                 350
   Gly His Cys Tyr Arg Ile His Arg Glu Glu Lys Lys Ile Gln Lys Tyr
           355                 360                 365
   Ala Leu Gln Ala Cys Arg Lys Glu Gly Gly Asp Leu Ala Ser Ile His
       370                 375                 380
   Ser Ile Glu Glu Phe Asp Phe Ile Phe Ser Gln Leu Gly Tyr Glu Pro
   385                 390                 395                 400
   Asn Asp Glu Leu Trp Ile Gly Leu Asn Asp Ile Lys Ile Gln Met Tyr
   405 410 415
   Phe Glu Trp Ser Asp Gly Thr Pro Val Thr Phe Thr Lys Trp Leu Pro
   420 425 430
   Gly Glu Pro Ser His Glu Asn Asn Arg Gln Glu Asp Cys Val Val Met
   435 440 445
   Lys Gly Lys Asp Gly Tyr Trp Ala Asp Arg Ala Cys Glu Gln Pro Leu
   450 455 460
   Gly Tyr Ile Cys Lys Met Val Ser Gln Ser His Ala Val Val Pro Glu
   465 470 475 480
   Gly Ala Asp Lys Gly Cys Arg Lys Gly Trp Lys Arg His Gly Phe Tyr
   485 490 495
   Cys Tyr Leu Ile Gly Ser Thr Leu Ser Thr Phe Thr Asp Ala Asn His
   500 505 510
   Thr Cys Thr Asn Glu Lys Ala Tyr Leu Thr Thr Val Glu Asp Arg Tyr
   515 520 525
   Glu Gln Ala Phe Leu Thr Ser Leu Val Gly Leu Arg Pro Glu Lys Tyr
   530 535 540
   Phe Trp Thr Gly Leu Ser Asp Val Gln Asn Lys Gly Thr Phe Arg Trp
   545 550 555 560
   Thr Val Asp Glu Gln Val Gln Phe Thr His Trp Asn Ala Asp Met Pro
   565 570 575
   Gly Arg Lys Ala Gly Cys Val Ala Met Lys Thr Gly Val Ala Gly Gly
   580 585 590
   Leu Trp Asp Val Leu Ser Cys Glu Glu Lys Ala Lys Phe Val Cys Lys
   595 600 605
   His Trp Ala Glu Gly Val Thr Arg Pro Pro Glu Pro Thr Thr Thr Pro
   610 615 620
   Glu Pro Lys Cys Pro Glu Asn Trp Gly Thr Thr Ser Lys Thr Ser Met
   625 630 635 640
   Cys Phe Lys Leu Tyr Ala Lys Gly Lys His Glu Lys Lys Thr Trp Phe
   645 650 655
   Glu Ser Arg Asp Phe Cys Lys Ala Ile Gly Gly Glu Leu Ala Ser Ile
   660 665 670
   Lys Ser Lys Asp Glu Gln Gln Val Ile Trp Arg Leu Ile Thr Ser Ser
   675 680 685
   Gly Ser Tyr His Glu Leu Phe Trp Leu Gly Leu Thr Tyr Gly Ser Pro
   690 695 700
   Ser Glu Gly Phe Thr Trp Ser Asp Gly Ser Pro Val Ser Tyr Glu Asn
   705 710 715 720
   Trp Ala Tyr Gly Glu Pro Asn Asn Tyr Gln Asn Val Glu Tyr Cys Gly
   725 730 735
   Glu Leu Lys Gly Asp Pro Gly Met Ser Trp Asn Asp Ile Asn Cys Glu
   740 745 750
   His Leu Asn Asn Trp Ile Cys Gln Ile Gln Lys Gly Lys Thr Leu Leu
   755 760 765
   Pro Glu Pro Thr Pro Ala Pro Gln Asp Asn Pro Pro Val Thr Ala Asp
   770 775 780
   Gly Trp Val Ile Tyr Lys Asp Tyr Gln Tyr Tyr Phe Ser Lys Glu Lys
   785 790 795 800
   Glu Thr Met Asp Asn Ala Arg Ala Phe Cys Lys Lys Asn Phe Gly Asp
   805 810 815
   Leu Ala Thr Ile Lys Ser Glu Ser Glu Lys Lys Phe Leu Trp Lys Tyr
   820 825 830
   Ile Asn Lys Asn Gly Gly Gln Ser Pro Tyr Phe Ile Gly Met Leu Ile
   835 840 845
   Ser Met Asp Lys Lys Phe Ile Trp Met Asp Gly Ser Lys Val Asp Phe
   850 855 860
   Val Ala Trp Ala Thr Gly Glu Pro Asn Phe Ala Asn Asp Asp Glu Asn
   865 870 875 880
   Cys Val Thr Met Tyr Thr Asn Ser Gly Phe Trp Asn Asp Ile Asn Cys
   885 890 895
   Gly Tyr Pro Asn Asn Phe Ile Cys Gln Arg His Asn Ser Ser Ile Asn
   900 905 910
   Ala Thr Ala Met Pro Thr Thr Pro Thr Thr Pro Gly Gly Cys Lys Glu
   915 920 925
   Gly Trp His Leu Tyr Lys Asn Lys Cys Phe Lys Ile Phe Gly Phe Ala
   930 935 940
   Asn Glu Glu Lys Lys Ser Trp Gln Asp Ala Arg Gln Ala Cys Lys Gly
   945 950 955 960
   Leu Lys Gly Asn Leu Val Ser Ile Glu Asn Ala Gln Glu Gln Ala Phe
   965 970 975
   Val Thr Tyr His Met Arg Asp Ser Thr Phe Asn Ala Trp Thr Gly Leu
   980 985 990
   Asn Asp Ile Asn Ala Glu His Met Phe Leu Trp Thr Ala Gly Gln Gly
   995 1000 1005
   Val His Tyr Thr Asn Trp Gly Lys Gly Tyr Pro Gly Gly Arg Arg
   1010 1015 1020
   Ser Ser Leu Ser Tyr Glu Asp Ala Asp Cys Val Val Val Ile Gly
   1025 1030 1035
   Gly Asn Ser Arg Glu Ala Gly Thr Trp Met Asp Asp Thr Cys Asp
   1040 1045 1050
   Ser Lys Gln Gly Tyr Ile Cys Gln Thr Gln Thr Asp Pro Ser Leu
   1055 1060 1065
   Pro Val Ser Pro Thr Thr Thr Pro Lys Asp Gly Phe Val Thr Tyr
   1070 1075 1080
   Gly Lys Ser Ser Tyr Ser Leu Met Lys Leu Lys Leu Pro Trp His
   1085 1090 1095
   Glu Ala Glu Thr Tyr Cys Lys Asp His Thr Ser Leu Leu Ala Ser
   1100 1105 1110
   Ile Leu Asp Pro Tyr Ser Asn Ala Phe Ala Trp Met Lys Met His
   1115 1120 1125
   Pro Phe Asn Val Pro Ile Trp Ile Ala Leu Asn Ser Asn Leu Thr
   1130 1135 1140
   Asn Asn Glu Tyr Thr Trp Thr Asp Arg Trp Arg Val Arg Tyr Thr
   1145 1150 1155
   Asn Trp Gly Ala Asp Glu Pro Lys Leu Lys Ser Ala Cys Val Tyr
   1160 1165 1170
   Met Asp Val Asp Gly Tyr Trp Arg Thr Ser Tyr Cys Asn Glu Ser
   1175 1180 1185
   Phe Tyr Phe Leu Cys Lys Lys Ser Asp Glu Ile Pro Ala Thr Glu
   1190 1195 1200
   Pro Pro Gln Leu Pro Gly Lys Cys Pro Glu Ser Glu Gln Thr Ala
   1205 1210 1215
   Trp Ile Pro Phe Tyr Gly His Cys Tyr Tyr Phe Glu Ser Ser Phe
   1220 1225 1230
   Thr Arg Ser Trp Gly Gln Ala Ser Leu Glu Cys Leu Arg Met Gly
   1235 1240 1245
   Ala Ser Leu Val Ser Ile Glu Thr Ala Ala Glu Ser Ser Phe Leu
   1250 1255 1260
   Ser Tyr Arg Val Glu Pro Leu Lys Ser Lys Thr Asn Phe Trp Ile
   1265 1270 1275
   Gly Met Phe Arg Asn Val Glu Gly Lys Trp Leu Trp Leu Asn Asp
   1280 1285 1290
   Asn Pro Val Ser Phe Val Asn Trp Lys Thr Gly Asp Pro Ser Gly
   1295 1300 1305
   Glu Arg Asn Asp Cys Val Val Leu Ala Ser Ser Ser Gly Leu Trp
   1310 1315 1320
   Asn Asn Ile His Cys Ser Ser Tyr Lys Gly Phe Ile Cys Lys Met
   1325 1330 1335
   Pro Lys Ile Ile Asp Pro Val Thr Thr His Ser Ser Ile Thr Thr
   1340 1345 1350
   Lys Ala Asp Gln Arg Lys Met Asp Pro Gln Pro Lys Gly Ser Ser
   1355 1360 1365
   Lys Ala
   1370
<210> 7
   <211> 118
   <212> PRT
   <213> Vicugna pacos
<400> 7
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Ser Leu Asp Tyr Tyr
               20                  25                  30
   Ala Ile Gly Trp Phe Arg Gln Ala Pro Gly Lys Glu Arg Glu Gly Ile
           35                  40                  45
   Ser Cys Ile Ser Tyr Lys Gly Gly Ser Thr Thr Tyr Ala Asp Ser Val
       50                  55                  60
   Lys Gly Arg Phe Thr Ile Ser Lys Asp Asn Ala Lys Asn Thr Ala Tyr
   65                  70                  75                  80
   Leu Gln Met Asn Ser Leu Lys Pro Glu Asp Thr Gly Ile Tyr Ser Cys
                   85                  90                  95
   Ala Ala Gly Phe Val Cys Tyr Asn Tyr Asp Tyr Trp Gly Gln Gly Thr
               100                 105                 110
   Gln Val Thr Val Ser Ser
           115
<210> 8
   <211> 118
   <212> PRT
   <213> Vicugna pacos
<400> 8
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Ile
           35                  40                  45
   Ser Cys Ile Ser Tyr Lys Gly Gly Ser Thr Thr Tyr Ala Asp Ser Val
       50                  55                  60
   Lys Gly Arg Phe Thr Ile Ser Lys Asp Asn Ala Lys Asn Thr Ala Tyr
   65                  70                  75                  80
   Leu Gln Met Asn Asn Leu Lys Pro Glu Asp Thr Gly Ile Tyr Tyr Cys
                   85                  90                  95
   Ala Ala Gly Phe Val Cys Tyr Asn Tyr Asp Tyr Trp Gly Pro Gly Thr
               100                 105                 110
   Gln Val Thr Val Ser Ser
           115
<210> 9
   <211> 122
   <212> PRT
   <213> Vicugna pacos
<400> 9
   Gln Val Gln Leu Gln Glu Ser Gly Gly Gly Leu Val Gln Ala Gly Asp
   1               5                   10                  15
   Ser Leu Arg Leu Ser Cys Ala Ala Ser Gly Arg Thr Phe Ser Ile Asn
               20                  25                  30
   Tyr Met Gly Trp Tyr Arg Gln Ala Pro Gly Lys Gln Arg Glu Leu Val
           35                  40                  45
   Ala Ala Ile Thr Ser Gly Ser Gly Ser Thr Asn Tyr Ala Asp Ser Val
       50                  55                  60
   Lys Gly Arg Phe Thr Ile Ser Arg Asp Asn Ala Lys Asn Thr Met Tyr
   65                  70                  75                  80
   Leu Gln Met Asn Ser Leu Lys Pro Glu Asp Thr Ala Val Tyr Tyr Cys
                   85                  90                  95
   Asn Ala Asp Met Asp Ser Ser Leu Ser Gly Gly Tyr Val Asp Val Trp
               100                 105                 110
   Gly Gln Gly Thr Gln Val Thr Val Ser Ser
           115                 120
<210> 10
   <211> 131
   <212> PRT
   <213> Vicugna pacos
<400> 10
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Phe Thr Asp Asp Asp Tyr
               20                  25                  30
   Asp Ile Gly Trp Phe Arg Gln Ala Pro Gly Lys Glu Arg Glu Gly Val
           35                  40                  45
   Ser Cys Ile Ser Ser Ser Asp Gly Ser Thr Tyr Tyr Ala Asp Ser Val
       50                  55                  60
   Lys Gly Arg Phe Thr Ile Ser Ser Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Phe Phe Arg Trp Asp Ser Gly Ser Tyr Tyr Val Arg Gly
               100                 105                 110
   Cys Arg His Ala Thr Tyr Asp Tyr Trp Gly Gln Gly Thr Gln Val Thr
           115                 120                 125
   Val Ser Ser
       130
<210> 11
   <211> 133
   <212> PRT
   <213> Vicugna pacos
<400> 11
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Leu Asp Asn Tyr
               20                  25                  30
   Thr Val Ala Trp Phe Arg Gln Ala Pro Gly Lys Glu Arg Glu Gly Val
           35                  40                  45
   Ser Cys Ile Ser Ser Ser Gly Gly Ser Thr Asn Tyr Ala Asp Ser Val
       50                  55                  60
   Lys Gly Arg Phe Thr Ile Ser Arg Asp Asn Ser Lys Lys Ser Val Tyr
   65                  70                  75                  80
   Leu Gln Met Asn Ser Leu Lys Pro Glu Asp Thr Ala Ile Tyr Thr Cys
                   85                  90                  95
   Ala Ala Glu Arg Ala Pro Pro Tyr Tyr Ser Gly Tyr Tyr Phe Phe Asp
               100                 105                 110
   Ser Thr Cys Val Ala Ala Ser Tyr Asp Tyr Trp Gly Gln Gly Thr Gln
           115                 120                 125
   Val Thr Val Ser Ser
       130
<210> 12
   <211> 121
   <212> PRT
   <213> Vicugna pacos
<400> 12
   Gln Val Gln Leu Gln Glu Ser Gly Gly Gly Leu Val Gln Pro Gly Gly
   1               5                   10                  15
   Ser Leu Lys Leu Ser Cys Ala Ala Ser Gly Ser Ile Phe Ser Ile Lys
               20                  25                  30
   Thr Met Gly Trp Tyr Arg Gln Ala Pro Gly Lys Gln Arg Glu Leu Val
           35                  40                  45
   Ala Ala Ile Thr Ser Gly Gly Ser Thr Asn Tyr Ala Asp Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Asn
                   85                  90                  95
   Ala Asp Gly Val Val Ala Trp Asp Gln Pro Tyr Asp Asn Tyr Trp Gly
               100                 105                 110
   Gln Gly Thr Gln Val Thr Val Ser Ser
           115                 120
<210> 13
   <211> 125
   <212> PRT
   <213> Vicugna pacos
<400> 13
   Gln Val Gln Leu Gln Glu Ser Gly Gly Gly Leu Val Gln Ala Gly Asp
   1               5                   10                  15
   Ser Leu Ser Ile Ser Cys Ala Ala Ser Gly Asp Thr Phe Asn His Tyr
               20                  25                  30
   Ser Trp Gly Trp Phe Arg Gln Ala Pro Gly Lys Ala Arg Glu Phe Val
           35                  40                  45
   Ala Ala Ile Ser Trp Asn Gly Gly Ser Lys Tyr Ala Asp Ser Val Lys
       50                  55                  60
   Gly Arg Phe Ala Ile Ser Arg Asp Ile Ala Lys Asn Thr Val Ser Leu
   65                  70                  75                  80
   Gln Met Asn Ser Leu Glu Pro Glu Asp Thr Ala Val Tyr Tyr Cys Ala
                   85                  90                  95
   Ala Asp Arg Arg Pro Tyr Asn Asp Trp Trp Asp Asp Trp Ser Trp Trp
               100                 105                 110
   Val Tyr Trp Gly Gln Gly Thr Gln Val Thr Val Ser Ser
           115                 120                 125
<210> 14
   <211> 127
   <212> PRT
   <213> Vicugna pacos
<400> 14
   Gln Val Gln Leu Gln Glu Ser Gly Gly Gly Leu Val Gln Pro Gly Glu
   1               5                   10                  15
   Ser Leu Arg Leu Ser Cys Lys Leu Ser Gly Phe Thr Leu Asp Tyr Tyr
               20                  25                  30
   Asp Ile Gly Trp Phe Arg Gln Ala Pro Gly Lys Glu Arg Glu Gly Val
           35                  40                  45
   Ser Cys Ile Ser Ser Ile Gly Gly Ser Ala Asn Tyr Ala Asp Ser Val
       50                  55                  60
   Lys Gly Arg Phe Thr Ile Ser Arg Asp Asn Val Lys Asn Thr Val Tyr
   65                  70                  75                  80
   Leu Gln Met Asn Ser Leu Lys Pro Glu Asp Thr Ala Ile Tyr Tyr Cys
                   85                  90                  95
   Ala Ala Glu Ala Gln Thr Pro Tyr Asn Asp Gly Asp Cys Thr Arg Ala
                 100                 105                 110
   Ser Tyr Asp Tyr Trp Gly Gln Gly Ile Gln Val Thr Val Ser Ser
           115                 120                 125
<210> 15
   <211> 119
   <212> PRT
   <213> Vicugna pacos
<400> 15
   Gln Val Gln Leu Gln Glu Ser Gly Gly Gly Leu Val Gln Pro Gly Gly
   1               5                   10                  15
   Ser Leu Arg Leu Ser Cys Val Val Ser Gly Ser Phe Leu Ser Ile Asn
               20                  25                  30
   His Met Gly Trp Tyr Arg Gln Val Ser Gly Glu Gln Arg Glu Leu Val
           35                  40                  45
   Ala Ala Ile Thr Ser Gly Gly Ser Thr Asn Tyr Ala Asp Ser Val Lys
       50                  55                  60
   Gly Arg Phe Thr Ile Ser Arg Asp Ser Ala Lys Asn Thr Val Tyr Leu
   65                  70                  75                  80
   Gln Met Asn Ser Leu Lys Pro Glu Asp Thr Ala Val Tyr Tyr Cys Asn
                   85                  90                  95
   Ala Asp Ala Leu Thr Met Leu Pro Pro Phe Asp Phe Trp Gly Gln Gly
               100                 105                 110
   Thr Gln Val Thr Val Ser Ser
           115
<210> 16
   <211> 120
   <212> PRT
   <213> Vicugna pacos
<400> 16
   Gln Val Gln Leu Gln Glu Ser Gly Gly Gly Leu Val Gln Pro Gly Gly
   1               5                   10                  15
   Ser Leu Met Leu Ser Cys Ala Ala Ser Gly Asn Ile Phe Thr Ile Asn
               20                  25                  30
   Arg Met Gly Trp Tyr Arg Gln Ala Pro Gly Lys Gln Arg Glu Leu Val
           35                  40                  45
   Ala Ala Ile Thr Ser Gly Gly Asn Thr Asn Tyr Ala Asp Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Asn
                   85                  90                  95
   Ala Ala Ile Val Thr Met Thr Ser Pro Tyr Ser Asp Tyr Trp Gly Gln
               100                 105                 110
   Gly Thr Gln Val Thr Val Ser Ser
           115                 120
<210> 17
   <211> 112
   <212> PRT
   <213> Vicugna pacos
<400> 17
   Gln Val Gln Leu Gln Glu Ser Gly Gly Thr Leu Val Gln Pro Gly Gly
   1               5                   10                  15
   Ser Leu Arg Leu Ser Cys Ala Ala Ser Gly Ser Thr Phe Ser Ile Asn
               20                  25                  30
   Asn Met Gly Trp Tyr Arg Gln Ala Pro Gly Lys Gln Arg Glu Leu Val
           35                  40                  45
   Ala Gly Ile Thr Gly Gly Asn Thr His Tyr Ala Asp Ser Val Lys Gly
       50                  55                  60
   Arg Phe Thr Ile Ser Arg Asp Asn Ala Lys Asn Thr Met Tyr Leu Gln
   65                  70                  75                  80
   Met Asn Gly Leu Lys Pro Glu Asp Thr Ala Val Tyr Tyr Cys Asn Ala
                   85                  90                  95
   Asn Trp Gly Ala Tyr Trp Gly Gln Gly Thr Gln Val Thr Val Ser Ser
               100                 105                 110
<210> 18
   <211> 121
   <212> PRT
   <213> Vicugna pacos
<400> 18
   Gln Val Gln Leu Gln Glu Ser Gly Gly Gly Leu Val Gln Pro Gly Gly
   1               5                   10                  15
   Ser Leu Gly Leu Ser Cys Ala Ala Ser Gly Arg Ile Ala Ser Ile Ser
               20                  25                  30
   Ala Met Gly Trp Tyr Arg Gln Ala Pro Gly Lys Gln Arg Glu Leu Val
           35                  40                  45
   Ala Ala Ile Thr Gly Ser Gly Arg Thr Asn Tyr Ala Asp Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Asn
                   85                  90                  95
   Leu Leu Met Val Asp Tyr Gly Leu Gly Leu Gly Thr Asp Tyr Trp Gly
               100                 105                 110
   Gln Gly Thr Gln Val Thr Val Ser Ser
           115                 120
<210> 19
   <211> 130
   <212> PRT
   <213> Vicugna pacos
<400> 19
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Pro Gly Phe Lys Leu Asp
               20                  25                  30
   Tyr Tyr Ala Ile Ala Trp Phe Arg Gln Ala Pro Gly Lys Glu Arg Glu
           35                  40                  45
   Gly Val Ser Cys Ile Gly Gly Ser Gly Ser Gly Leu Thr Thr Tyr Val
       50                  55                  60
   Glu Asn Ser Val Lys Asp Arg Phe Thr Ile Ser Arg Asp Asn Ala Gln
   65                  70                  75                  80
   Asn Thr Val Tyr Leu His Met Asn Ser Leu Lys Pro Glu Asp Thr Gly
                   85                  90                  95
   Ile Tyr Tyr Cys Ala Ala Asp Thr Tyr Tyr Tyr Cys Ser Lys Arg Val
   100 105 110
   Trp Arg Asn Asp Tyr Gly Ser Trp Gly Gln Gly Thr Gln Val Thr Val
   115 120 125
   Ser Ser
   130
<210> 20
   <211> 125
   <212> PRT
   <213> Vicugna pacos
<400> 20
   Gln Val Gln Leu Gln Glu Ser Gly Gly Gly Leu Val Gln Ala Gly Gly
   1 5 10 15
   Ser Leu Arg Leu Ser Cys Ala Ala Ser Gly Gly Thr Phe Asp Asp Ser
   20 25 30
   Val Ile Gly Trp Phe Arg Gln Ala Pro Gly Lys Glu Arg Glu Gly Val
   35 40 45
   Ser Cys Ile Ser Ser Asn Asp Gly Thr Thr His Tyr Ala Ser Pro Val
   50 55 60
   Lys Gly Arg Phe Thr Ile Ser Ser Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Glu Thr Pro Ser Ile Gly Ser Pro Cys Thr Ser Ala Ser Tyr
   100 105 110
   Asp Tyr Trp Gly Gln Gly Thr Gln Val Thr Val Ser Ser
   115 120 125
<210> 21
   <211> 125
   <212> PRT
   <213> Vicugna pacos
<400> 21
   Gln Val Gln Leu Gln Glu Ser Gly Gly Gly Leu Val Gln Pro Gly Gly
   1 5 10 15
   Ser Leu Arg Leu Ser Cys Thr Ala Thr Gly Phe Thr Leu Lys Asn His
   20 25 30
   His Ile Gly Trp Leu Arg Gln Ala Pro Gly Lys Glu Arg Glu Gly Val
   35 40 45
   Ala Ser Ile Asn Ser Ser Gly Gly Ser Thr Asn Tyr Ala Asp Ser Val
   50 55 60
   Gln Gly Arg Phe Thr Ile Ser Arg Asp Asn Ala Lys Asn Thr Val Phe
   65 70 75 80
   Leu Gln Met Asn Ser Leu Lys Ser Glu Asp Thr Ala Val Tyr Tyr Cys
   85 90 95
   Ala Arg Leu Arg Arg Tyr Tyr Gly Leu Asn Leu Asp Pro Gly Ser Tyr
   100 105 110
   Asp Tyr Trp Gly Gln Gly Thr Gln Val Thr Val Ser Ser
   115 120 125
<210> 22
   <211> 124
   <212> PRT
   <213> Vicugna pacos
<400> 22
   Gln Val Gln Leu Gln Glu Ser Gly Gly Gly Leu Val Gln Ala Gly Gly
   1 5 10 15
   Ser Leu Arg Leu Ser Cys Ala Ala Ser Gly Arg Ile Phe Ser Ala Tyr
   20 25 30
   Ala Met Gly Trp Phe Arg Gln Ala Pro Gly Lys Glu Arg Glu Phe Val
   35 40 45
   Ala Ala Ile Ser Arg Ser Gly Asp Ser Thr Asp Tyr Ala Asp Ser Val
   50 55 60
   Lys Gly Arg Phe Thr Ile Ser Arg Asp Ser Ala Lys Asn Met Val Tyr
   65 70 75 80
   Leu Gln Met Asn Ser Leu Lys Pro Glu Asp Thr Ala Leu Tyr His Cys
   85 90 95
   Ala Ala Arg Thr Val Ser Ala Pro Pro Ser Ala Ala Trp Gly Tyr Gly
   100 105 110
   Tyr Trp Gly Gln Gly Thr Gln Val Thr Val Ser Ser
   115 120
<210> 23
   <211> 130
   <212> PRT
   <213> Vicugna pacos
<400> 23
   Gln Val Gln Leu Gln Glu Ser Gly Gly Gly Leu Val Gln Pro Gly Gly
   1 5 10 15
   Ser Leu Arg Leu Ser Cys Ala Ala Ser Gly Arg Thr Phe Ser Asn Tyr
   20 25 30
   Val Asn Tyr Ala Met Gly Trp Phe Arg Gln Phe Pro Gly Lys Glu Arg
   35 40 45
   Glu Phe Val Ala Ser Ile Ser Trp Ser Ser Val Thr Thr Tyr Tyr Ala
   50 55 60
   Asp Ser Val Lys Gly Arg Phe Thr Ile Ser Arg Asp Asn Ala Lys Asn
   65 70 75 80
   Thr Val Tyr Leu Gln Met Asn Ser Leu Lys Pro Glu Asp Thr Ala Val
   85 90 95
   Tyr Tyr Cys Ala Ala His Leu Ala Gln Tyr Ser Asp Tyr Ala Tyr Arg
   100 105 110
   Asp Pro His Gln Phe Gly Ala Trp Gly Gln Gly Thr Gln Val Thr Val
   115 120 125
   Ser Ser
   130
<210> 24
   <211> 117
   <212> PRT
   <213> Vicugna pacos
<400> 24
   Gln Val Gln Leu Gln Glu Ser Gly Gly Gly Leu Val Gln Ala Gly Gly
   1               5                   10                  15
   Ser Leu Arg Leu Ser Cys Leu Ala Ser Gly Asp Thr Phe Ser Asn Tyr
               20                  25                  30
   Val Met Ala Trp Phe Arg Gln Ala Pro Gly Lys Glu Arg Glu Ile Val
           35                  40                  45
   Ala Ala Ile Arg Leu Ser Gly Ala Arg Tyr Val Pro Asp Ser Val Lys
       50                  55                  60
   Gly Arg Phe Thr Ile Ser Arg Asp Asn Ala Lys Asn Ala Met Tyr Leu
   65                  70                  75                  80
   Gln Met Thr Ser Leu Lys Pro Glu Asp Thr Ala Arg Tyr Tyr Cys Ala
                   85                  90                  95
   Ala Gly His Thr Trp Gly Gln Tyr Ala Tyr Trp Gly Gln Gly Thr Gln
               100                 105                 110
   Val Thr Val Ser Ser
           115
<210> 25
   <211> 119
   <212> PRT
   <213> Vicugna pacos
<400> 25
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Arg Thr Phe Ser Ser Ala
               20                  25                  30
   Ala Met Gly Trp Phe Arg Gln Ala Pro Gly Lys Glu Arg Glu Pro Val
           35                  40                  45
   Ala Leu Ile Asn Leu Asp Asp Gly Glu Thr Tyr Tyr Ala Asp Ile Ala
       50                  55                  60
   Lys Gly Arg Phe Thr Leu Ser Lys Asp Asn Ala Lys Asn Ser Val Tyr
   65                  70                  75                  80
   Leu Gln Met Asn Ser Leu Lys Pro Glu Asp Thr Ala Val Tyr Tyr Cys
                   85                  90                  95
   Ala Val Arg Gly Arg Phe Asp Asp Asn Tyr Glu Tyr Trp Gly Gln Gly
               100                 105                 110
   Thr Gln Val Thr Val Ser Ser
           115
<210> 26
   <211> 122
   <212> PRT
   <213> Vicugna pacos
<400> 26
   Gln Val Gln Leu Gln Glu Ser Gly Gly Gly Leu Val Gln Ala Gly Asp
   1               5                   10                  15
   Ser Leu Arg Leu Ser Cys Ala Ala Ser Gly Arg Thr Phe Ser Ile Asn
               20                  25                  30
   Tyr Met Gly Trp Tyr Arg Gln Ala Pro Gly Lys Gln Arg Glu Leu Val
           35                  40                  45
   Ala Ala Ile Thr Ser Gly Ser Gly Ser Thr Asn Tyr Ala Asp Ser Val
       50                  55                  60
   Lys Gly Arg Phe Thr Ile Ser Arg Asp Asn Ala Lys Lys Thr Met Tyr
   65                  70                  75                  80
   Leu Gln Met Asn Ser Leu Lys Pro Glu Asp Thr Ala Val Tyr Tyr Cys
                   85                  90                  95
   Asn Ala Asp Met Asp Ser Ser Leu Ser Gly Gly Tyr Val Asp Val Trp
               100                 105                 110
   Gly Gln Gly Thr Gln Val Thr Val Ser Ser
           115                 120
<210> 27
   <211> 112
   <212> PRT
   <213> Vicugna pacos
<400> 27
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Ser Thr Phe Ser Ile Asn
               20                  25                  30
   Asn Met Gly Trp Tyr Arg Gln Ala Pro Gly Lys Gln Arg Glu Leu Val
           35                  40                  45
   Ala Gly Ile Thr Gly Gly Asn Thr His Tyr Ala Asp Ser Val Lys Gly
       50                  55                  60
   Arg Phe Thr Ile Ser Arg Asp Asn Ala Lys Asn Thr Met Tyr Leu Gln
   65                  70                  75                  80
   Met Asn Ser Leu Lys Pro Glu Asp Thr Ala Val Tyr Tyr Cys Asn Ala
   85 90 95
   Asn Trp Gly Ala Tyr Trp Gly Gln Gly Thr Gln Val Thr Val Ser Ser
   100 105 110
<210> 28
   <211> 119
   <212> PRT
   <213> Vicugna pacos
<400> 28
   Gln Val Gln Leu Gln Glu Ser Gly Gly Gly Leu Val Gln Pro Gly Gly
   1 5 10 15
   Ser Leu Arg Leu Ser Cys Ala Ala Ser Gly Ser Ile Val Ser Ile Asn
   20 25 30
   Ala Met Gly Trp Tyr Arg Gln Ala Pro Gly Lys Gln Arg Glu Leu Val
   35 40 45
   Ala Leu Val Thr Gly Ser Gly Arg Thr Asn Leu Ala Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Lys Pro Glu Asp Thr Ala Val Tyr Tyr Cys Asn
   85 90 95
   Val Leu Val Ile Gly Pro Leu Glu Gly Tyr Asp Tyr Trp Gly Gln Gly
   100 105 110
   Thr Gln Val Thr Val Ser Ser
   115
<210> 29
   <211> 121
   <212> PRT
   <213> Vicugna pacos
<400> 29
   Gln Val Gln Leu Gln Glu Ser Gly Gly Gly Leu Val Gln Pro Gly Gly
   1 5 10 15
   Ser Leu Lys Leu Ser Cys Ala Ala Ser Gly Ser Ile Phe Ser Ile Lys
   20 25 30
   Thr Met Gly Trp Tyr Arg Gln Ala Pro Gly Lys Gln Arg Glu Leu Val
   35 40 45
   Ala Ala Val Ser Ser Gly Gly Ser Thr Asn Tyr Ala Asp Ser Val Lys
   50 55 60
   Gly Arg Phe Thr Ile Ser Arg Asp Asn Ala Lys Asn Ala Val Tyr Leu
   65 70 75 80
   Gln Met Asn Ser Leu Lys Pro Glu Asp Thr Ala Val Tyr Tyr Cys Asn
   85 90 95
   Ala Asp Gly Val Val Ala Trp Asp Gln Pro Tyr Asp Asn Tyr Trp Gly
   100 105 110
   Gln Gly Thr Gln Val Thr Val Ser Ser
   115 120
<210> 30
   <211> 118
   <212> PRT
   <213> Vicugna pacos
<400> 30
   Gln Val Gln Leu Gln Glu Ser Gly Gly Gly Leu Val Gln Ala Gly Gly
   1 5 10 15
   Ser Leu Arg Leu Ser Cys Val Asp Gln Gly Arg Thr Phe Ser Val Asn
   20 25 30
   Ala Met Ala Trp Tyr Arg Gln Ala Pro Gly Lys Gln Arg Glu Leu Val
   35 40 45
   Ala Ser Ile Thr Ser Ser Gly Leu Asp Thr Gln Tyr Ala Glu Gly Met
   50 55 60
   Lys Gly Arg Phe Thr Ile Ser Lys Gly Asn Asp Lys Phe Ser Thr Tyr
   65 70 75 80
   Leu Gln Met Asn Asn Leu Lys Pro Asp Asp Thr Ala Val Tyr Tyr Cys
   85 90 95
   Asn Ala Glu Arg Trp Asp Asn Gly Met Val Tyr Trp Gly Lys Gly Thr
   100 105 110
   Gln Val Thr Val Ser Ser
   115
<210> 31
   <211> 132
   <212> PRT
   <213> Vicugna pacos
<400> 31
   Gln Val Gln Leu Gln Glu Ser Gly Gly Gly Leu Val Gln Ala Gly Asp
   1               5                   10                  15
   Ser Leu Arg Leu Ser Cys Leu Ala Thr Gly Ser Met Phe Ser Ile Asn
               20                  25                  30
   Ala Trp Gly Trp Tyr Arg Gln Ala Pro Gly Lys Gln Arg Glu Leu Val
           35                  40                  45
   Ala Ser Ile Thr Ser Gly Gly Gly Ser Thr Glu Tyr Ala Glu Ser Val
       50                  55                  60
   Lys Gly Arg Phe Thr Ile Ser Arg Asp Ser Ala Lys Asn Met Leu Tyr
   65                  70                  75                  80
   Leu Gln Met Asn Ser Leu Arg Pro Glu Asp Thr Ala Val Tyr Tyr Cys
                   85                  90                  95
   Asn Ala Glu Arg Trp Asp Gly Tyr Ala Leu Gly Tyr Ser Pro Asn His
               100                 105                 110
   Gly Ser Gly His Arg Pro Tyr Asn Tyr Trp Gly Gln Gly Thr Gln Val
           115                 120                 125
   Thr Val Ser Ser
       130
<210> 32
   <211> 120
   <212> PRT
   <213> Vicugna pacos
<400> 32
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Ser Ile Phe Ser Ile Asn
               20                  25                  30
   Ala Trp Gly Trp Tyr Arg Gln Ala Pro Gly Lys Gln Arg Glu Leu Val
           35                  40                  45
   Ala Glu Ile Thr Ser Ser Gly Ser Thr Asn Tyr Ala Asp Ser Val Lys
       50                  55                  60
   Gly Arg Phe Thr Ile Ser Gly Asp Asn Ala Lys Asn Ser Val Tyr Leu
   65                  70                  75                  80
   His Met Asn Asn Leu Glu Pro Glu Asp Thr Ala Val Tyr Tyr Cys Lys
                   85                  90                  95
   Ala Val Ala Val Thr Phe Thr Thr Pro Arg Ser Asp Tyr Trp Gly Arg
               100                 105                 110
   Gly Thr Gln Val Thr Val Ser Ser
           115                 120
<210> 33
   <211> 116
   <212> PRT
   <213> Vicugna pacos
<400> 33
   Gln Val Gln Leu Gln Glu Ser Gly Gly Gly Leu Val Gln Pro Gly Gly
   1               5                   10                  15
   Ser Leu Arg Leu Ser Cys Ala Pro Ser Gly Ser Ile Ile Ser Ile Asn
               20                  25                  30
   Ala Met Ala Trp Tyr Arg Gln Ala Pro Gly Lys Glu Arg Glu Leu Val
           35                  40                  45
   Ala Ala Ile Ser Ser Gly Gly Ser Thr Tyr Tyr Ala Asp Ser Val Lys
       50                  55                  60
   Gly Arg Phe Thr Ile Ser Gly Asp Ile Ala Lys Asn Leu Leu Trp Leu
   65                  70                  75                  80
   Gln Met Asn Ser Leu Lys Pro Glu Asp Thr Ala Met Tyr Tyr Cys Ala
                   85                  90                  95
   Pro Gly Gly Gly Trp Arg Pro Gly Ala Trp Gly Gln Gly Thr Gln Val
               100                 105                 110
   Thr Val Ser Ser
           115
<210> 34
   <211> 116
   <212> PRT
   <213> Vicugna pacos
<400> 34
   Gln Val Gln Leu Gln Glu Ser Gly Gly Gly Leu Val Gln Pro Gly Gly
   1               5                   10                  15
   Ser Leu Arg Leu Ser Cys Ala Gly Ser Gly Phe Thr Val Ser Thr Ser
               20                  25                  30
   Met Ile Asn Trp Ala Arg Gln Val Pro Gly Lys Glu Leu Glu Trp Leu
           35                  40                  45
   Val Asp Val Leu Pro Ser Gly Ser Thr Tyr Tyr Ala Asp Pro Val Lys
       50                  55                  60
   Gly Arg Phe Thr Ile Ser Arg Asp Asn Ala Gln Asn Thr Ile Tyr Leu
   65                  70                  75                  80
   Gln Met Asn Tyr Leu Lys Pro Glu Asp Thr Ala Ile Tyr Tyr Cys Ala
                   85                  90                  95
   Ile Asn Arg Glu Thr Met Pro Pro Phe Arg Gly Gln Gly Thr Gln Val
               100                 105                 110
   Thr Val Ser Ser
           115
<210> 35
   <211> 121
   <212> PRT
   <213> Vicugna pacos
<400> 35
   Gln Val Gln Leu Gln Glu Ser Gly Gly Gly Leu Val Gln Pro Gly Gly
   1               5                   10                  15
   Ser Leu Arg Leu Ser Cys Thr Ala Ser Gly Phe Pro Phe Ser Ser Ala
               20                  25                  30
   Pro Met Ser Trp Val Arg Gln Ala Pro Gly Lys Glu Leu Glu Trp Val
           35                  40                  45
   Ser Tyr Ile Gly Tyr Thr Gly Thr Ile Thr Asp Tyr Ala Asn Ser Val
       50                  55                  60
   Lys Gly Arg Phe Thr Ile Ser Arg Asp Asn Ala Lys Asn Arg Leu Tyr
   65                  70                  75                  80
   Leu Gln Met Asn Ser Leu Lys Pro Glu Asp Thr Ala Val Tyr Phe Cys
                   85                  90                  95
   Ala Gln Gly Tyr Ala Arg Leu Ile Ala Asp Ser Asp Leu Val Arg Gly
               100                 105                 110
   Gln Gly Thr Gln Val Thr Val Ser Ser
           115                 120
<210> 36
   <211> 121
   <212> PRT
   <213> Vicugna pacos
<400> 36
   Gln Val Gln Leu Gln Glu Ser Gly Gly Arg Leu Gly Ala Ala Gly Gly
   1               5                   10                  15
   Ser Leu Arg Leu Ser Cys Thr Ala Ser Gly Phe Pro Phe Asn Ile Tyr
               20                  25                  30
   Pro Met Ser Trp Val Arg Gln Ala Pro Gly Lys Gly Phe Glu Trp Val
           35                  40                  45
   Ser Tyr Ile Ser His Gly Gly Thr Thr Thr Asp Tyr Ser Asp Ala Val
       50                  55                  60
   Lys Gly Arg Phe Thr Ile Ser Arg Asp Asn Ala Lys Asn Arg Leu Tyr
   65                  70                  75                  80
   Leu Gln Met Asp Ser Leu Lys Pro Glu Asp Thr Ala Val Tyr Phe Cys
                   85                  90                  95
   Ala Gln Gly Tyr Ala Arg Leu Met Thr Asp Ser Glu Leu Val Arg Gly
               100                 105                 110
   Gln Gly Thr Gln Val Thr Val Ser Ser
           115                 120
<210> 37
   <211> 25
   <212> PRT
   <213> Vicugna pacos
<400> 37
   Gln Val Gln Leu Gln Glu Ser Gly Gly Gly Leu Val Gln Pro Gly Gly
   1               5                   10                  15
   Ser Leu Arg Leu Ser Cys Ala Ala Ser
               20                  25
<210> 38
   <211> 25
   <212> PRT
   <213> Vicugna pacos
<400> 38
   Gln Val Gln Leu Gln Glu Ser Gly Gly Gly Leu Val Gln Pro Gly Gly
   1               5                   10                  15
   Ser Leu Arg Leu Ser Cys Ala Ala Ser
               20                  25
<210> 39
   <211> 25
   <212> PRT
   <213> Vicugna pacos
<400> 39
   Gln Val Gln Leu Gln Glu Ser Gly Gly Gly Leu Val Gln Ala Gly Asp
   1               5                   10                  15
   Ser Leu Arg Leu Ser Cys Ala Ala Ser
               20                  25
<210> 40
   <211> 25
   <212> PRT
   <213> Vicugna pacos
<400> 40
   Gln Val Gln Leu Gln Glu Ser Gly Gly Gly Leu Val Gln Ala Gly Gly
   1               5                   10                  15
   Ser Leu Arg Leu Ser Cys Ala Ala Ser
               20                  25
<210> 41
   <211> 25
   <212> PRT
   <213> Vicugna pacos
<400> 41
   Gln Val Gln Leu Gln Glu Ser Gly Gly Gly Leu Val Gln Pro Gly Gly
   1               5                   10                  15
   Ser Leu Arg Leu Ser Cys Ala Ala Ser
               20                  25
<210> 42
   <211> 25
   <212> PRT
   <213> Vicugna pacos
<400> 42
   Gln Val Gln Leu Gln Glu Ser Gly Gly Gly Leu Val Gln Pro Gly Gly
   1               5                   10                  15
   Ser Leu Lys Leu Ser Cys Ala Ala Ser
               20                  25
<210> 43
   <211> 25
   <212> PRT
   <213> Vicugna pacos
<400> 43
   Gln Val Gln Leu Gln Glu Ser Gly Gly Gly Leu Val Gln Ala Gly Asp
   1               5                   10                  15
   Ser Leu Ser Ile Ser Cys Ala Ala Ser
               20                  25
<210> 44
   <211> 25
   <212> PRT
   <213> Vicugna pacos
<400> 44
   Gln Val Gln Leu Gln Glu Ser Gly Gly Gly Leu Val Gln Pro Gly Glu
   1               5                   10                  15
   Ser Leu Arg Leu Ser Cys Lys Leu Ser
               20                  25
<210> 45
   <211> 25
   <212> PRT
   <213> Vicugna pacos
<400> 45
   Gln Val Gln Leu Gln Glu Ser Gly Gly Gly Leu Val Gln Pro Gly Gly
   1               5                   10                  15
   Ser Leu Arg Leu Ser Cys Val Val Ser
               20                  25
<210> 46
   <211> 25
   <212> PRT
   <213> Vicugna pacos
<400> 46
   Gln Val Gln Leu Gln Glu Ser Gly Gly Gly Leu Val Gln Pro Gly Gly
   1               5                   10                  15
   Ser Leu Met Leu Ser Cys Ala Ala Ser
               20                  25
<210> 47
   <211> 25
   <212> PRT
   <213> Vicugna pacos
<400> 47
   Gln Val Gln Leu Gln Glu Ser Gly Gly Thr Leu Val Gln Pro Gly Gly
   1               5                   10                  15
   Ser Leu Arg Leu Ser Cys Ala Ala Ser
               20                  25
<210> 48
   <211> 25
   <212> PRT
   <213> Vicugna pacos
<400> 48
   Gln Val Gln Leu Gln Glu Ser Gly Gly Gly Leu Val Gln Pro Gly Gly
   1               5                   10                  15
   Ser Leu Gly Leu Ser Cys Ala Ala Ser
               20                  25
<210> 49
   <211> 26
   <212> PRT
   <213> Vicugna pacos
<400> 49
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly
               20                  25
<210> 50
   <211> 25
   <212> PRT
   <213> Vicugna pacos
<400> 50
   Gln Val Gln Leu Gln Glu Ser Gly Gly Gly Leu Val Gln Ala Gly Gly
   1               5                   10                  15
   Ser Leu Arg Leu Ser Cys Ala Ala Ser
               20                  25
<210> 51
   <211> 25
   <212> PRT
   <213> Vicugna pacos
<400> 51
   Gln Val Gln Leu Gln Glu Ser Gly Gly Gly Leu Val Gln Pro Gly Gly
   1 5 10 15
   Ser Leu Arg Leu Ser Cys Thr Ala Thr
   20 25
<210> 52
   <211> 25
   <212> PRT
   <213> Vicugna pacos
<400> 52
   Gln Val Gln Leu Gln Glu Ser Gly Gly Gly Leu Val Gln Ala Gly Gly
   1 5 10 15
   Ser Leu Arg Leu Ser Cys Ala Ala Ser
   20 25
<210> 53
   <211> 25
   <212> PRT
   <213> Vicugna pacos
<400> 53
   Gln Val Gln Leu Gln Glu Ser Gly Gly Gly Leu Val Gln Pro Gly Gly
   1 5 10 15
   Ser Leu Arg Leu Ser Cys Ala Ala Ser
   20 25
<210> 54
   <211> 25
   <212> PRT
   <213> Vicugna pacos
<400> 54
   Gln Val Gln Leu Gln Glu Ser Gly Gly Gly Leu Val Gln Ala Gly Gly
   1 5 10 15
   Ser Leu Arg Leu Ser Cys Leu Ala Ser
   20 25
<210> 55
   <211> 25
   <212> PRT
   <213> Vicugna pacos
<400> 55
   Gln Val Gln Leu Gln Glu Ser Gly Gly Gly Leu Val Gln Ala Gly Gly
   1               5                   10                  15
   Ser Leu Arg Leu Ser Cys Ala Ala Ser
               20                  25
<210> 56
   <211> 25
   <212> PRT
   <213> Vicugna pacos
<400> 56
   Gln Val Gln Leu Gln Glu Ser Gly Gly Gly Leu Val Gln Ala Gly Asp
   1               5                   10                  15
   Ser Leu Arg Leu Ser Cys Ala Ala Ser
               20                  25
<210> 57
   <211> 25
   <212> PRT
   <213> Vicugna pacos
<400> 57
   Gln Val Gln Leu Gln Glu Ser Gly Gly Gly Leu Val Gln Ala Gly Gly
   1               5                   10                  15
   Ser Leu Arg Leu Ser Cys Ala Ala Ser
               20                  25
<210> 58
   <211> 25
   <212> PRT
   <213> Vicugna pacos
<400> 58
   Gln Val Gln Leu Gln Glu Ser Gly Gly Gly Leu Val Gln Pro Gly Gly
   1               5                   10                  15
   Ser Leu Arg Leu Ser Cys Ala Ala Ser
               20                  25
<210> 59
   <211> 25
   <212> PRT
   <213> Vicugna pacos
<400> 59
   Gln Val Gln Leu Gln Glu Ser Gly Gly Gly Leu Val Gln Pro Gly Gly
   1               5                   10                  15
   Ser Leu Lys Leu Ser Cys Ala Ala Ser
               20                  25
<210> 60
   <211> 25
   <212> PRT
   <213> Vicugna pacos
<400> 60
   Gln Val Gln Leu Gln Glu Ser Gly Gly Gly Leu Val Gln Ala Gly Gly
   1               5                   10                  15
   Ser Leu Arg Leu Ser Cys Val Asp Gln
               20                  25
<210> 61
   <211> 25
   <212> PRT
   <213> Vicugna pacos
<400> 61
   Gln Val Gln Leu Gln Glu Ser Gly Gly Gly Leu Val Gln Ala Gly Asp
   1               5                   10                  15
   Ser Leu Arg Leu Ser Cys Leu Ala Thr
               20                  25
<210> 62
   <211> 25
   <212> PRT
   <213> Vicugna pacos
<400> 62
   Gln Val Gln Leu Gln Glu Ser Gly Gly Gly Leu Val Gln Pro Gly Gly
   1               5                   10                  15
   Ser Leu Arg Leu Ser Cys Ala Ala Ser
               20                  25
<210> 63
   <211> 25
   <212> PRT
   <213> Vicugna pacos
<400> 63
   Gln Val Gln Leu Gln Glu Ser Gly Gly Gly Leu Val Gln Pro Gly Gly
   1               5                   10                  15
   Ser Leu Arg Leu Ser Cys Ala Pro Ser
               20                  25
<210> 64
   <211> 25
   <212> PRT
   <213> Vicugna pacos
<400> 64
   Gln Val Gln Leu Gln Glu Ser Gly Gly Gly Leu Val Gln Pro Gly Gly
   1               5                   10                  15
   Ser Leu Arg Leu Ser Cys Ala Gly Ser
               20                  25
<210> 65
   <211> 25
   <212> PRT
   <213> Vicugna pacos
<400> 65
   Gln Val Gln Leu Gln Glu Ser Gly Gly Gly Leu Val Gln Pro Gly Gly
   1               5                   10                  15
   Ser Leu Arg Leu Ser Cys Thr Ala Ser
               20                  25
<210> 66
   <211> 25
   <212> PRT
   <213> Vicugna pacos
<400> 66
   Gln Val Gln Leu Gln Glu Ser Gly Gly Arg Leu Gly Ala Ala Gly Gly
   1               5                   10                  15
   Ser Leu Arg Leu Ser Cys Thr Ala Ser
               20                  25
<210> 67
   <211> 10
   <212> PRT
   <213> Vicugna pacos
<400> 67
   Gly Phe Ser Leu Asp Tyr Tyr Ala Ile Gly
   1               5                   10
<210> 68
   <211> 10
   <212> PRT
   <213> Vicugna pacos
<400> 68
   Gly Phe Thr Leu Asp Tyr Tyr Ala Ile Gly
   1 5 10
<210> 69
   <211> 10
   <212> PRT
   <213> Vicugna pacos
<400> 69
   Gly Arg Thr Phe Ser Ile Asn Tyr Met Gly
   1 5 10
<210> 70
   <211> 10
   <212> PRT
   <213> Vicugna pacos
<400> 70
   Gly Phe Thr Asp Asp Asp Tyr Asp Ile Gly
   1 5 10
<210> 71
   <211> 10
   <212> PRT
   <213> Vicugna pacos
<400> 71
   Gly Phe Thr Leu Asp Asn Tyr Thr Val Ala
   1 5 10
<210> 72
   <211> 10
   <212> PRT
   <213> Vicugna pacos
<400> 72
   Gly Ser Ile Phe Ser Ile Lys Thr Met Gly
   1 5 10
<210> 73
   <211> 10
   <212> PRT
   <213> Vicugna pacos
<400> 73
   Gly Asp Thr Phe Asn His Tyr Ser Trp Gly
   1 5 10
<210> 74
   <211> 10
   <212> PRT
   <213> Vicugna pacos
<400> 74
   Gly Phe Thr Leu Asp Tyr Tyr Asp Ile Gly
   1 5 10
<210> 75
   <211> 10
   <212> PRT
   <213> Vicugna pacos
<400> 75
   Gly Ser Phe Leu Ser Ile Asn His Met Gly
   1 5 10
<210> 76
   <211> 10
   <212> PRT
   <213> Vicugna pacos
<400> 76
   Gly Asn Ile Phe Thr Ile Asn Arg Met Gly
   1 5 10
<210> 77
   <211> 10
   <212> PRT
   <213> Vicugna pacos
<400> 77
   Gly Ser Thr Phe Ser Ile Asn Asn Met Gly
   1 5 10
<210> 78
   <211> 10
   <212> PRT
   <213> Vicugna pacos
<400> 78
   Gly Arg Ile Ala Ser Ile Ser Ala Met Gly
   1 5 10
<210> 79
   <211> 11
   <212> PRT
   <213> Vicugna pacos
<400> 79
   Pro Gly Phe Lys Leu Asp Tyr Tyr Ala Ile Ala
   1 5 10
<210> 80
   <211> 10
   <212> PRT
   <213> Vicugna pacos
<400> 80
   Gly Gly Thr Phe Asp Asp Ser Val Ile Gly
   1 5 10
<210> 81
   <211> 10
   <212> PRT
   <213> Vicugna pacos
<400> 81
   Gly Phe Thr Leu Lys Asn His His Ile Gly
   1 5 10
<210> 82
   <211> 10
   <212> PRT
   <213> Vicugna pacos
<400> 82
   Gly Arg Ile Phe Ser Ala Tyr Ala Met Gly
   1 5 10
<210> 83
   <211> 13
   <212> PRT
   <213> Vicugna pacos
<400> 83
   Gly Arg Thr Phe Ser Asn Tyr Val Asn Tyr Ala Met Gly
   1 5 10
<210> 84
   <211> 10
   <212> PRT
   <213> Vicugna pacos
<400> 84
   Gly Asp Thr Phe Ser Asn Tyr Val Met Ala
   1 5 10
<210> 85
   <211> 10
   <212> PRT
   <213> Vicugna pacos
<400> 85
   Gly Arg Thr Phe Ser Ser Ala Ala Met Gly
   1 5 10
<210> 86
   <211> 10
   <212> PRT
   <213> Vicugna pacos
<400> 86
   Gly Arg Thr Phe Ser Ile Asn Tyr Met Gly
   1 5 10
<210> 87
   <211> 10
   <212> PRT
   <213> Vicugna pacos
<400> 87
   Gly Ser Thr Phe Ser Ile Asn Asn Met Gly
   1 5 10
<210> 88
   <211> 10
   <212> PRT
   <213> Vicugna pacos
<400> 88
   Gly Ser Ile Val Ser Ile Asn Ala Met Gly
   1 5 10
<210> 89
   <211> 10
   <212> PRT
   <213> Vicugna pacos
<400> 89
   Gly Ser Ile Phe Ser Ile Lys Thr Met Gly
   1 5 10
<210> 90
   <211> 10
   <212> PRT
   <213> Vicugna pacos
<400> 90
   Gly Arg Thr Phe Ser Val Asn Ala Met Ala
   1 5 10
<210> 91
   <211> 10
   <212> PRT
   <213> Vicugna pacos
<400> 91
   Gly Ser Met Phe Ser Ile Asn Ala Trp Gly
   1 5 10
<210> 92
   <211> 10
   <212> PRT
   <213> Vicugna pacos
<400> 92
   Gly Ser Ile Phe Ser Ile Asn Ala Trp Gly
   1 5 10
<210> 93
   <211> 10
   <212> PRT
   <213> Vicugna pacos
<400> 93
   Gly Ser Ile Ile Ser Ile Asn Ala Met Ala
   1 5 10
<210> 94
   <211> 10
   <212> PRT
   <213> Vicugna pacos
<400> 94
   Gly Phe Thr Val Ser Thr Ser Met Ile Asn
   1 5 10
<210> 95
   <211> 10
   <212> PRT
   <213> Vicugna pacos
<400> 95
   Gly Phe Pro Phe Ser Ser Ala Pro Met Ser
   1 5 10
<210> 96
   <211> 10
   <212> PRT
   <213> Vicugna pacos
<400> 96
   Gly Phe Pro Phe Asn Ile Tyr Pro Met Ser
   1 5 10
<210> 97
   <211> 14
   <212> PRT
   <213> Vicugna pacos
<400> 97
   Trp Phe Arg Gln Ala Pro Gly Lys Glu Arg Glu Gly Ile Ser
   1               5                   10
<210> 98
   <211> 14
   <212> PRT
   <213> Vicugna pacos
<400> 98
   Trp Phe Arg Gln Ala Pro Gly Lys Glu Arg Glu Gly Ile Ser
   1               5                   10
<210> 99
   <211> 14
   <212> PRT
   <213> Vicugna pacos
<400> 99
   Trp Tyr Arg Gln Ala Pro Gly Lys Gln Arg Glu Leu Val Ala
   1               5                   10
<210> 100
   <211> 14
   <212> PRT
   <213> Vicugna pacos
<400> 100
   Trp Phe Arg Gln Ala Pro Gly Lys Glu Arg Glu Gly Val Ser
   1               5                   10
<210> 101
   <211> 14
   <212> PRT
   <213> Vicugna pacos
<400> 101
   Trp Phe Arg Gln Ala Pro Gly Lys Glu Arg Glu Gly Val Ser
           1               5                   10
<210> 102
   <211> 14
   <212> PRT
   <213> Vicugna pacos
<400> 102
   Trp Tyr Arg Gln Ala Pro Gly Lys Gln Arg Glu Leu Val Ala
   1               5                   10
<210> 103
   <211> 14
   <212> PRT
   <213> Vicugna pacos
<400> 103
   Trp Phe Arg Gln Ala Pro Gly Lys Ala Arg Glu Phe Val Ala
   1               5                   10
<210> 104
   <211> 14
   <212> PRT
   <213> Vicugna pacos
<400> 104
   Trp Phe Arg Gln Ala Pro Gly Lys Glu Arg Glu Gly Val Ser
   1               5                   10
<210> 105
   <211> 14
   <212> PRT
   <213> Vicugna pacos
<400> 105
   Trp Tyr Arg Gln Val Ser Gly Glu Gln Arg Glu Leu Val Ala
   1               5                   10
<210> 106
   <211> 14
   <212> PRT
   <213> Vicugna pacos
<400> 106
   Trp Tyr Arg Gln Ala Pro Gly Lys Gln Arg Glu Leu Val Ala
   1               5                   10
<210> 107
   <211> 14
   <212> PRT
   <213> Vicugna pacos
<400> 107
   Trp Tyr Arg Gln Ala Pro Gly Lys Gln Arg Glu Leu Val Ala
   1               5                   10
<210> 108
   <211> 14
   <212> PRT
   <213> Vicugna pacos
<400> 108
   Trp Tyr Arg Gln Ala Pro Gly Lys Gln Arg Glu Leu Val Ala
   1 5 10
<210> 109
   <211> 14
   <212> PRT
   <213> Vicugna pacos
<400> 109
   Trp Phe Arg Gln Ala Pro Gly Lys Glu Arg Glu Gly Val Ser
   1 5 10
<210> 110
   <211> 14
   <212> PRT
   <213> Vicugna pacos
<400> 110
   Trp Phe Arg Gln Ala Pro Gly Lys Glu Arg Glu Gly Val Ser
   1 5 10
<210> 111
   <211> 14
   <212> PRT
   <213> Vicugna pacos
<400> 111
   Trp Leu Arg Gln Ala Pro Gly Lys Glu Arg Glu Gly Val Ala
   1 5 10
<210> 112
   <211> 14
   <212> PRT
   <213> Vicugna pacos
<400> 112
   Trp Phe Arg Gln Ala Pro Gly Lys Glu Arg Glu Phe Val Ala
   1 5 10
<210> 113
   <211> 14
   <212> PRT
   <213> Vicugna pacos
<400> 113
   Trp Phe Arg Gln Phe Pro Gly Lys Glu Arg Glu Phe Val Ala
   1 5 10
<210> 114
   <211> 14
   <212> PRT
   <213> Vicugna pacos
<400> 114
   Trp Phe Arg Gln Ala Pro Gly Lys Glu Arg Glu Ile Val Ala
   1 5 10
<210> 115
   <211> 14
   <212> PRT
   <213> Vicugna pacos
<400> 115
   Trp Phe Arg Gln Ala Pro Gly Lys Glu Arg Glu Pro Val Ala
   1 5 10
<210> 116
   <211> 14
   <212> PRT
   <213> Vicugna pacos
<400> 116
   Trp Tyr Arg Gln Ala Pro Gly Lys Gln Arg Glu Leu Val Ala
   1 5 10
<210> 117
   <211> 14
   <212> PRT
   <213> Vicugna pacos
<400> 117
   Trp Tyr Arg Gln Ala Pro Gly Lys Gln Arg Glu Leu Val Ala
   1 5 10
<210> 118
   <211> 14
   <212> PRT
   <213> Vicugna pacos
<400> 118
   Trp Tyr Arg Gln Ala Pro Gly Lys Gln Arg Glu Leu Val Ala
   1 5 10
<210> 119
   <211> 14
   <212> PRT
   <213> Vicugna pacos
<400> 119
   Trp Tyr Arg Gln Ala Pro Gly Lys Gln Arg Glu Leu Val Ala
   1 5 10
<210> 120
   <211> 14
   <212> PRT
   <213> Vicugna pacos
<400> 120
   Trp Tyr Arg Gln Ala Pro Gly Lys Gln Arg Glu Leu Val Ala
   1               5                   10
<210> 121
   <211> 14
   <212> PRT
   <213> Vicugna pacos
<400> 121
   Trp Tyr Arg Gln Ala Pro Gly Lys Gln Arg Glu Leu Val Ala
   1               5                   10
<210> 122
   <211> 14
   <212> PRT
   <213> Vicugna pacos
<400> 122
   Trp Tyr Arg Gln Ala Pro Gly Lys Gln Arg Glu Leu Val Ala
   1               5                   10
<210> 123
   <211> 14
   <212> PRT
   <213> Vicugna pacos
<400> 123
   Trp Tyr Arg Gln Ala Pro Gly Lys Glu Arg Glu Leu Val Ala
   1               5                   10
<210> 124
   <211> 14
   <212> PRT
   <213> Vicugna pacos
<400> 124
   Trp Ala Arg Gln Val Pro Gly Lys Glu Leu Glu Trp Leu Val
   1               5                   10
<210> 125
   <211> 14
   <212> PRT
   <213> Vicugna pacos
<400> 125
   Trp Val Arg Gln Ala Pro Gly Lys Glu Leu Glu Trp Val Ser
   1 5 10
<210> 126
   <211> 14
   <212> PRT
   <213> Vicugna pacos
<400> 126
   Trp Val Arg Gln Ala Pro Gly Lys Gly Phe Glu Trp Val Ser
   1 5 10
<210> 127
   <211> 9
   <212> PRT
   <213> Vicugna pacos
<400> 127
   Cys Ile Ser Tyr Lys Gly Gly Ser Thr
   1 5
<210> 128
   <211> 9
   <212> PRT
   <213> Vicugna pacos
<400> 128
   Cys Ile Ser Tyr Lys Gly Gly Ser Thr
   1 5
<210> 129
   <211> 9
   <212> PRT
   <213> Vicugna pacos
<400> 129
   Ala Ile Thr Ser Gly Ser Gly Ser Thr
   1 5
<210> 130
   <211> 9
   <212> PRT
   <213> Vicugna pacos
<400> 130
   Cys Ile Ser Ser Ser Asp Gly Ser Thr
   1 5
<210> 131
   <211> 9
   <212> PRT
   <213> Vicugna pacos
<400> 131
   Cys Ile Ser Ser Ser Gly Gly Ser Thr
   1               5
<210> 132
   <211> 8
   <212> PRT
   <213> Vicugna pacos
<400> 132
   Ala Ile Thr Ser Gly Gly Ser Thr
   1               5
<210> 133
   <211> 8
   <212> PRT
   <213> Vicugna pacos
<400> 133
   Ala Ile Ser Trp Asn Gly Gly Ser
   1               5
<210> 134
   <211> 9
   <212> PRT
   <213> Vicugna pacos
<400> 134
   Cys Ile Ser Ser Ile Gly Gly Ser Ala
   1               5
<210> 135
   <211> 8
   <212> PRT
   <213> Vicugna pacos
<400> 135
   Ala Ile Thr Ser Gly Gly Ser Thr
   1               5
<210> 136
   <211> 8
   <212> PRT
   <213> Vicugna pacos
<400> 136
   Ala Ile Thr Ser Gly Gly Asn Thr
   1               5
<210> 137
   <211> 7
   <212> PRT
   <213> Vicugna pacos
<400> 137
   Gly Ile Thr Gly Gly Asn Thr
   1 5
<210> 138
   <211> 8
   <212> PRT
   <213> Vicugna pacos
<400> 138
   Ala Ile Thr Gly Ser Gly Arg Thr
   1 5
<210> 139
   <211> 10
   <212> PRT
   <213> Vicugna pacos
<400> 139
   Cys Ile Gly Gly Ser Gly Ser Gly Leu Thr
   1 5 10
<210> 140
   <211> 9
   <212> PRT
   <213> Vicugna pacos
<400> 140
   Cys Ile Ser Ser Asn Asp Gly Thr Thr
   1 5
<210> 141
   <211> 9
   <212> PRT
   <213> Vicugna pacos
<400> 141
   Ser Ile Asn Ser Ser Gly Gly Ser Thr
   1 5
<210> 142
   <211> 9
   <212> PRT
   <213> Vicugna pacos
<400> 142
   Ala Ile Ser Arg Ser Gly Asp Ser Thr
   1 5
<210> 143
   <211> 9
   <212> PRT
   <213> Vicugna pacos
<400> 143
   Ser Ile Ser Trp Ser Ser Val Thr Thr
   1               5
<210> 144
   <211> 8
   <212> PRT
   <213> Vicugna pacos
<400> 144
   Ala Ile Arg Leu Ser Gly Ala Arg
   1               5
<210> 145
   <211> 9
   <212> PRT
   <213> Vicugna pacos
<400> 145
   Leu Ile Asn Leu Asp Asp Gly Glu Thr
   1               5
<210> 146
   <211> 9
   <212> PRT
   <213> Vicugna pacos
<400> 146
   Ala Ile Thr Ser Gly Ser Gly Ser Thr
   1               5
<210> 147
   <211> 7
   <212> PRT
   <213> Vicugna pacos
<400> 147
   Gly Ile Thr Gly Gly Asn Thr
   1               5
<210> 148
   <211> 8
   <212> PRT
   <213> Vicugna pacos
<400> 148
   Leu Val Thr Gly Ser Gly Arg Thr
   1               5
<210> 149
   <211> 8
   <212> PRT
   <213> Vicugna pacos
<400> 149
   Ala Val Ser Ser Gly Gly Ser Thr
   1 5
<210> 150
   <211> 9
   <212> PRT
   <213> Vicugna pacos
<400> 150
   Ser Ile Thr Ser Ser Gly Leu Asp Thr
   1 5
<210> 151
   <211> 9
   <212> PRT
   <213> Vicugna pacos
<400> 151
   Ser Ile Thr Ser Gly Gly Gly Ser Thr
   1 5
<210> 152
   <211> 8
   <212> PRT
   <213> Vicugna pacos
<400> 152
   Glu Ile Thr Ser Ser Gly Ser Thr
   1 5
<210> 153
   <211> 8
   <212> PRT
   <213> Vicugna pacos
<400> 153
   Ala Ile Ser Ser Gly Gly Ser Thr
   1 5
<210> 154
   <211> 8
   <212> PRT
   <213> Vicugna pacos
<400> 154
   Asp Val Leu Pro Ser Gly Ser Thr
   1               5
<210> 155
   <211> 9
   <212> PRT
   <213> Vicugna pacos
<400> 155
   Tyr Ile Gly Tyr Thr Gly Thr Ile Thr
   1               5
<210> 156
   <211> 9
   <212> PRT
   <213> Vicugna pacos
<400> 156
   Tyr Ile Ser His Gly Gly Thr Thr Thr
   1               5
<210> 157
   <211> 40
   <212> PRT
   <213> Vicugna pacos
<400> 157
   Thr Tyr Ala Asp Ser Val Lys Gly Arg Phe Thr Ile Ser Lys Asp Asn
   1               5                   10                  15
   Ala Lys Asn Thr Ala Tyr Leu Gln Met Asn Ser Leu Lys Pro Glu Asp
               20                  25                  30
   Thr Gly Ile Tyr Ser Cys Ala Ala
           35                  40
<210> 158
   <211> 40
   <212> PRT
   <213> Vicugna pacos
<400> 158
   Thr Tyr Ala Asp Ser Val Lys Gly Arg Phe Thr Ile Ser Lys Asp Asn
   1               5                   10                  15
   Ala Lys Asn Thr Ala Tyr Leu Gln Met Asn Asn Leu Lys Pro Glu Asp
               20                  25                  30
   Thr Gly Ile Tyr Tyr Cys Ala Ala
           35                  40
<210> 159
   <211> 40
   <212> PRT
   <213> Vicugna pacos
<400> 159
   Asn Tyr Ala Asp Ser Val Lys Gly Arg Phe Thr Ile Ser Arg Asp Asn
   1               5                   10                  15
   Ala Lys Asn Thr Met Tyr Leu Gln Met Asn Ser Leu Lys Pro Glu Asp
               20                  25                  30
   Thr Ala Val Tyr Tyr Cys Asn Ala
           35                  40
<210> 160
   <211> 40
   <212> PRT
   <213> Vicugna pacos
<400> 160
   Tyr Tyr Ala Asp Ser Val Lys Gly Arg Phe Thr Ile Ser Ser Asp Asn
   1               5                   10                  15
   Ala Lys Asn Thr Val Tyr Leu Gln Met Asn Ser Leu Lys Pro Glu Asp
               20                  25                  30
   Thr Ala Val Tyr Tyr Cys Ala Ala
           35                  40
<210> 161
   <211> 40
   <212> PRT
   <213> Vicugna pacos
<400> 161
   Asn Tyr Ala Asp Ser Val Lys Gly Arg Phe Thr Ile Ser Arg Asp Asn
   1               5                   10                  15
   Ser Lys Lys Ser Val Tyr Leu Gln Met Asn Ser Leu Lys Pro Glu Asp
               20                  25                  30
   Thr Ala Ile Tyr Thr Cys Ala Ala
           35                  40
<210> 162
   <211> 40
   <212> PRT
   <213> Vicugna pacos
<400> 162
   Asn Tyr Ala Asp Ser Val Lys Gly Arg Phe Thr Ile Ser Arg Asp Asn
   1               5                   10                  15
   Ala Lys Asn Thr Val Tyr Leu Gln Met Asn Ser Leu Lys Pro Glu Asp
               20                  25                  30
   Thr Ala Val Tyr Tyr Cys Asn Ala
           35                  40
<210> 163
   <211> 40
   <212> PRT
   <213> Vicugna pacos
<400> 163
   Lys Tyr Ala Asp Ser Val Lys Gly Arg Phe Ala Ile Ser Arg Asp Ile
   1               5                   10                  15
   Ala Lys Asn Thr Val Ser Leu Gln Met Asn Ser Leu Glu Pro Glu Asp
               20                  25                  30
   Thr Ala Val Tyr Tyr Cys Ala Ala
           35                  40
<210> 164
   <211> 40
   <212> PRT
   <213> Vicugna pacos
<400> 164
   Asn Tyr Ala Asp Ser Val Lys Gly Arg Phe Thr Ile Ser Arg Asp Asn
   1               5                   10                  15
   Val Lys Asn Thr Val Tyr Leu Gln Met Asn Ser Leu Lys Pro Glu Asp
               20                  25                  30
   Thr Ala Ile Tyr Tyr Cys Ala Ala
           35                  40
<210> 165
   <211> 40
   <212> PRT
   <213> Vicugna pacos
<400> 165
   Asn Tyr Ala Asp Ser Val Lys Gly Arg Phe Thr Ile Ser Arg Asp Ser
   1               5                   10                  15
   Ala Lys Asn Thr Val Tyr Leu Gln Met Asn Ser Leu Lys Pro Glu Asp
               20                  25                  30
   Thr Ala Val Tyr Tyr Cys Asn Ala
           35                  40
<210> 166
   <211> 40
   <212> PRT
   <213> Vicugna pacos
<400> 166
   Asn Tyr Ala Asp Ser Val Lys Gly Arg Phe Thr Ile Ser Arg Asp Asn
   1               5                   10                  15
   Ala Lys Asn Thr Val Tyr Leu Gln Met Asn Ser Leu Lys Pro Glu Asp
               20                  25                  30
   Thr Ala Val Tyr Tyr Cys Asn Ala
           35                  40
<210> 167
   <211> 40
   <212> PRT
   <213> Vicugna pacos
<400> 167
   His Tyr Ala Asp Ser Val Lys Gly Arg Phe Thr Ile Ser Arg Asp Asn
   1               5                   10                  15
   Ala Lys Asn Thr Met Tyr Leu Gln Met Asn Gly Leu Lys Pro Glu Asp
               20                  25                  30
   Thr Ala Val Tyr Tyr Cys Asn Ala
           35                  40
<210> 168
   <211> 40
   <212> PRT
   <213> Vicugna pacos
<400> 168
   Asn Tyr Ala Asp Ser Val Lys Gly Arg Phe Thr Ile Ser Arg Asp Asn
   1 5 10 15
   Ala Lys Asn Thr Val Tyr Leu Gln Met Asn Ser Leu Lys Pro Glu Asp
   20 25 30
   Thr Ala Val Tyr Tyr Cys Asn Leu
   35 40
<210> 169
   <211> 41
   <212> PRT
   <213> Vicugna pacos
<400> 169
   Thr Tyr Val Glu Asn Ser Val Lys Asp Arg Phe Thr Ile Ser Arg Asp
   1 5 10 15
   Asn Ala Gln Asn Thr Val Tyr Leu His Met Asn Ser Leu Lys Pro Glu
   20 25 30
   Asp Thr Gly Ile Tyr Tyr Cys Ala Ala
   35 40
<210> 170
   <211> 40
   <212> PRT
   <213> Vicugna pacos
<400> 170
   His Tyr Ala Ser Pro Val Lys Gly Arg Phe Thr Ile Ser Ser Asp Asn
   1 5 10 15
   Ala Lys Asn Thr Val Tyr Leu Gln Met Asn Ser Leu Lys Pro Glu Asp
   20 25 30
   Thr Ala Val Tyr Tyr Cys Ala Ala
   35 40
<210> 171
   <211> 40
   <212> PRT
   <213> Vicugna pacos
<400> 171
   Asn Tyr Ala Asp Ser Val Gln Gly Arg Phe Thr Ile Ser Arg Asp Asn
   1               5                   10                  15
   Ala Lys Asn Thr Val Phe Leu Gln Met Asn Ser Leu Lys Ser Glu Asp
               20                  25                  30
   Thr Ala Val Tyr Tyr Cys Ala Arg
           35                  40
<210> 172
   <211> 40
   <212> PRT
   <213> Vicugna pacos
<400> 172
   Asp Tyr Ala Asp Ser Val Lys Gly Arg Phe Thr Ile Ser Arg Asp Ser
   1               5                   10                  15
   Ala Lys Asn Met Val Tyr Leu Gln Met Asn Ser Leu Lys Pro Glu Asp
               20                  25                  30
   Thr Ala Leu Tyr His Cys Ala Ala
           35                  40
<210> 173
   <211> 40
   <212> PRT
   <213> Vicugna pacos
<400> 173
   Tyr Tyr Ala Asp Ser Val Lys Gly Arg Phe Thr Ile Ser Arg Asp Asn
   1               5                   10                  15
   Ala Lys Asn Thr Val Tyr Leu Gln Met Asn Ser Leu Lys Pro Glu Asp
               20                  25                  30
   Thr Ala Val Tyr Tyr Cys Ala Ala
           35                  40
<210> 174
   <211> 40
   <212> PRT
   <213> Vicugna pacos
<400> 174
   Tyr Val Pro Asp Ser Val Lys Gly Arg Phe Thr Ile Ser Arg Asp Asn
   1               5                   10                  15
   Ala Lys Asn Ala Met Tyr Leu Gln Met Thr Ser Leu Lys Pro Glu Asp
               20                  25                  30
   Thr Ala Arg Tyr Tyr Cys Ala Ala
           35                  40
<210> 175
   <211> 40
   <212> PRT
   <213> Vicugna pacos
<400> 175
   Tyr Tyr Ala Asp Ile Ala Lys Gly Arg Phe Thr Leu Ser Lys Asp Asn
   1               5                   10                  15
   Ala Lys Asn Ser Val Tyr Leu Gln Met Asn Ser Leu Lys Pro Glu Asp
               20                  25                  30
   Thr Ala Val Tyr Tyr Cys Ala Val
           35                  40
<210> 176
   <211> 40
   <212> PRT
   <213> Vicugna pacos
<400> 176
   Asn Tyr Ala Asp Ser Val Lys Gly Arg Phe Thr Ile Ser Arg Asp Asn
   1               5                   10                  15
   Ala Lys Lys Thr Met Tyr Leu Gln Met Asn Ser Leu Lys Pro Glu Asp
               20                  25                  30
   Thr Ala Val Tyr Tyr Cys Asn Ala
           35                  40
<210> 177
   <211> 40
   <212> PRT
   <213> Vicugna pacos
<400> 177
   His Tyr Ala Asp Ser Val Lys Gly Arg Phe Thr Ile Ser Arg Asp Asn
   1               5                   10                  15
   Ala Lys Asn Thr Met Tyr Leu Gln Met Asn Ser Leu Lys Pro Glu Asp
               20                  25                  30
   Thr Ala Val Tyr Tyr Cys Asn Ala
           35                  40
<210> 178
   <211> 40
   <212> PRT
   <213> Vicugna pacos
<400> 178
   Asn Leu Ala Asp Ser Val Lys Gly Arg Phe Thr Ile Ser Arg Asp Asn
   1               5                   10                  15
   Ala Lys Asn Thr Val Tyr Leu Gln Met Asn Ser Leu Lys Pro Glu Asp
               20                  25                  30
   Thr Ala Val Tyr Tyr Cys Asn Val
           35                  40
<210> 179
   <211> 40
   <212> PRT
   <213> Vicugna pacos
<400> 179
   Asn Tyr Ala Asp Ser Val Lys Gly Arg Phe Thr Ile Ser Arg Asp Asn
   1               5                   10                  15
   Ala Lys Asn Ala Val Tyr Leu Gln Met Asn Ser Leu Lys Pro Glu Asp
               20                  25                  30
   Thr Ala Val Tyr Tyr Cys Asn Ala
           35                  40
<210> 180
   <211> 40
   <212> PRT
   <213> Vicugna pacos
<400> 180
   Gln Tyr Ala Glu Gly Met Lys Gly Arg Phe Thr Ile Ser Lys Gly Asn
   1               5                   10                  15
   Asp Lys Phe Ser Thr Tyr Leu Gln Met Asn Asn Leu Lys Pro Asp Asp
               20                  25                  30
   Thr Ala Val Tyr Tyr Cys Asn Ala
           35                  40
<210> 181
   <211> 40
   <212> PRT
   <213> Vicugna pacos
<400> 181
   Glu Tyr Ala Glu Ser Val Lys Gly Arg Phe Thr Ile Ser Arg Asp Ser
   1               5                   10                  15
   Ala Lys Asn Met Leu Tyr Leu Gln Met Asn Ser Leu Arg Pro Glu Asp
               20                  25                  30
   Thr Ala Val Tyr Tyr Cys Asn Ala
           35                  40
<210> 182
   <211> 40
   <212> PRT
   <213> Vicugna pacos
<400> 182
   Asn Tyr Ala Asp Ser Val Lys Gly Arg Phe Thr Ile Ser Gly Asp Asn
   1               5                   10                  15
   Ala Lys Asn Ser Val Tyr Leu His Met Asn Asn Leu Glu Pro Glu Asp
               20                  25                  30
   Thr Ala Val Tyr Tyr Cys Lys Ala
           35                  40
<210> 183
   <211> 40
   <212> PRT
   <213> Vicugna pacos
<400> 183
   Tyr Tyr Ala Asp Ser Val Lys Gly Arg Phe Thr Ile Ser Gly Asp Ile
   1               5                   10                  15
   Ala Lys Asn Leu Leu Trp Leu Gln Met Asn Ser Leu Lys Pro Glu Asp
               20                  25                  30
   Thr Ala Met Tyr Tyr Cys Ala Pro
           35                  40
<210> 184
   <211> 40
   <212> PRT
   <213> Vicugna pacos
<400> 184
   Tyr Tyr Ala Asp Pro Val Lys Gly Arg Phe Thr Ile Ser Arg Asp Asn
   1               5                   10                  15
   Ala Gln Asn Thr Ile Tyr Leu Gln Met Asn Tyr Leu Lys Pro Glu Asp
               20                  25                  30
   Thr Ala Ile Tyr Tyr Cys Ala Ile
           35                  40
<210> 185
   <211> 40
   <212> PRT
   <213> Vicugna pacos
<400> 185
   Asp Tyr Ala Asn Ser Val Lys Gly Arg Phe Thr Ile Ser Arg Asp Asn
   1               5                   10                  15
   Ala Lys Asn Arg Leu Tyr Leu Gln Met Asn Ser Leu Lys Pro Glu Asp
               20                  25                  30
   Thr Ala Val Tyr Phe Cys Ala Gln
           35                  40
<210> 186
   <211> 40
   <212> PRT
   <213> Vicugna pacos
<400> 186
   Asp Tyr Ser Asp Ala Val Lys Gly Arg Phe Thr Ile Ser Arg Asp Asn
   1 5 10 15
   Ala Lys Asn Arg Leu Tyr Leu Gln Met Asp Ser Leu Lys Pro Glu Asp
   20 25 30
   Thr Ala Val Tyr Phe Cys Ala Gln
   35 40
<210> 187
   <211> 9
   <212> PRT
   <213> Vicugna pacos
<400> 187
   Gly Phe Val Cys Tyr Asn Tyr Asp Tyr
   1 5
<210> 188
   <211> 9
   <212> PRT
   <213> Vicugna pacos
<400> 188
   Gly Phe Val Cys Tyr Asn Tyr Asp Tyr
   1 5
<210> 189
   <211> 13
   <212> PRT
   <213> Vicugna pacos
<400> 189
   Asp Met Asp Ser Ser Leu Ser Gly Gly Tyr Val Asp Val
   1 5 10
<210> 190
   <211> 22
   <212> PRT
   <213> Vicugna pacos
<400> 190
   Asp Phe Phe Arg Trp Asp Ser Gly Ser Tyr Tyr Val Arg Gly Cys Arg
   1 5 10 15
   His Ala Thr Tyr Asp Tyr
   20
<210> 191
   <211> 24
   <212> PRT
   <213> Vicugna pacos
<400> 191
   Glu Arg Ala Pro Pro Tyr Tyr Ser Gly Tyr Tyr Phe Phe Asp Ser Thr
   1               5                   10                  15
   Cys Val Ala Ala Ser Tyr Asp Tyr
               20
<210> 192
   <211> 13
   <212> PRT
   <213> Vicugna pacos
<400> 192
   Asp Gly Val Val Ala Trp Asp Gln Pro Tyr Asp Asn Tyr
   1               5                   10
<210> 193
   <211> 17
   <212> PRT
   <213> Vicugna pacos
<400> 193
   Asp Arg Arg Pro Tyr Asn Asp Trp Trp Asp Asp Trp Ser Trp Trp Val
   1               5                   10                  15
   Tyr
<210> 194
   <211> 18
   <212> PRT
   <213> Vicugna pacos
<400> 194
   Glu Ala Gln Thr Pro Tyr Asn Asp Gly Asp Cys Thr Arg Ala Ser Tyr
   1               5                   10                  15
   Asp Tyr
<210> 195
   <211> 11
   <212> PRT
   <213> Vicugna pacos
<400> 195
   Asp Ala Leu Thr Met Leu Pro Pro Phe Asp Phe
   1 5 10
<210> 196
   <211> 12
   <212> PRT
   <213> Vicugna pacos
<400> 196
   Ala Ile Val Thr Met Thr Ser Pro Tyr Ser Asp Tyr
   1 5 10
<210> 197
   <211> 5
   <212> PRT
   <213> Vicugna pacos
<400> 197
   Asn Trp Gly Ala Tyr
   1 5
<210> 198
   <211> 13
   <212> PRT
   <213> Vicugna pacos
<400> 198
   Leu Met Val Asp Tyr Gly Leu Gly Leu Gly Thr Asp Tyr
   1 5 10
<210> 199
   <211> 17
   <212> PRT
   <213> Vicugna pacos
<400> 199
   Asp Thr Tyr Tyr Tyr Cys Ser Lys Arg Val Trp Arg Asn Asp Tyr Gly
   1 5 10 15
   Ser
<210> 200
   <211> 16
   <212> PRT
   <213> Vicugna pacos
<400> 200
   Glu Thr Pro Ser Ile Gly Ser Pro Cys Thr Ser Ala Ser Tyr Asp Tyr
   1 5 10 15
<210> 201
   <211> 16
   <212> PRT
   <213> Vicugna pacos
<400> 201
   Leu Arg Arg Tyr Tyr Gly Leu Asn Leu Asp Pro Gly Ser Tyr Asp Tyr
   1               5                   10                  15
<210> 202
   <211> 15
   <212> PRT
   <213> Vicugna pacos
<400> 202
   Arg Thr Val Ser Ala Pro Pro Ser Ala Ala Trp Gly Tyr Gly Tyr
   1               5                   10                  15
<210> 203
   <211> 18
   <212> PRT
   <213> Vicugna pacos
<400> 203
   His Leu Ala Gln Tyr Ser Asp Tyr Ala Tyr Arg Asp Pro His Gln Phe
   1               5                   10                  15
   Gly Ala
<210> 204
   <211> 9
   <212> PRT
   <213> Vicugna pacos
<400> 204
   Gly His Thr Trp Gly Gln Tyr Ala Tyr
   1               5
<210> 205
   <211> 10
   <212> PRT
   <213> Vicugna pacos
<400> 205
   Arg Gly Arg Phe Asp Asp Asn Tyr Glu Tyr
   1               5                   10
<210> 206
   <211> 13
   <212> PRT
   <213> Vicugna pacos
<400> 206
   Asp Met Asp Ser Ser Leu Ser Gly Gly Tyr Val Asp Val
   1 5 10
<210> 207
   <211> 5
   <212> PRT
   <213> Vicugna pacos
<400> 207
   Asn Trp Gly Ala Tyr
   1 5
<210> 208
   <211> 11
   <212> PRT
   <213> Vicugna pacos
<400> 208
   Leu Val Ile Gly Pro Leu Glu Gly Tyr Asp Tyr
   1 5 10
<210> 209
   <211> 13
   <212> PRT
   <213> Vicugna pacos
<400> 209
   Asp Gly Val Val Ala Trp Asp Gln Pro Tyr Asp Asn Tyr
   1 5 10
<210> 210
   <211> 9
   <212> PRT
   <213> Vicugna pacos
<400> 210
   Glu Arg Trp Asp Asn Gly Met Val Tyr
   1 5
<210> 211
   <211> 23
   <212> PRT
   <213> Vicugna pacos
<400> 211
   Glu Arg Trp Asp Gly Tyr Ala Leu Gly Tyr Ser Pro Asn His Gly Ser
   1 5 10 15
   Gly His Arg Pro Tyr Asn Tyr
   20
<210> 212
   <211> 12
   <212> PRT
   <213> Vicugna pacos
<400> 212
   Val Ala Val Thr Phe Thr Thr Pro Arg Ser Asp Tyr
   1 5 10
<210> 213
   <211> 8
   <212> PRT
   <213> Vicugna pacos
<400> 213
   Gly Gly Gly Trp Arg Pro Gly Ala
   1 5
<210> 214
   <211> 8
   <212> PRT
   <213> Vicugna pacos
<400> 214
   Asn Arg Glu Thr Met Pro Pro Phe
   1 5
<210> 215
   <211> 12
   <212> PRT
   <213> Vicugna pacos
<400> 215
   Gly Tyr Ala Arg Leu Ile Ala Asp Ser Asp Leu Val
   1 5 10
<210> 216
   <211> 12
   <212> PRT
   <213> Vicugna pacos
<400> 216
   Gly Tyr Ala Arg Leu Met Thr Asp Ser Glu Leu Val
   1 5 10
<210> 217
   <211> 11
   <212> PRT
   <213> Vicugna pacos
<400> 217
   Trp Gly Gln Gly Thr Gln Val Thr Val Ser Ser
   1               5                   10
<210> 218
   <211> 11
   <212> PRT
   <213> Vicugna pacos
<400> 218
   Trp Gly Pro Gly Thr Gln Val Thr Val Ser Ser
   1               5                   10
<210> 219
   <211> 11
   <212> PRT
   <213> Vicugna pacos
<400> 219
   Trp Gly Gln Gly Thr Gln Val Thr Val Ser Ser
   1               5                   10
<210> 220
   <211> 11
   <212> PRT
   <213> Vicugna pacos
<400> 220
   Trp Gly Gln Gly Thr Gln Val Thr Val Ser Ser
   1               5                   10
<210> 221
   <211> 11
   <212> PRT
   <213> Vicugna pacos
<400> 221
   Trp Gly Gln Gly Thr Gln Val Thr Val Ser Ser
   1               5                   10
<210> 222
   <211> 11
   <212> PRT
   <213> Vicugna pacos
<400> 222
   Trp Gly Gln Gly Thr Gln Val Thr Val Ser Ser
   1               5                   10
<210> 223
   <211> 11
   <212> PRT
   <213> Vicugna pacos
<400> 223
   Trp Gly Gln Gly Thr Gln Val Thr Val Ser Ser
   1 5 10
<210> 224
   <211> 11
   <212> PRT
   <213> Vicugna pacos
<400> 224
   Trp Gly Gln Gly Ile Gln Val Thr Val Ser Ser
   1 5 10
<210> 225
   <211> 11
   <212> PRT
   <213> Vicugna pacos
<400> 225
   Trp Gly Gln Gly Thr Gln Val Thr Val Ser Ser
   1 5 10
<210> 226
   <211> 11
   <212> PRT
   <213> Vicugna pacos
<400> 226
   Trp Gly Gln Gly Thr Gln Val Thr Val Ser Ser
   1 5 10
<210> 227
   <211> 11
   <212> PRT
   <213> Vicugna pacos
<400> 227
   Trp Gly Gln Gly Thr Gln Val Thr Val Ser Ser
   1 5 10
<210> 228
   <211> 11
   <212> PRT
   <213> Vicugna pacos
<400> 228
   Trp Gly Gln Gly Thr Gln Val Thr Val Ser Ser
   1 5 10
<210> 229
   <211> 11
   <212> PRT
   <213> Vicugna pacos
<400> 229
   Trp Gly Gln Gly Thr Gln Val Thr Val Ser Ser
   1 5 10
<210> 230
   <211> 11
   <212> PRT
   <213> Vicugna pacos
<400> 230
   Trp Gly Gln Gly Thr Gln Val Thr Val Ser Ser
   1 5 10
<210> 231
   <211> 11
   <212> PRT
   <213> Vicugna pacos
<400> 231
   Trp Gly Gln Gly Thr Gln Val Thr Val Ser Ser
   1 5 10
<210> 232
   <211> 11
   <212> PRT
   <213> Vicugna pacos
<400> 232
   Trp Gly Gln Gly Thr Gln Val Thr Val Ser Ser
   1 5 10
<210> 233
   <211> 11
   <212> PRT
   <213> Vicugna pacos
<400> 233
   Trp Gly Gln Gly Thr Gln Val Thr Val Ser Ser
   1 5 10
<210> 234
   <211> 11
   <212> PRT
   <213> Vicugna pacos
<400> 234
   Trp Gly Gln Gly Thr Gln Val Thr Val Ser Ser
   1 5 10
<210> 235
   <211> 11
   <212> PRT
   <213> Vicugna pacos
<400> 235
   Trp Gly Gln Gly Thr Gln Val Thr Val Ser Ser
   1 5 10
<210> 236
   <211> 11
   <212> PRT
   <213> Vicugna pacos
<400> 236
   Trp Gly Gln Gly Thr Gln Val Thr Val Ser Ser
   1 5 10
<210> 237
   <211> 11
   <212> PRT
   <213> Vicugna pacos
<400> 237
   Trp Gly Gln Gly Thr Gln Val Thr Val Ser Ser
   1 5 10
<210> 238
   <211> 11
   <212> PRT
   <213> Vicugna pacos
<400> 238
   Trp Gly Gln Gly Thr Gln Val Thr Val Ser Ser
   1 5 10
<210> 239
   <211> 11
   <212> PRT
   <213> Vicugna pacos
<400> 239
   Trp Gly Gln Gly Thr Gln Val Thr Val Ser Ser
   1 5 10
<210> 240
   <211> 11
   <212> PRT
   <213> Vicugna pacos
<400> 240
   Trp Gly Lys Gly Thr Gln Val Thr Val Ser Ser
   1 5 10
<210> 241
   <211> 11
   <212> PRT
   <213> Vicugna pacos
<400> 241
   Trp Gly Gln Gly Thr Gln Val Thr Val Ser Ser
   1 5 10
<210> 242
   <211> 11
   <212> PRT
   <213> Vicugna pacos
<400> 242
   Trp Gly Arg Gly Thr Gln Val Thr Val Ser Ser
   1 5 10
<210> 243
   <211> 11
   <212> PRT
   <213> Vicugna pacos
<400> 243
   Trp Gly Gln Gly Thr Gln Val Thr Val Ser Ser
   1 5 10
<210> 244
   <211> 11
   <212> PRT
   <213> Vicugna pacos
<400> 244
   Arg Gly Gln Gly Thr Gln Val Thr Val Ser Ser
   1 5 10
<210> 245
   <211> 11
   <212> PRT
   <213> Vicugna pacos
<400> 245
   Arg Gly Gln Gly Thr Gln Val Thr Val Ser Ser
   1 5 10
<210> 246
   <211> 11
   <212> PRT
   <213> Vicugna pacos
<400> 246
   Arg Gly Gln Gly Thr Gln Val Thr Val Ser Ser
   1               5                   10
<210> 247
   <211> 125
   <212> PRT
   <213> Artificial Sequence
<220>
   <223> Anti-mouse MMR Nanobody clone 1
<400> 247
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Asn Ile Phe Ser Ile Asn
               20                  25                  30
   Ala Ile Gly Trp Tyr Arg Gln Ala Pro Gly Lys Gln Arg Glu Leu Val
           35                  40                  45
   Ala Thr Ile Thr Leu Ser Gly Ser Thr Asn Tyr Ala Asp Ser Val Lys
       50                  55                  60
   Gly Arg Phe Ser Ile Ser Arg Asp Asn Ala Lys Asn Thr Val Tyr Leu
   65                  70                  75                  80
   Gln Met Asn Ser Leu Lys Pro Glu Asp Thr Ala Val Tyr Tyr Cys Asn
                   85                  90                  95
   Ala Asn Thr Tyr Ser Asp Ser Asp Val Tyr Gly Tyr Trp Gly Gln Gly
               100                 105                 110
   Thr Gln Val Thr Val Ser Ser His His His His His His
           115                 120                 125
<210> 248
   <211> 10
   <212> PRT
   <213> Artificial Sequence
<220>
   <223> Linker sequence
<400> 248
   Gly Ser Gly Ser Gly Ser Gly Ser Gly Ser
   1               5                   10
<210> 249
   <211> 20
   <212> PRT
   <213> Artificial Sequence
<220>
   <223> Linker sequence
<400> 249
   Gly Ser Gly Ser Gly Ser Gly Ser Gly Ser Gly Ser Gly Ser Gly Ser
   1               5                   10                  15
   Gly Ser Gly Ser
               20
<210> 250
   <211> 15
   <212> PRT
   <213> Artificial Sequence
<220>
   <223> Linker sequence
<400> 250
   Gly Gly Gly Gly Ser Gly Gly Gly Gly Ser Gly Gly Gly Gly Ser
   1               5                   10                  15
<210> 251
   <211> 17
   <212> PRT
   <213> Artificial Sequence
<220>
   <223> Linker sequence
<400> 251
   Ala His His Ser Glu Asp Pro Ser Ser Lys Ala Pro Lys Ala Pro Met
   1               5                   10                  15
   Ala
<210> 252
   <211> 16
   <212> PRT
   <213> Artificial Sequence
<220>
   <223> Linker sequence
<400> 252
   Ser Pro Ser Thr Pro Pro Thr Pro Ser Pro Ser Thr Pro Pro Ala Ser
   1               5                   10                  15
<210> 253
   <211> 4
   <212> PRT
   <213> Artificial Sequence
<220>
   <223> Factor Xa cleavage site
<400> 253
   Ile Glu Gly Arg
   1
<210> 254
   <211> 4
   <212> PRT
   <213> Artificial Sequence
<220>
   <223> thrombin cleavage site
<400> 254
   Leu Val Pro Arg
   1
<210> 255
   <211> 5
   <212> PRT
   <213> Artificial Sequence
<220>
   <223> enterokinase cleaving site
<400> 255
   Asp Asp Asp Asp Lys
   1 5
<210> 256
   <211> 8
   <212> PRT
   <213> Artificial Sequence
<220>
   <223> PreScission cleavage site
<400> 256
   Leu Glu Val Leu Phe Gln Gly Pro
   1 5
<210> 257
   <211> 22
   <212> DNA
   <213> Artificial Sequence
<220>
   <223> leader sequence specific CALL001
<400> 257
   gtcctggctc tcttctacaa gg 22
<210> 258
   <211> 23
   <212> DNA
   <213> Artificial Sequence
<220>
   <223> CH2 exon specific CALL002
<400> 258
   ggtacgtgct gttgaactgt tcc 23
<210> 259
   <211> 29
   <212> DNA
   <213> Artificial Sequence
<220>
   <223> primer A6E
<400> 259
   gatgtgcagc tgcaggagtc tggrggagg 29
<210> 260
   <211> 34
   <212> DNA
   <213> Artificial Sequence
<220>
   <223> primer PMCF
<400> 260
   ctagtgcggc cgctgaggag acggtgacct gggt 34
<210> 261
   <211> 10
   <212> PRT
   <213> Artificial Sequence
<220>
   <223> HA tag
<400> 261
   Tyr Pro Tyr Asp Val Pro Asp Tyr Gly Ser
   1               5                   10
<210> 262
   <211> 19
   <212> PRT
   <213> Artificial Sequence
<220>
   <223> HA and 6xHis tag
<400> 262
   Ala Ala Ala Tyr Pro Tyr Asp Val Pro Asp Tyr Gly Ser His His His
   1               5                   10                  15
   His His His